(19) Europäisches Patentamt
European Patent Office
Office européen des brevets

(11) EP 2 913 351 A1

(12) # EUROPÄISCHE PATENTANMELDUNG

(43) Veröffentlichungstag:
**02.09.2015 Patentblatt 2015/36**

(21) Anmeldenummer: **14157228.9**

(22) Anmeldetag: **28.02.2014**

(51) Int Cl.:
***C08F 220/18*** (2006.01) ***C08F 220/20*** (2006.01)
***C08F 220/58*** (2006.01) ***C08F 220/60*** (2006.01)
***A61Q 5/00*** (2006.01) ***A61K 31/78*** (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **BASF SE**
**67056 Ludwigshafen (DE)**

(72) Erfinder: **Die Erfindernennung liegt noch nicht vor**

(74) Vertreter: **Reitstötter Kinzebach**
**Patentanwälte**
**Im Zollhof 1**
**67061 Ludwigshafen (DE)**

(54) **Verfahren zur Herstellung von Copolymeren der 2-Acrylamido-2-methylpropansulfonsäure**

(57) Die vorliegende Erfindung betrifft ein Verfahren zur Herstellung einer Copolymerzusammensetzung A) durch radikalische Copolymerisation einer Monomerzusammensetzung, die 2-Acrylamido-2-methylpropansulfonsäure (Monomer a) und wenigstens ein damit copolymerisierbares Comonomer enthält, mit der Maßgabe,
- dass die 2-Acrylamido-2-methylpropansulfonsäure a) vor ihrem Einsatz in der Polymerisation mit einer Aminkomponente AM) neutralisiert wird, die aus Aminen ausgewählt ist, die ausschließlich sekundäre und/oder tertiäre Aminogruppen aufweisen,
- wobei die Aminogruppen der Aminkomponente AM) in wenigstens äquimolarer Menge bezogen auf die Sufonsäuregruppen der 2-Acrylamido-2-methylpropan-sulfonsäure eingesetzt werden und
- die Polymerisation in einem Lösungsmittel erfolgt, das wenigstens einen Alkohol umfasst oder aus wenigstens einem Alkohol besteht,
eine Copolymerzusammensetzung A), die durch ein solches Verfahren erhältlich ist, ein kosmetisches Mittel, das eine solche Copolymerzusammensetzung A) enthält und die Verwendung dieser Copolymerzusammensetzung A).

EP 2 913 351 A1

**Beschreibung**

HINTERGRUND DER ERFINDUNG

Die vorliegende Erfindung betrifft ein Verfahren zur Herstellung von Copolymeren der 2-Acrylamido-2-methylpropansulfonsäure, die nach diesem Verfahren erhältliche Copolymerzusammensetzung und deren Verwendung.

STAND DER TECHNIK

2-Acrylamido-2-methylpropansulfonsäure (AMPS, 2-Acrylamido-tert.-butyl-sulfonsäure) ist ein sehr reaktionsfähiges und gut wasserlösliches Sulfonsäuregruppen-haltiges Monomer, das zur Herstellung von Homo- und Copolymeren mit außergewöhnlichen Produkteigenschaften geeignet ist. AMPS hat aufgrund seiner Funktionalität breiten Einsatz in einer Vielzahl von Anwendungen gefunden.

In der Kosmetik und Medizin werden AMPS-Copolymere unter Anderem als Hydrogele und in Emulsionen als Verdicker, Stabilisator, Dispergator und Gleitmittel angewendet. Im Textilbereich erhöhen AMPS-Copolymere die Anfärbbarkeit von polyacryl- und polyesterhaltigen Textilien, in Waschmitteln steigern sie die Waschwirkung von Tensiden durch Bindung mehrwertiger Kationen und reduzieren die Schmutzanlagerung. Im Bereich der Wasserbehandlung stabilisieren AMPS-Copolymere mit anderen ionischen und neutralen Monomeren mehrwertige Kationen in hartem Wasser und verhindert als "scale inhibitor" die Kesselsteinbildung und Korrosion. Im Bausektor wirken AMPS-Copolymere als Wasserrückhaltemittel in Mörteln und Putzen, führen zu einer verbesserten Dispergierbarkeit von Partikeln, erhöhen das Gleitvermögen und verbessern insbesondere in Copolymeren mit Acryl- oder Methacrylsäure als Superplasticer die mechanische und chemische Stabilität von hochfließfähigem und selbstverdichtendem Beton. Bei der Öl- und Gasexploration zeichnen sich AMPS-Copolymere durch ihre Stabilität gegenüber hohen Temperaturen und Drücken sowie hohen Salzkonzentrationen aus. Sie verringern die Reibung und den Flüssigkeitsverlust in Bohr- und Zementierungsflüssigkeiten und findet Anwendung in der tertiären Ölförderung (enhanced oil recovery, EOR) und bei dem als "hydraulic fracturing" bezeichneten Aufbrechen von Gesteinsformationen zur Förderung von Schiefergas.

Die US 5,260,391 beschreibt ein Copolymer auf Basis von AMPS, N-Vinylpyrrolidon, Acrylamid und Acrylsäure(salzen) und dessen Verwendung bei der Zementierung von Gas- und Ölquellen. Die Polymerisation erfolgt nach üblichen Verfahren der Lösungs-, Suspensions- oder Emulsionspolymerisation, wobei eine Initiierung mit hohem Energieeintrag, z. B. durch Elektronenstrahlen, bevorzugt ist.

Die EP 0 003 235 A1 beschreibt ein Verfahren zur Herstellung wasserlöslicher Copolymerisate auf Basis von AMPS und Polyetheracrylaten durch Lösungspolymerisation in Wasser und/oder wenigstens einem organischen wasserlöslichen Lösungsmittel.

Die US 2011/0319561 A1 beschreibt ein Verfahren zur Herstellung von Copolymeren auf Basis von AMPS und Acrylsäure durch direkte Emulsionspolymerisation. Die Copolymere können vernetzt sein und enthalten in einer bevorzugten Ausführung ein Monomer mit assoziativ wirkenden Gruppen einpolymerisiert, so dass eine hydrophob modifizierte Alkali-quellbare Emulsion (HASE) resultiert.

Die WO 2004/058837 beschreibt ampholytische Copolymere und deren Verwendung in kosmetischen und pharmazeutischen Mitteln. Eine Ausführungsform betrifft Copolymere, die Vinylpyrrolidon, AMPS und ein Monomer mit einer kationogenen/kationischen Gruppe einpolymerisiert enthalten. Als ein geeignetes Monomer mit einer kationogenen/kationischen Gruppe ist neben einer Vielzahl weiterer auch Vinylimidazol erwähnt. Es ist ebenfalls beschrieben, zur Polymerisation das Natriumsalz von AMPS einzusetzen. Dieses Dokument lehrt nicht, zur Salzbildung ein Amin einzusetzen, das ausschließlich sekundäre und/oder tertiäre Aminogruppen aufweist. Konkret wird in den Ausführungsbeispielen zur Herstellung eines AMPS-Copolymers eine Mischung aus gleichen Gewichtsteilen AMPS und AMPS-Natriumsalz eingesetzt und in einem Ethanol/Wasser-Gemisch umgesetzt.

Die WO 2010/009953 beschreibt ein Stylingmittel, das dem Haar einen guten Halt verleiht und das ein Copolymer enthält, das Natriumacrylat, ein AMPS-Kalium- oder Ammoniumsalz und Dimethylacrylamid einpolymerisiert enthält. Bevorzugt liegt das Copolymer in Form einer inversen, auto-inversiblen Polymerlatex vor, die eine Ölphase, eine Wasserphase und wenigstens einen Öl-in-Wasser-Emulgator enthält.

Die EP 1 028 129 A1 beschreibt vernetzte wasserlösliche oder wasserquellbare Polymere, die Acrylamidoalkylsulfonsäuren und N-Vinylcarbonsäureamide (speziell N-Vinylformamid) einpolymerisiert enthalten und deren Verwendung als Verdicker, Stabilisatoren von Emulsionen und Dispersionen und Rheologiemodifizierungsmittel für kosmetische und pharmazeutische Formulierungen. Bevorzugt wird zur Herstellung dieser Polymere ein Ammoniumsalz von AMPS eingesetzt. Zu dessen Herstellung wird AMPS vor Beginn der Polymerisation mit gasförmigem $NH_3$ neutralisiert. Die Polymerisation erfolgt vorzugsweise in wenigstens einem wasserlöslichen Alkohol, vorzugsweise in tert.-Butanol, nach dem Verfahren der Fällungspolymerisation. Der Wassergehalt darf dabei 10 Gew.-% nicht überschreiten. Dieses Dokument lehrt nicht, zur Salzbildung aus der 2-Acrylamido-2-methylpropansulfonsäure eine andere Base als Ammoniak und speziell kein Amin einzusetzen, das ausschließlich sekundäre und/oder tertiäre Aminogruppen aufweist..

Es besteht nach wie vor Bedarf an Verfahren zur Herstellung von AMPS-Copolymeren mit guten anwendungstechnischen Eigenschaften. Dazu zählt zum einen für einen Einsatz als Haarfestigerpolymer die Bildung klarer, glatter Filme, die dem Haar einen guten Halt verleihen und gleichzeitig gut auswaschbar sind. Dazu zählt weiterhin der Einsatz als polymere oberflächenaktive Verbindung (Polymertensid). Dazu zählt weiterhin der Einsatz zur Einstellung der rheologischen Eigenschaften diverser Produkte, so dass sie z. B. in Form von Gelen formuliert werden können.

Überraschenderweise wurde nun gefunden, dass diese Aufgabe durch ein Polymerisationsverfahren gelöst wird, bei dem die 2-Acrylamido-2-methylpropansulfonsäure vor ihrem Einsatz in der radikalischen Polymerisation mit einer Aminkomponente neutralisiert wird, die aus Aminen ausgewählt ist, die ausschließlich sekundäre und/oder tertiäre Aminogruppen aufweisen, und wobei die die Polymerisation in einem alkoholischen Lösungsmittel erfolgt. Insbesondere wird ein wasserfreies alkoholisches Lösungsmittel zur Polymerisation eingesetzt.

ZUSAMMENFASSUNG DER ERFINDUNG

Ein erster Gegenstand der Erfindung ist ein Verfahren zur Herstellung einer Copolymerzusammensetzung A) durch radikalische Copolymerisation einer Monomerzusammensetzung, die 2-Acrylamido-2-methylpropansulfonsäure (Monomer a) und wenigstens ein damit copolymerisierbares Comonomer enthält, mit der Maßgabe,

- dass die 2-Acrylamido-2-methylpropansulfonsäure a) vor ihrem Einsatz in der Polymerisation mit einer Aminkomponente AM) neutralisiert wird, die aus Aminen ausgewählt ist, die ausschließlich sekundäre und/oder tertiäre Aminogruppen aufweisen,
- wobei die Aminogruppen der Aminkomponente AM) in wenigstens äquimolarer Menge bezogen auf die Sufonsäuregruppen der 2-Acrylamido-2-methylpropan-sulfonsäure eingesetzt werden und
- die Polymerisation in einem Lösungsmittel erfolgt, das wenigstens einen Alkohol umfasst.

In einer speziellen Ausführungsform beträgt der Wassergehalt der zur Polymerisation eingesetzten Reaktionsmischung höchstens 5 Gew.-%, besonders bevorzugt höchstens 3 Gew.-%, insbesondere höchstens 1 Gew.-%, bezogen auf das Gesamtgewicht der Reaktionsmischung.

Ein weiterer Gegenstand der Erfindung ist eine Copolymerzusammensetzung A) wie zuvor und im Folgenden definiert, die nach diesem Verfahren erhältlich ist.

Ein weiterer Gegenstand der Erfindung ist ein kosmetisches Mittel, enthaltend

A) wenigstens eine Copolymerzusammensetzung, wie zuvor und im Folgenden definiert,

B) wenigstens einen kosmetisch akzeptablen Wirkstoff und

C) gegebenenfalls wenigstens einen weiteren, von A) und B) verschiedenen kosmetisch akzeptablen Hilfsstoff.

Ein weiterer Gegenstand der Erfindung ist die Verwendung einer Copolymerzusammensetzung A), erhältlich durch ein Verfahren, wie zuvor und im Folgenden definiert, als Hilfsstoff in der Kosmetik, als Hilfsstoff in der Pharmazie, als Hilfsstoff in der Lebensmittelindustrie, als oberflächenaktive Verbindung, als oder in Klebemittel(n) sowie als oder in Beschichtungsmittel(n) für die Textil-, Papier-, Druck- und Lederindustrie.

Ein weiterer Gegenstand der Erfindung ist die Verwendung einer Copolymerzusammensetzung A), erhältlich durch ein Verfahren, wie zuvor und im Folgenden definiert, als filmbildendes Polymer in der Kosmetik oder Pharmazie, insbesondere als Festigerpolymer in der Haarkosmetik.

Ein weiterer Gegenstand der Erfindung ist die Verwendung einer Copolymerzusammensetzung A), erhältlich durch ein Verfahren, wie zuvor und im Folgenden definiert, als grenzflächenaktive Verbindung, insbesondere als Polymeremulgator oder Polymerstabilisator.

Ein weiterer Gegenstand der Erfindung ist die Verwendung einer Copolymerzusammensetzung A), erhältlich durch ein Verfahren, wie zuvor und im Folgenden definiert, als Verdickungsmittel.

AUSFÜHRUNGSFORMEN DER ERFINDUNG

Im Einzelnen umfasst die Erfindung die folgenden bevorzugten Ausführungsformen:

1. Verfahren zur Herstellung einer Copolymerzusammensetzung A) durch radikalische Copolymerisation einer Monomerzusammensetzung, die 2-Acrylamido-2-methylpropansulfonsäure (Monomer a) und wenigstens ein damit copolymerisierbares Comonomer enthält, mit der Maßgabe,

- dass die 2-Acrylamido-2-methylpropansulfonsäure a) vor ihrem Einsatz in der Polymerisation mit einer Aminkomponente AM) neutralisiert wird, die aus Aminen ausgewählt ist, die ausschließlich sekundäre und/oder tertiäre Aminogruppen aufweisen,
- wobei die Aminogruppen der Aminkomponente AM) in wenigstens äquimolarer Menge bezogen auf die Sufonsäuregruppen der 2-Acrylamido-2-methylpropansulfonsäure eingesetzt werden und
- die Polymerisation in einem Lösungsmittel erfolgt, das wenigstens einen Alkohol umfasst.

2. Verfahren nach Ausführungsform 1, wobei der Wassergehalt der zur Polymerisation eingesetzten Reaktionsmischung höchstens 5 Gew.-%, bevorzugt höchstens 3 Gew.-%, insbesondere höchstens 1 Gew.-%, bezogen auf das Gesamtgewicht der Reaktionsmischung, beträgt.

3. Verfahren nach Ausführungsform 1, wobei die Monomerzusammensetzung wenigstens eine N-Vinylimidazol-Verbindung b) der allgemeinen Formel (I)

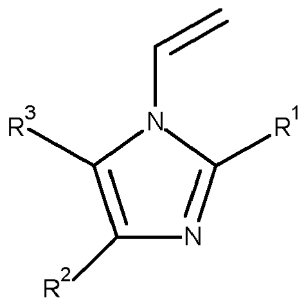

(I)

enthält, worin $R^1$, $R^2$ und $R^3$ unabhängig voneinander für Wasserstoff, $C_1$-$C_4$-Alkyl oder Phenyl stehen.

4. Verfahren nach einer der vorhergehenden Ausführungsformen, wobei die zur Herstellung der Copolymerzusammensetzung A) eingesetzte Monomerzusammensetzung wenigstens ein amidgruppenhaltiges Monomer c) enthält, das ausgewählt ist unter einer $\alpha,\beta$-ethylenisch ungesättigten amidgruppenhaltigen Verbindung der allgemeinen Formel (II)

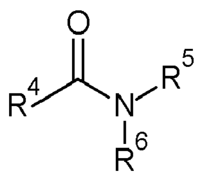

(II)

wobei

einer der Reste $R^4$ bis $R^6$ für eine Gruppe der Formel $CH_2$=$CR^7$- mit $R^7$ = H oder $C_1$-$C_4$-Alkyl steht und die übrigen Reste $R^4$ bis $R^6$ unabhängig voneinander für H, Alkyl, Cycloalkyl, Heterocycloalkyl, Aryl oder Hetaryl stehen,

wobei $R^5$ und $R^6$ gemeinsam mit dem Stickstoffatom, an das sie gebunden sind, auch für einen fünf- bis siebengliedrigen Heterocyclus stehen können.

5. Verfahren nach einer der vorhergehenden Ausführungsformen, wobei die Monomerzusammensetzung wenigstens eine von den Verbindungen a) verschiedene Verbindung d) mit einer radikalisch polymerisierbaren, $\alpha,\beta$-ethylenisch ungesättigten Doppelbindung und wenigstens einer anionogenen und/oder anionischen Gruppen pro Molekül enthält, vorzugsweise ausgewählt unter Acrylsäure, Methacrylsäure und Mischungen davon.

6. Verfahren nach einer der vorhergehenden Ausführungsformen, wobei die zur Herstellung der Copolymerzusammensetzung A) eingesetzte Monomerzusammensetzung zusätzlich wenigstens ein ethergruppenhaltiges Monomer e) enthält, das ausgewählt ist unter Verbindungen der allgemeinen Formeln III

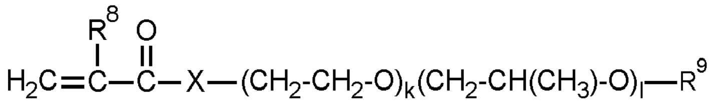

(III)

worin
die Reihenfolge der Alkylenoxideinheiten in den Verbindungen (III) beliebig ist,

k und l unabhängig voneinander für eine ganze Zahl von 0 bis 1000 stehen, wobei die Summe aus k und l mindestens 5 beträgt,

$R^8$ für Wasserstoff oder $C_1$-$C_4$-Alkyl, vorzugsweise Methyl, steht,

$R^9$ für Wasserstoff, $C_1$-$C_{30}$-Alkyl, $C_2$-$C_{30}$-Alkenyl oder $C_5$-$C_8$-Cycloalkyl steht,

X für O oder eine Gruppe der Formel $NR^{10}$ steht, worin $R^{10}$ für H, Alkyl, Alkenyl, Cycloalkyl, Heterocycloalkyl, Aryl oder Hetaryl steht.

7. Verfahren nach Ausführungsform 6, wobei die zur Herstellung der Copolymerzusammensetzung A) eingesetzte Monomerzusammensetzung wenigstens eine Verbindung e) enthält, die ausgewählt ist unter Verbindungen der allgemeinen Formel (III.1)

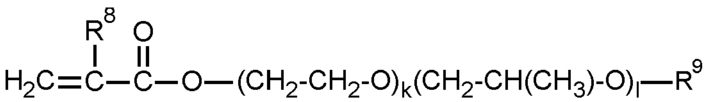

(III.1)

worin
die Reihenfolge der Alkylenoxideinheiten beliebig ist,

k und l unabhängig voneinander für eine ganze Zahl von 0 bis 100 stehen,

$R^8$ für Wasserstoff oder Methyl steht,

$R^9$ für $C_8$-$C_{30}$-Alkyl steht.

8. Verfahren nach einer der vorhergehenden Ausführungsformen, wobei die Monomerzusammensetzung wenigstens eine von der Komponente b) verschiedene Verbindung f) mit einer radikalisch polymerisierbaren, $\alpha,\beta$-ethylenisch ungesättigten Doppelbindung und mindestens einer kationogenen Gruppe pro Molekül enthält.

9. Verfahren nach einer der vorhergehenden Ausführungsformen, wobei die Monomerzusammensetzung wenigstens eine radikalisch polymerisierbare vernetzende Verbindung g) enthält, die wenigstens zwei $\alpha,\beta$-ethylenisch ungesättigte Doppelbindungen pro Molekül enthält.

10. Verfahren nach einer der vorhergehenden Ausführungsformen, wobei die zur Herstellung der Copolymerzusammensetzung A) eingesetzte Monomerzusammensetzung wenigstens ein weiteres Monomer h) enthält, das ausgewählt ist unter

h1) Estern $\alpha,\beta$-ethylenisch ungesättigter Mono- und Dicarbonsäuren mit $C_1$-$C_{30}$-Alkanolen,
h2) Estern $\alpha,\beta$-ethylenisch ungesättigter Mono- und Dicarbonsäuren mit $C_2$-$C_{30}$-Diolen,
h3) Amiden $\alpha,\beta$-ethylenisch ungesättigter Mono- und Dicarbonsäuren mit $C_2$-$C_{30}$-Aminoalkoholen, die eine primäre oder sekundäre Aminogruppe aufweisen,
h4) amidgruppenhaltigen Monomeren, die eine Gruppe der Formeln (IIIa) oder (IIIb)

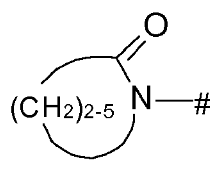 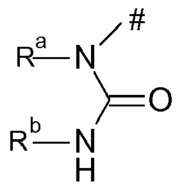

(IVa) (IVb)

aufweisen, worin

| | |
|---|---|
| # | für die Bindungsstelle zu einer Gruppe mit einer radikalisch polymerisierbaren, α,β-ethylenisch ungesättigten Doppelbindung steht, wobei in den Verbindungen (IIIa) # nicht für die Bindungsstelle zu einer Gruppe der Formel $CH_2$=$CR^7$- mit $R^7$ = H oder $C_1$-$C_4$-Alkyl steht, |
| $R^a$ | für H oder $C_1$-$C_4$-Alkyl steht, |
| $R^b$ | für H oder $C_1$-$C_4$-Alkyl steht, oder |
| $R^a$ und $R^b$ | gemeinsam für $(CH_2)_{1-4}$ stehen, |

h5) Urethan(meth)acrylaten mit Alkylenoxidgruppen,
h6) $C_1$-$C_{30}$-Alkylvinylethern,
h7) Vinylaromaten,
h8) Vinylhalogeniden, Vinylidenhalogeniden,

und Mischungen davon.

11. Verfahren nach einer der vorhergehenden Ausführungsformen, wobei die Aminkomponente AM) ausgewählt ist unter N-Vinylimidazolverbindungen (= Monomer b), N-($C_1$-$C_8$)-Alkylimidazolen, N-(Di-($C_1$-$C_4$)-Alkylamino-($C_1$-$C_8$)-alkyl)imidazolen, N-($C_1$-$C_8$)-Alkylpiperazinen, N-Hydroxyalkylpiperazinen, Alkyldialkanolaminen, Trialkanolaminen und Mischungen davon.

12. Verfahren nach einer der vorhergehenden Ausführungsformen, wobei die Aminkomponente AM) wenigstens ein N-($C_1$-$C_8$)-Alkylimidazol umfasst oder aus wenigstens einem N-($C_1$-$C_8$)-Alkylimidazol besteht.

13. Verfahren nach einer der vorhergehenden Ausführungsformen, wobei die Aminkomponente AM) ausgewählt ist unter N-Vinylimidazol, N-Methylimidazol, Methyldiethanolamin, Triethanolamin und Mischungen davon.

14. Verfahren nach einer der vorhergehenden Ausführungsformen, wobei die Polymerisation in einem polaren Lösungsmittel erfolgt, das wenigstens einen bei Normalbedingungen flüssigen Alkohol enthält.

15. Verfahren nach einer der Ausführungsformen 1 bis 14, wobei die Polymerisation als Lösungspolymerisation in einem Lösungsmittel erfolgt, das

- Ethanol und/oder Isopropanol, oder
- Isopropanol und Propylenglycol

enthält.

16. Verfahren nach einer der Ausführungsformen 1 bis 15, wobei die zur Copolymerisation eingesetzte Monomerzusammensetzung

- 3 bis 25 Gew.-%, bezogen auf das Gesamtgewicht der zur Polymerisation eingesetzten Monomere, 2-Acrylamido-2-methylpropansulfonsäure a),

- 0 bis 90 Gew.-%, bezogen auf das Gesamtgewicht der zur Polymerisation eingesetzten Monomere, wenigstens einer Verbindung c), ausgewählt unter $C_1$-$C_6$-Alkyl(meth)acrylamiden,

- 0 bis 90 Gew.-%, bezogen auf das Gesamtgewicht der zur Polymerisation eingesetzten Monomere, wenigstens einer Verbindung h1), ausgewählt unter $C_1$-$C_6$-Alkyl(meth)acrylaten,

- 0 bis 20 Gew.-%, bezogen auf das Gesamtgewicht der zur Polymerisation eingesetzten Monomere, wenigstens einer N-Vinylimidazol-Verbindung b),

- 0 bis 50 Gew.-%, bezogen auf das Gesamtgewicht der zur Polymerisation eingesetzten Monomere, wenigstens einer Verbindung d), ausgewählt unter Acrylsäure, Methacrylsäure, Salzen der Acrylsäure, Salzen der Methacrylsäure und Mischungen davon,

enthält, mit der Maßgabe, dass das Gesamtgewicht der Monomere c) und h1) 10 bis 90 Gew.-%, bezogen auf das Gesamtgewicht der zur Polymerisation eingesetzten Monomere, beträgt, und dass sich die zur Polymerisation eingesetzten Monomere auf 100 Gew.-% addieren.

17. Verfahren nach Ausführungsform 16, wobei die Komponente c) ausgewählt ist unter N-Methyl(meth)acrylamid, N-Ethyl(meth)acrylamid,
N-Propyl(meth)acrylamid, N-(n-Butyl)(meth)acrylamid,
N-tert.-Butyl(meth)acrylamid und Mischungen davon.

18. Verfahren nach Ausführungsform 16 oder 17, wobei die Komponente h1) ausgewählt ist unter Methyl(meth)acrylat, Ethyl(meth)acrylat, n-Butyl(meth)acrylat, tert.-Butyl(meth)acrylat, und Mischungen davon.

19. Verfahren nach einer der Ausführungsformen 16 oder 17, wobei die Polymerisation als Lösungspolymerisation in einem Lösungsmittel erfolgt, das wenigstens einen bei Normalbedingungen flüssigen Alkohol enthält, bevorzugt in einem Lösungsmittel das Isopropanol und/oder Ethanol enthält oder aus Isopropanol und/oder Ethanol besteht.

20. Verfahren nach einer der Ausführungsformen 1 bis 15, wobei die zur Copolymerisation eingesetzte Monomerzusammensetzung

- 0,5 bis 30 Gew.-%, bezogen auf das Gesamtgewicht der zur Polymerisation eingesetzten Monomere, 2-Acrylamido-2-methylpropansulfonsäure a),

- 5 bis 70 Gew.-%, bezogen auf das Gesamtgewicht der zur Polymerisation eingesetzten Monomere, wenigstens einer Verbindung d), die ausgewählt ist unter Acrylsäure, Methacrylsäure, Salzen der Acrylsäure, Salzen der Methacrylsäure und Mischungen davon,

- 5 bis 50 Gew.-%, bezogen auf das Gesamtgewicht der zur Polymerisation eingesetzten Monomere, wenigstens einer Verbindung e), die ausgewählt ist mit $C_8$-$C_{30}$-Alkylgruppen terminierten Polyether(meth)acrylaten und Mischungen davon,

- 0 bis 25 Gew.-%, bezogen auf das Gesamtgewicht der zur Polymerisation eingesetzten Monomere, wenigstens einer Verbindung c), ausgewählt unter $C_1$-$C_6$-Alkyl(meth)acrylamiden,

- 0 bis 25 Gew.-%, bezogen auf das Gesamtgewicht der zur Polymerisation eingesetzten Monomere, wenigstens einer Verbindung h1), ausgewählt unter $C_1$-$C_{30}$-Alkyl(meth)acrylaten,

enthält, mit der Maßgabe, dass sich die zur Polymerisation eingesetzten Monomere auf 100 Gew.-% addieren.

21. Verfahren nach Ausführungsform 20, wobei die Komponente c) ausgewählt ist unter N-Methyl(meth)acrylamid, N-Ethyl(meth)acrylamid,
N-Propyl(meth)acrylamid, N-(n-Butyl)(meth)acrylamid,
N-tert.-Butyl(meth)acrylamid und Mischungen davon.

22. Verfahren nach Ausführungsform 20 oder 21, wobei die Komponente h1) ausgewählt ist unter Methyl(meth)acrylat, Ethyl(meth)acrylat, n-Butyl(meth)acrylat, tert.-Butyl(meth)acrylat, Lauryl(meth)acrylat, Stearyl(meth)acrylat, und Mischungen davon.

23. Verfahren nach Ausführungsform 20 bis 22 wobei die Komponente e) ausgewählt ist unter insbesondere mit

$C_{12}$-$C_{24}$-Alkylgruppen terminierten Polyethylenglycol-(meth)acrylaten und Mischungen davon.

24. Verfahren nach einer der Ausführungsformen 20 bis 23, wobei die Polymerisation als Lösungspolymerisation in einem wasserfreien Lösungsmittel erfolgt, bevorzugt in einem Lösungsmittel das Isopropanol und/oder Propylenglycol enthält oder in einem Lösungsmittel das aus Isopropanol und/oder Propylenglycol besteht.

25. Verfahren nach einer der Ausführungsformen 1 bis 15, wobei die zur Copolymerisation eingesetzte Monomerzusammensetzung

- 0,5 bis 50 Gew.-%, bezogen auf das Gesamtgewicht der zur Polymerisation eingesetzten Monomere, 2-Acrylamido-2-methylpropansulfonsäure a),
- 5 bis 90 Gew.-%, bezogen auf das Gesamtgewicht der zur Polymerisation eingesetzten Monomere, wenigstens einer Verbindung d), die ausgewählt ist unter Acrylsäure, Methacrylsäure, Salzen der Acrylsäure, Salzen der Methacrylsäure und Mischungen davon,
- 5 bis 50 Gew.-%, bezogen auf das Gesamtgewicht der zur Polymerisation eingesetzten Monomere, wenigstens einer Verbindung e), die ausgewählt ist unter mit $C_8$-$C_{30}$-Alkylgruppen terminierten Polyether(meth)acrylaten,
- 0,1 bis 3 Gew.-%, bezogen auf das Gesamtgewicht der zur Polymerisation eingesetzten Monomere, wenigstens eines Vernetzers g),
- 0 bis 40 Gew.-%, bezogen auf das Gesamtgewicht der zur Polymerisation eingesetzten Monomere, wenigstens einer Verbindung c), ausgewählt unter $C_1$-$C_6$-Alkyl(meth)acrylamiden,
- 0 bis 40 Gew.-%, bezogen auf das Gesamtgewicht der zur Polymerisation eingesetzten Monomere, wenigstens einer Verbindung h1), ausgewählt unter $C_1$-$C_{30}$-Alkyl(meth)acrylaten,

enthält, mit der Maßgabe, dass sich die zur Polymerisation eingesetzten Monomere auf 100 Gew.-% addieren.

26. Verfahren nach Ausführungsform 25, wobei die Komponente c) ausgewählt ist unter N-Methyl(meth)acrylamid, N-Ethyl(meth)acrylamid,
N-Propyl(meth)acrylamid, N-(n-Butyl)(meth)acrylamid,
N-tert.-Butyl(meth)acrylamid und Mischungen davon.

27. Verfahren nach Ausführungsform 25 oder 26, wobei die Komponente h1) ausgewählt ist unter Methyl(meth)acrylat, Ethyl(meth)acrylat, n-Butyl(meth)acrylat, tert.-Butyl(meth)acrylat, Lauryl(meth)acrylat, Stearyl(meth)acrylat, und Mischungen davon.

28. Verfahren nach Ausführungsform 25 bis 27 wobei die Komponente e) ausgewählt ist unter insbesondere mit $C_{12}$-$C_{24}$-Alkylgruppen terminierten Polyethylenglycol-(meth)acrylaten und Mischungen davon.

29. Verfahren nach Ausführungsform 25 bis 28, wobei die Komponente g) ausgewählt ist unter Ethylenglykoldi(meth)acrylat, Pentaerythrittriallylether und Mischungen davon.

30. Verfahren nach einer der Ausführungsformen 25 bis 29, wobei die Polymerisation als Lösungspolymerisation erfolgt.

31. Verfahren nach Ausführungsform 30, wobei die Polymerisation in einem Lösungsmittel gestartet wird, das Isopropanol enthält und wobei im Verlauf der Polymerisation Propylenglycol zugegeben wird oder Isopropanol zumindest teilweise durch Propylenglycol ersetzt wird.

32. Copolymerzusammensetzung A), erhältlich durch ein Verfahren, wie in einer der Ausführungsformen 1 bis 31 definiert.

33. Kosmetisches Mittel, enthaltend

A) wenigstens eine Copolymerzusammensetzung, erhältlich durch ein Verfahren, wie in einer der Ausführungsformen 1 bis 31 definiert,

B) wenigstens einen kosmetisch akzeptablen Wirkstoff und

C) gegebenenfalls wenigstens einen weiteren, von A) und B) verschiedenen kosmetisch akzeptablen Hilfsstoff.

34. Verwendung einer Copolymerzusammensetzung A), erhältlich durch ein Verfahren, wie in einer der Ausführungsformen 1 bis 31 definiert, als Hilfsstoff in der Kosmetik, als Hilfsstoff in der Pharmazie, als Hilfsstoff in der Lebensmittelindustrie, als oberflächenaktive Verbindung, als oder in Klebemittel(n) sowie als oder in Beschichtungsmittel(n) für die Textil-, Papier-, Druck- und Lederindustrie.

35. Verwendung einer Copolymerzusammensetzung A), erhältlich durch ein Verfahren, wie in einer der Ausführungsformen 16 bis 19 definiert, als filmbildendes Polymer in der Kosmetik oder Pharmazie, insbesondere als Festigerpolymer in der Haarkosmetik.

36. Verwendung einer Copolymerzusammensetzung A), erhältlich durch ein Verfahren, wie in einer der Ausführungsformen 20 bis 24 definiert, als grenzflächenaktive Verbindung, insbesondere als Polymeremulgator oder Polymerstabilisator.

37. Verwendung einer Copolymerzusammensetzung A), erhältlich durch ein Verfahren, wie in einer der Ausführungsformen 25 bis 31 definiert, als Verdickungsmittel.

BESCHREIBUNG DER ERFINDUNG

Die Herstellung der erfindungsgemäßen Copolymerzusammensetzungen A) erfolgt vorzugsweise durch LösungspolymerisationDie Polymerisation erfolgt in einem Lösungsmittel, das wenigstens einen Alkohol umfasst.Die Polymerisation erfolgt zudem in einem im Wesentlichen wasserfreien Lösungsmittel.

Das erfindungsgemäße Verfahren selbst zeichnet sich durch vorteilhafte Eigenschaften aus und führt zudem auch zu Copolymerzusammensetzungen mit besonders vorteilhaften Eigenschaften.

Die nach dem erfindungsgemäßen Verfahren erhältlichen im Wesentlichen unvernetzten Lösungspolymerisate eignen sich in besonders vorteilhafter Weise für einen Einsatz als Haarfestigerpolymer. Sie bilden klare, glatte Filme, die dem Haar einen guten Halt verleihen und gleichzeitig gut auswaschbar sind. Sie zeichnen sich durch eine hohe Biegesteifigkeit aus. Die nach dem erfindungsgemäßen Verfahren erhältlichen Lösungspolymerisate, die neben AMPS und gegebenenfalls Acrylsäure noch wenigstens ein assoziatives Monomer einpolymerisiert enthalten eignen sich in besonders vorteilhafter Weise für einen Einsatz als polymere oberflächenaktive Verbindung (Polymertensid). Diese Copolymere sind vorzugsweise unvernetzt. Des Weiteren enthalten diese Copolymere vorzugsweise keine N-Vinyllactame, wie N-Vinylpyrrolidon oder N-Vinylcaprolactam, einpolymerisiert. Die nach dem erfindungsgemäßen Verfahren erhältlichen vernetzten Lösungspolymerisate eignen sich in besonders vorteilhafter Weise für einen Einsatz zur Einstellung der rheologischen Eigenschaften diverser Produkte, z. B. von Shampoos.

Im Rahmen der vorliegenden Erfindung wird zur Charakterisierung der Eigenschaften der eingesetzten Tenside unter Anderem der HLB-Wert (hydrophilic lipophilic balance) verwendet. Eine Definition des HLB-Werts findet sich in W. C. Griffin, J. Soc. Cosmetic Chem., Band 5, 249 (1954). Für solche nichtionische sowie für ionische Tensiden, für die der HLB-Wert nicht berechnet werden kann, gelingt vielfach eine experimentelle Bestimmung durch die Emulsionsvergleichsmethode. Der HLB-Wert des unbekannten Tensids errechnet sich nach der Formel:

$$HLB = \frac{R - (H \times S)}{N}$$

R = der erforderliche HLB-Wert des Öls, der bekannt sein muss,
H = der HLB-Wert des bekannten Tensids,
S = Gewichtsprozent des bekannten Tensids, ausgedrückt als Dezimalzahl (z. B. 45 % = 0,45),
N = Gewichtsprozent des Tensids, dessen HLB ermittelt werden soll, ausgedrückt als Dezimalzahl.

HLB-Werte und Verfahren zu ihrer Bestimmung sind in Standardwerken beschrieben, z. B. K. H. Schrader, Grundlagen und Rezepturen der Kosmetika, Hüthig Verlag, 2. Aufl., 1989.

Im Rahmen der vorliegenden Erfindung umfasst der Ausdruck Alkyl geradkettige und verzweigte Alkylgruppen.

Dazu zählen vorzugsweise geradkettige oder verzweigte $C_1$-$C_{30}$-Alkylgruppen, besonders bevorzugt $C_1$-$C_{20}$-Alkylgruppen. Geeignete kurzkettige Alkylgruppen sind z. B. geradkettige oder verzweigte $C_1$-$C_7$-Alkyl-, bevorzugt $C_1$-$C_6$-Alkyl- und besonders bevorzugt $C_1$-$C_4$-Alkylgruppen. Dazu zählen insbesondere Methyl, Ethyl, Propyl, Isopropyl, n-Butyl, 2-Butyl, sec.-Butyl, tert.-Butyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, etc.

Geeignete längerkettige $C_8$-$C_{30}$-Alkyl- bzw. $C_8$-$C_{30}$-Alkenylgruppen sind geradkettige und verzweigte Alkyl- bzw. Alkenylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen, bzw. um überwiegend lineare Alkenylreste, wie sie auch in natürlichen oder synthetischen Fettsäuren und Fettalkoholen sowie Oxoalkoholen vorkommen, die einfach, zweifach oder mehrfach ungesättigt sein können. Geeignete längerkettige $C_8$-$C_{30}$-Alkylgruppen sind z. B. n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, Arachinyl, Behenyl, Lignocerinyl, Melissinyl, etc. Geeignete längerkettige $C_8$-$C_{30}$-Alkenylgruppen umfassen z. B. n-Octenyl, n-Nonenyl, n-Decenyl, n-Undecenyl, n-Dodecenyl, n-Tridecenyl, n-Tetradecenyl, n-Pentadecenyl, n-Hexadecenyl, n-Heptadecenyl, n-Octadecenyl, n-Nonadecenyl, n-Eicosenyl, n-Docosenyl, n-Tetracosenyl, Hexacosenyl, Triacontenyl, etc.

Cycloalkyl steht vorzugsweise für $C_5$-$C_8$-Cycloalkyl, wie Cyclopentyl, Cyclohexyl, Cycloheptyl oder Cyclooctyl.

Aryl umfasst unsubstituierte und substituierte Arylgruppen und steht vorzugsweise für Phenyl, Tolyl, Xylyl, Mesityl, Naphthyl, Fluorenyl, Anthracenyl, Phenanthrenyl, Naphthacenyl und insbesondere für Phenyl, Tolyl, Xylyl oder Mesityl.

Im Folgenden werden Verbindungen, die sich von Acrylsäure und Methacrylsäure ableiten können, teilweise verkürzt durch Einfügen der Silbe "(meth)" in die von der Acrylsäure abgeleitete Verbindung bezeichnet.

Unter wasserlöslichen Monomeren und Polymeren werden im Rahmen der vorliegenden Erfindung Monomere und Polymere verstanden, die sich zu mindestens 1 g/l bei 20 °C in Wasser lösen. Unter wasserdispergierbaren Monomeren und Polymeren werden Monomere und Polymere verstanden, die unter Anwendung von Scherkräften, beispielsweise durch Rühren, in dispergierbare Partikel zerfallen. Hydrophile Monomere sind vorzugsweise wasserlöslich oder zumindest wasserdispergierbar.

<u>Monomer a)</u>

Als Monomer a) wird erfindungsgemäß eine neutralisierte 2-Acrylamido-2-methylpropansulfonsäure eingesetzt. Dazu wird die 2-Acrylamido-2-methylpropansulfonsäure vor ihrem Einsatz in der Polymerisation mit einer Neutralisation mit einer Aminkomponente AM) unterzogen, die aus Aminen ausgewählt ist, die ausschließlich sekundäre und/oder tertiäre Aminogruppen aufweisen.

Vorzugsweise ist die Aminkomponente AM) zur Neutralisation der 2-Acrylamido-2-methylpropansulfonsäure ausgewählt unter Aminen der Formeln $HNR^cR^d$ und $NR^cR^d$-$R^e$, worin die Reste $R^c$, $R^d$ und $R^e$ ausgewählt sind unter $C_1$- bis $C_{20}$-Alkyl-, $C_3$- bis $C_8$-Cycloalkyl-, $C_1$- bis $C_{20}$-Alkoxy-, Aryl- und Heteroarylresten. Dabei können die Alkyl- und Alkoxygruppen in Abhängigkeit von ihrer Kettenlänge durch 1 bis 10 nicht benachbarte Heteroatome, vorzugsweise ausgewählt unter N und O, unterbrochen sein, wobei die N-Heteroatome wiederum jeweils einen Substituenten, vorzugsweise ausgewählt unter $C_1$-$C_6$-Alkyl, $C_3$-$C_8$-Cycloalkyl, $C_6$-$C_{14}$-Aryl und Heteroaryl, tragen können. Weiterhin können $R^c$ und $R^d$ gemeinsam mit dem N-Atom, an das sie gebunden sind, einen 5-, 6-oder 7-gliedrigen Zyklus bilden, der ein oder zwei weitere Heteroatome, ausgewählt unter N und O aufweisen und/oder mit einem, zwei oder drei $C_1$- bis $C_6$-Alkylresten substituiert sein kann. Aryl- und Heteroarylreste weisen gegebenenfalls einen bis drei Substituenten, ausgewählt z. B. unter Hydroxy und den vorgenannten Alkyl-, Cycloalkyl- oder Alkoxyresten und Polyisobutenresten, auf.

Geeignete Reste $R^c$, $R^d$ und $R^e$ sind beispielsweise Wasserstoff, Methyl, Ethyl, n-Propyl, Isopropyl, n-Butyl, iso-Butyl, sek.-Butyl, tert.-Butyl, n-Pentyl und n-Hexyl, Cyclopentyl, Cyclohexyl, Phenyl, Tolyl, Xylyl, Naphthyl, Tetrahydrofuranyl, Tetrahydropyranyl, Dioxanyl, Pyrrolidyl, Piperidyl, Pyridyl und Pyrimidyl.

Geeignete Verbindungen der Formel $HNR^cR^d$, die ausschließlich eine sekundäre Aminofunktion aufweisen, sind beispielweise Dimethylamin, Diethylamin, Methylethylamin, Di-n-propylamin, Diisopropylamin, Diisobutylamin, Di-sek.-butylamin, Di-tert.-butylamin, Dipentylamin, Dihexylamin, Dicyclopentylamin, Dicyclohexylamin und Diphenylamin.

Geeignete Verbindungen der Formel $HNR^cR^d$, die ausschließlich eine sekundäre Aminofunktion aufweisen und bei denen der Rest $R^c$ und $R^d$ für durch das Heteroatom O unterbrochene und/oder substituierte Alkylreste steht, sind beispielweise $(CH_3\text{-O-}C_2H_4)_2NH$, $(C_2H_5\text{-O-}C_2H_4)_2NH$, $(CH_3\text{-O-}C_3H_6)_2NH$, $(C_2H_5\text{-O-}C_3H_6)_2NH$, $(n\text{-}C_4H_9\text{-O-}C_4H_8)_2NH$, $(HO\text{-}C_2H_4)_2NH$, $(HO\text{-}C_3H_6)_2NH$ und $(HO\text{-}C_4H_8)_2NH$.

Geeignete Verbindungen der Formel $HNR^cR^d$, bei denen $R^c$ und $R^d$ gemeinsam mit dem N-Atom, an das sie gebunden sind, einen 5-, 6- oder 7-gliedrigen Zyklus bilden, der ein oder zwei Heteroatome, ausgewählt unter N und

O, aufweisen und mit einem, zwei oder drei $C_1$-bis $C_6$-Alkylresten substituiert sein kann, sind beispielsweise Pyrrolidin, Piperidin, Morpholin und Piperazin sowie deren substituierte Derivate, wie N-$C_1$-bis $C_6$-Alkylpiperazine und Dimethylmorpholin.

Zur Neutralisation geeignete cyclische Amine, die wenigstens eine tertiäre Aminofunktion aufweisen, sind z. B. N-Alkylimidazole. Bevorzugt ist N-Methylimidazol.

Ein bevorzugtes N-(Di-($C_1$-$C_4$)-Alkylamino-($C_1$-$C_8$)-alkyl)imidazol ist N-(3-Dimethyl-aminopropyl)imidazol.

Zur Neutralisation geeignete cyclische Amine, die eine sekundäre und eine tertiäre Aminofunktion aufweisen, sind z. B. N-Alkylpiperazine und N-Hydroxyalkylpiperazine. Bevorzugt ist 1-(2-Hydroxyethyl)-piperazin.

Zur Neutralisation geeignete Verbindungen mit mindestens einer tertiären Aminogruppe sind z. B. Trialkanolamine, wie Triethanolamin, Tri-n-propanolamin, Tri-n-butanol-amin, Methyldiethanolamin, Ethyldiethanolamin, N,N-Dimethylethanolamin, N,N-Dimethylpropanolamin, N,N-Diethylethanolamin, etc. Herstellungsbedingt können Trialkanolamine als weitere Komponente(n) das entsprechende Dialkanolamin und/oder Monoalkanolamin enthalten. Eine solche Mischung eignet sich ebenfalls in dem erfindungsgemäßen Verfahren.

Vorzugsweise ist die Aminkomponente AM) ausgewählt ist unter N-Vinylimidazolverbindungen (Monomer b), N-($C_1$-$C_8$)-Alkylimidazolen, N-(Di-($C_1$-$C_4$)-Alkylamino-($C_1$-$C_8$)-alkyl)imidazolen, N-($C_1$-$C_8$)-Alkylpiperazinen, N-Hydroxyalkylpiperazinen, Alkyldialkanolaminen, Trialkanolaminen und Mischungen davon.

Vorzugsweise ist die Aminkomponente AM) ausgewählt ist unter N-Vinylimidazolverbindungen (Monomer b), N-($C_1$-$C_8$)-Alkylimidazolen und Mischungen davon.

Besonders bevorzugt umfasst die Aminkomponente AM) wenigstens ein N-($C_1$-$C_8$)-Alkylimidazol umfasst oder aus wenigstens einem N-($C_1$-$C_8$)-Alkylimidazol besteht.

Insbesondere ist die Aminkomponente AM) ausgewählt unter N-Vinylimidazol, N-Methylimidazol, Methyldiethanolamin, Triethanolamin und Mischungen davon.

Bevorzugt weist eine 1 gew.-%ige Lösung der neutralisierten 2-Acrylamido-2-methylpropansulfonsäure in Wasser bei 20 °C einen pH-Wert von größer als 1,5, bevorzugt von größer als 2,5 auf. Bevorzugt liegt der pH-Wert in einem Bereich von 1,5 bis 6,5.

Bevorzugt enthält die zur Herstellung der Copolymerzusammensetzung A) eingesetzte Monomerzusammensetzung das Monomer a) in einer Menge von 1 bis 99 Gew.-%, vorzugsweise 2 bis 95 Gew.-%, besonders bevorzugt 3 bis 70 Gew.-%, bezogen auf das Gesamtgewicht der zur Polymerisation eingesetzten Monomere, eingesetzt (d. h. Monomere a) und, soweit vorhanden, Monomere b), c), d), e), f), g) und h) addieren sich zu 100 Gew.-%). Die Gewichtsangabe bezieht sich dabei auf das Monomer a) in seiner nicht neutralisierten Form. Wenn die zur Neutralisation der 2-Acrylamido-2-methylpropansulfonsäure a) eingesetzte Aminkomponente AM) ein basisches Monomer, speziell eine N-Vinylimidazolverbindung (= Monomer b), enthält, so wird dieses bei der mit seinem Gewichtsanteil bei der Bestimmung des Gesamtgewichts der zur Polymerisation eingesetzten Monomere berücksichtigt.

<u>Monomer b)</u>

In einer bevorzugten Ausführungsform enthält die zur Herstellung der Copolymerzusammensetzung a) eingesetzte Monomerzusammensetzung wenigstens eine N-Vinylimidazol-Verbindung b) der allgemeinen Formel (I)

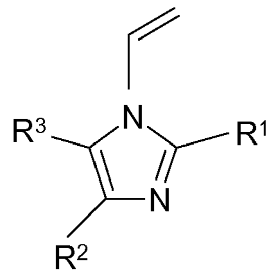

(I)

worin $R^1$ bis $R^3$ unabhängig voneinander für Wasserstoff, $C_1$-$C_4$-Alkyl oder Phenyl stehen.

Beispiele für Verbindungen der allgemeinen Formel (I) sind folgender Tabelle 1 zu entnehmen:

Tabelle 1

| **$R^1$** | **$R^2$** | **$R^3$** |
|---|---|---|
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |
| Me = Methyl<br>Ph = Phenyl | | |

Bevorzugt als Monomer b) ist 1-Vinylimidazol (N-Vinylimidazol) und sind Mischungen, die N-Vinylimidazol enthalten.

Soweit vorhanden, wird die Komponente b) in einer Menge von 0,01 bis 50 Gew.-%, vorzugsweise 0,1 bis 40 Gew.-%, besonders bevorzugt 1 bis 35 Gew.-%, bezogen auf das Gesamtgewicht der zur Polymerisation eingesetzten Monomere, eingesetzt (d. h. Monomere a) und, soweit vorhanden, Monomere b), c), d), e), f), g) und h) addieren sich zu 100 Gew.-%). Die Komponente b) wird dabei mit ihrem Gewichtsanteil dem Gesamtgewicht der zur Polymerisation eingesetzten Monomere zugerechnet, auch wenn sie teilweise oder vollständig als Komponente AM) zur Neutralisation des AMPS (= Monomer a) eingesetzt wird.

<u>Amidgruppenhaltiges Monomer c)</u>

Im Rahmen der vorliegenden Erfindung umfasst der Begriff "amidgruppenhaltiges Monomer" auch harnstoffgruppenhaltige Monomere.

Vorzugsweise wird die Komponente c) in einer Menge von 0 bis 90 Gew.-%, besonders bevorzugt 0,1 bis 85 Gew.-%, insbesondere 1 bis 80 Gew.-%, bezogen auf das Gesamtgewicht der zur Polymerisation eingesetzten Monomere eingesetzt (d. h. Monomere a) und, soweit vorhanden, Monomere b), c), d), e), f), g) und h) addieren sich zu 100 Gew.-%).

Bevorzugt enthält die zur Herstellung der Copolymerzusammensetzung A) eingesetzte Monomerzusammensetzung wenigstens ein amidgruppenhaltiges Monomer c), das ausgewählt ist unter $\alpha,\beta$-ethylenisch ungesättigten amidgruppenhaltigen Verbindungen der allgemeinen Formel (II)

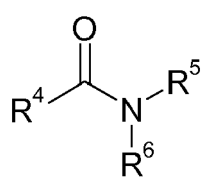

(II)

wobei

einer der Reste $R^4$ bis $R^6$ für eine Gruppe der Formel $CH_2$=$CR^7$- mit $R^7$ = H oder $C_1$-$C_4$-Alkyl steht und die übrigen Reste $R^4$ bis $R^6$ unabhängig voneinander für H, Alkyl, Cycloalkyl, Heterocycloalkyl, Aryl oder Hetaryl stehen,

wobei $R^5$ und $R^6$ gemeinsam mit dem Stickstoffatom, an das sie gebunden sind, auch für einen fünf- bis siebengliedrigen Heterocyclus stehen können.

Bevorzugt sind die Monomere c) der Formel (II) ausgewählt unter primären Amiden α,β-ethylenisch ungesättigter Monocarbonsäuren, N-Vinylamiden gesättigter Monocarbonsäuren, N-Vinyllactamen, N-Alkyl- und N,N-Dialkylamiden α,β-ethylenisch ungesättigter Monocarbonsäuren und Mischungen davon.

Geeignete Monomere c) sind weiterhin Acrylsäureamid und Methacrylsäureamid.

Geeignete Monomere c) sind weiterhin Methyl(meth)acrylamid, Methylethacrylamid, Ethyl(meth)acrylamid, Ethylethacrylamid, n-Propyl(meth)acrylamid, Isopropyl(meth)-acrylamid, n-Butyl(meth)acrylamid, tert.-Butyl(meth)acrylamid, tert.-Butylethacrylamid, n-Pentyl(meth)acrylamid, n-Hexyl(meth)acrylamid, n-Heptyl(meth)acrylamid, n-Octyl(meth)acrylamid,1,1,3,3-Tetramethylbutyl(meth)acrylamid, Ethylhexyl(meth)acrylamid, n-Nonyl(meth)acrylamid, n-Decyl(meth)acrylamid, n-Undecyl(meth)acrylamid, Tridecyl(meth)acrylamid, Myristyl(meth)acrylamid, Pentadecyl(meth)acrylamid, Palmityl(meth)acrylamid, Heptadecyl(meth)acrylamid, Nonadecyl(meth)acrylamid, Arrachinyl(meth)acrylamid, Behenyl(meth)acrylamid, Lignocerenyl(meth)acrylamid, Cerotinyl(meth)acrylamid, Melissinyl(meth)acrylamid, Palmitoleinyl(meth)acrylamid, Oleyl(meth)acrylamid, Linolyl(meth)acrylamid, Linolenyl(meth)acrylamid, Stearyl(meth)acrylamid, Lauryl(meth)acrylamid, N-Methyl-N-(n-octyl)(meth)acrylamid, N,N-Dimethyl(meth)acrylamid, N,N-Diethyl(meth)acrylamid, N,N-Di-(n-octyl)(meth)acrylamid und Mischungen davon.

Geeignete Monomere c) sind weiterhin Piperidinyl(meth)acrylamid, Morpholinyl(meth)-acrylamid und Mischungen davon.

Anionogenes/anionisches Monomer d)

In dem erfindungsgemäßen Verfahren zur Herstellung der Copolymerzusammensetzung A) kann gegebenenfalls als Komponente d) eine von der 2-Acrylamido-2-methylpropansulfonsäure a) verschiedene Verbindung mit einer radikalisch polymerisierbaren, α,β-ethylenisch ungesättigten Doppelbindung und mindestens einer anionogenen und/oder anionischen Gruppe pro Molekül eingesetzt werden.

Vorzugsweise sind die Verbindungen d) ausgewählt unter monoethylenisch ungesättigten Carbonsäuren, Phosphonsäuren und Mischungen davon.

Zu den Monomeren d) zählen monoethylenisch ungesättigte Mono- und Dicarbonsäuren mit 3 bis 25 vorzugsweise 3 bis 6 C-Atomen, die auch in Form ihrer Salze oder Anhydride eingesetzt werden können. Beispiele hierfür sind Acrylsäure, Methacrylsäure, Ethacrylsäure, α-Chloracrylsäure, Crotonsäure, Maleinsäure, Maleinsäureanhydrid, Itaconsäure, Citraconsäure, Mesaconsäure, Glutaconsäure, Aconitsäure und Fumarsäure. Zu den Monomeren d) zählen weiterhin die Halbester von monoethylenisch ungesättigten Dicarbonsäuren mit 4 bis 10 vorzugsweise 4 bis 6 C-Atomen, z. B. von Maleinsäure wie Maleinsäuremonomethylester. Zu den Monomeren d) zählen auch monoethylenisch ungesättigte Sulfonsäuren und Phosphonsäuren, beispielsweise Sulfoethylacrylat, Sulfoethylmethacrylat, Sulfopropylacrylat, Sulfopropylmethacrylat, Vinylphosphonsäure und Allylphosphonsäure. Zu den Monomeren d) zählen auch die Salze der zuvor genannten Säuren, insbesondere die Natrium-, Kalium- und Ammoniumsalze sowie die Salze mit Aminen. Die Monomere d) können als solche oder als Mischungen untereinander eingesetzt werden. Die angegebenen Gewichtsanteile beziehen sich sämtlich auf die Säureform.

Vorzugsweise ist die Komponente d) ausgewählt unter Acrylsäure, Methacrylsäure, Ethacrylsäure, α-Chloracrylsäure, Crotonsäure, Maleinsäure, Maleinsäureanhydrid, Fumarsäure, Itaconsäure, Citraconsäure, Mesaconsäure, Glutaconsäure, Aconitsäure und Mischungen davon.

Die zur Herstellung der erfindungsgemäßen Copolymerzusammensetzungen A) eingesetzte Komponente d) ist vorzugsweise ausgewählt unter Acrylsäure, Salzen der Acrylsäure, Methacrylsäure, Salzen der Methacrylsäure und Mischungen davon.

In einer ersten bevorzugten Ausführungsform besteht die Komponente a) aus Acrylsäure oder aus wenigstens einem Salz der Acrylsäure oder aus einer Mischung aus Acrylsäure und wenigstens einem Salz der Acrylsäure.

In einer zweiten bevorzugten Ausführungsform besteht die Komponente a) aus einer Mischung aus Acrylsäure, Methacrylsäure und/oder wenigstens einem Salz der (Meth)acrylsäure.

Die Komponente d) wird vorzugsweise in einer Menge von 0 bis 95 Gew.-%, besonders bevorzugt 0,1 bis 90 Gew.-%, insbesondere 1 bis 75 Gew.-%, speziell 5 bis 50 Gew.-%, bezogen auf das Gesamtgewicht der zur Polymerisation eingesetzten Monomere, eingesetzt (d. h. Monomere a) und, soweit vorhanden, Monomere b), c), d), e), f), g) und h) addieren sich zu 100 Gew.-%).

Ethergruppenhaltiges Monomer e)

Zur Herstellung der erfindungsgemäßen Copolymerzusammensetzung A) kann zusätzlich wenigstens ein von den Komponenten a) bis e) verschiedenes, damit copolymerisierbares ethergruppenhaltiges Monomer e) eingesetzt werden.

Geeignete Monomere e) sind ausgewählt unter Verbindungen der allgemeinen Formeln (III)

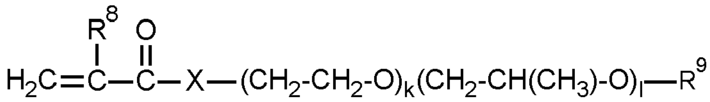

(III)

worin
die Reihenfolge der Alkylenoxideinheiten in den Verbindungen (III) beliebig ist,

- k und l unabhängig voneinander für eine ganze Zahl von 0 bis 1000 stehen, wobei die Summe aus k und l mindestens 5 beträgt,
- $R^8$ für Wasserstoff oder $C_1$-$C_4$-Alkyl, vorzugsweise Methyl, steht,
- $R^9$ für Wasserstoff, $C_1$-$C_{30}$-Alkyl, $C_2$-$C_{30}$-Alkenyl oder $C_5$-$C_8$-Cycloalkyl steht,
- X für O oder eine Gruppe der Formel $NR^{10}$ steht, worin $R^{10}$ für H, Alkyl, Alkenyl, Cycloalkyl, Heterocycloalkyl, Aryl oder Hetaryl steht.

In der Formel (III) steht k vorzugsweise für eine ganze Zahl von 1 bis 500, insbesondere 3 bis 250. Bevorzugt steht l für eine ganze Zahl von 0 bis 100.

Bevorzugt steht $R^8$ in der Formel (III) für Wasserstoff, Methyl, Ethyl, n-Propyl, Isopropyl, n-Butyl, sec.-Butyl oder tert.-Butyl. Insbesondere steht $R^8$ in den Formeln IV a) und IV b) für Wasserstoff, Methyl oder Ethyl.

Vorzugsweise steht $R^9$ für Wasserstoff, Methyl, Ethyl, n-Propyl, Isopropyl, n-Butyl, sec.-Butyl, n-Pentyl, n-Hexyl, n-Heptyl, n-Octyl, 1,1,3,3-Tetramethylbutyl, Ethylhexyl, n-Nonyl, n-Decyl, n-Undecyl, Tridecyl, Myristyl, Pentadecyl, Palmityl, Heptadecyl, Octadecyl, Nonadecyl, Arachinyl, Behenyl, Lignocerenyl, Cerotinyl, Melissinyl, Palmitoleinyl, Oleyl, Linolyl, Linolenyl, Stearyl, Lauryl.

Bevorzugt steht $R^9$ für Cetyl (Hexadecyl), Stearyl (Octadecyl) oder für Cetearyl, d. h. ein Gemisch aus Cetyl und Stearyl.

Vorzugsweise steht X in der Formel (III) a) für O oder NH.

Geeignete Polyetheracrylate (III) sind z. B. die Polykondensationsprodukte der zuvor genannten $\alpha,\beta$-ethylenisch ungesättigten Mono- und/oder Dicarbonsäuren und deren Säurechloriden, -amiden und Anhydriden mit Polyetherolen. Geeignete Polyetherole können leicht durch Umsetzung von Ethylenoxid, 1,2-Propylenoxid und/oder Epichlorhydrin mit einem Startermolekül, wie Wasser oder einem kurzkettigen Alkohol $R^9$-OH hergestellt werden. Die Alkylenoxide können einzeln, alternierend nacheinander oder als Mischung eingesetzt werden. Die Polyetheracrylate III a) können allein oder in Mischungen zur Herstellung der erfindungsgemäß eingesetzten Polymere verwendet werden.

Bevorzugt wird zur Herstellung der Copolymerzusammensetzung A) ein Monomer e) der Formel (III.1)

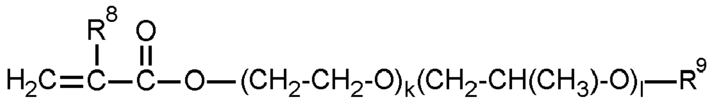

(III.1)

eingesetzt, worin
die Reihenfolge der Alkylenoxideinheiten beliebig ist,

- k und l unabhängig voneinander für eine ganze Zahl von 0 bis 100 stehen, wobei die Summe aus k und l mindestens 5 beträgt,

$R^8$ für Wasserstoff oder Methyl steht,

$R^9$ für $C_8$-$C_{30}$-Alkyl steht.

Bevorzugt steht in den Verbindungen der Formel (III.1) k für eine ganze Zahl von 5 bis 100 und l für 0.
In einer speziellen Ausführung steht in den Verbindungen der Formel (III.1) $R^9$ für Cetyl, Stearyl oder für Cetearyl.
Eine Mischung eines Stearylpolyethylenglykol-1100-methacrylats (25%ig) mit Methylmethacrylat ist kommerziell unter der Bezeichnung Plex-6877-O erhältlich. Eine Mischung eines $C_{16}$-$_{18}$-Alkyl-polyethylenglykolmethacrylats mit Methacrylsäure ist kommerziell unter der Bezeichnung Lutencryl® 250 ($C_{16-18}$ Alkyl-PEG-Methacrylat in Methacrylsäure (50%ig)) erhältlich.
Soweit vorhanden, wird die Komponente e) in einer Menge von 0,01 bis 25 Gew.-%, vorzugsweise 0,1 bis 20 Gew.-%, besonders bevorzugt 0,2 bis 15 Gew.-%, bezogen auf das Gesamtgewicht der zur Polymerisation eingesetzten Verbindungen, eingesetzt (d. h. die Monomere a), Monomere b), Monomere c) und, soweit vorhanden, Monomere d), e), f) und g) addieren sich zu 100 Gew.-%).

Kationogenes Monomer f)

Die zur Herstellung der Copolymerzusammensetzung A) eingesetzte Monomerzusammensetzung kann wenigstens eine von den N-Vinylimidazol-Verbindungen b) verschiedene Verbindung f) mit einer radikalisch polymerisierbaren, $\alpha,\beta$-ethylenisch ungesättigten Doppelbindung und wenigstens einer kationogenen Gruppen pro Molekül enthalten.
Soweit vorhanden, wird die Komponente f) in einer Menge von 0,01 bis 50 Gew.-%, vorzugsweise 0,1 bis 40 Gew.-%, besonders bevorzugt 0,2 bis 25 Gew.-%, bezogen auf das Gesamtgewicht der zur Polymerisation eingesetzten Monomere, eingesetzt (d. h. Monomere a) und, soweit vorhanden, Monomere b), c), d), e), f), g) und h) addieren sich zu 100 Gew.-%).
Bevorzugt handelt es sich bei den kationogenen Gruppen der Komponente f) um stickstoffhaltige Gruppen, wie primäre, sekundäre und tertiäre Aminogruppen sowie quaternäre Ammoniumgruppen. Vorzugsweise handelt es sich bei den stickstoffhaltigen Gruppen um tertiäre Aminogruppen oder quaternäre Ammoniumgruppen.
In einer speziellen Ausführungsform werden die Verbindungen der Komponente f) teilweise oder vollständig neutralisiert. Dazu können Verbindungen der Komponente f) mit wenigstens einem säuregruppenhaltigen Monomer, wie AMPS, Acrylsäure, Methacrylsäure, etc. zumindest teilweise neutralisiert werden. Eine solche Protonierung kann im Allgemeinen sowohl vor als auch während der Polymerisation erfolgen.
Bevorzugte Monomere f) sind N-Methylaminoethyl(meth)acrylat, N-Ethylaminoethyl(meth)acrylat, N-(n-Propyl)aminoethyl(meth)acrylat, N-(tert.-Butyl)aminoethyl(meth)acrylat, N,N-Dimethylaminomethyl(meth)acrylat, N,N-Dimethylaminoethyl(meth)acrylat, N,N-Diethylaminomethyl(meth)acrylat, N,N-Diethylaminoethyl(meth)acrylat, N,N-Dimethylaminopropyl(meth)acrylat, N,N-Diethylaminopropyl(meth)acrylat und N,N-Dimethylaminocyclohexyl(meth)acrylat.
Besonders bevorzugt sind N,N-Dimethylaminoethylacrylat,
N,N-Dimethylaminoethylmethacrylat und Mischungen davon.
Geeignete Monomere f) sind weiterhin die Amide der zuvor genannten $\alpha,\beta$-ethylenisch ungesättigten Mono- und Dicarbonsäuren mit Diaminen, welche mindestens eine primäre oder sekundäre Aminogruppe aufweisen. Bevorzugt sind Diamine, die eine tertiäre und eine primäre oder sekundäre Aminogruppe aufweisen.
Bevorzugt als Monomere f) sind z. B. N-[tert.-Butylaminoethyl](meth)acrylamid, N-[2-(Dimethylamino)ethyl]acrylamid, N-[2-(Dimethylamino)ethyl]methacrylamid, N-[3-(Dimethylamino)propyl]acrylamid, N-[3-(Dimethylamino)propyl]methacrylamid, N-[4-(Dimethylamino)butyl]acrylamid, N-[4-(Dimethylamino)butyl]methacrylamid, N-[2-(Diethylamino)ethyl]acrylamid, N-[4-(Dimethylamino)cyclohexyl]acrylamid und N-[4-(Dimethylamino)cyclohexyl]methacrylamid.
In einer geeigneten Ausführungsform umfasst die Komponente f) als vinylsubstituierte heteroaromatische Verbindung wenigstens eine Verbindung der Formel

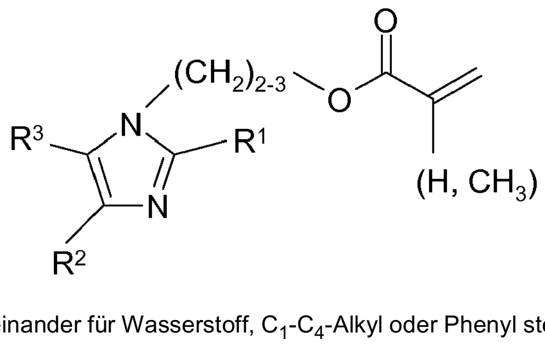

worin $R^1$ bis $R^3$ unabhängig voneinander für Wasserstoff, $C_1$-$C_4$-Alkyl oder Phenyl stehen. Bevorzugt stehen $R^1$ bis $R^3$

für Wasserstoff.

Geeignete Monomere f) sind weiterhin von Vinylimidazolen b) verschiedene vinyl- und allylsubstituierte Stickstoffheterocyclen, wie 2- und 4-Vinylpyridin, 2- und 4-Allylpyridin, und die Salze davon.

Bevorzugt enthält die zur Herstellung der Copolymerzusammensetzung A) eingesetzte Monomerzusammensetzung als Monomer f) N-Vinylimidazol oder besteht aus N-Vinylimidazol.

Bevorzugt wird N-Vinylimidazol in einer Menge von 0,01 bis 10 Gew.-%, bevorzugt 0,1 bis 8 Gew.-%, bezogen auf das Gesamtgewicht der Monomerzusammensetzung, eingesetzt (d. h. Monomere a) und, soweit vorhanden, Monomere b), c), d), e), f), g) und h) addieren sich zu 100 Gew.-%).

<u>Vernetzer g)</u>

Zur Herstellung der Copolymerzusammensetzungen A) wird erfindungsgemäß wenigstens ein Vernetzer g), d. h. eine Verbindung mit zwei oder mehr als zwei ethylenisch ungesättigten, nichtkonjugierten Doppelbindungen, eingesetzt.

Vorzugsweise werden Vernetzer in einer Menge von 0,01 bis 5 Gew.-%, besonders bevorzugt 0,05 bis 4 Gew.-%, bezogen auf das Gesamtgewicht der zur Polymerisation eingesetzten Monomere eingesetzt (d. h. Monomere a) und, soweit vorhanden, Monomere b), c), d), e), f), g) und h) addieren sich zu 100 Gew.-%).

Geeignete Vernetzer g) sind zum Beispiel Acrylester, Methacrylester, Allylether oder Vinylether von mindestens zweiwertigen Alkoholen. Die OH-Gruppen der zugrunde liegenden Alkohole können dabei ganz oder teilweise verethert oder verestert sein; die Vernetzer enthalten aber mindestens zwei ethylenisch ungesättigte Gruppen.

Beispiele für die zugrunde liegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglykolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thiapentan-1,5-diol, sowie Polyethylenglykole, Polypropylenglykole und Polytetrahydrofurane mit Molekulargewichten von jeweils 200 bis 10000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrunde liegende Alkohole mit mehr als zwei OH-Gruppen sind Trimethylolpropan, Glycerin, Pentaerythrit, 1,2,5-Pentantriol, 1,2,6-Hexantriol, Cyanursäure, Sorbitane, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden. Bevorzugt sind Ethylenglykoldi(meth)acrylat und Polyethylenglykoldi(meth)acrylate.

Weitere geeignete Vernetzer g) sind die Vinylester oder die Ester einwertiger, ungesättigter Alkohole mit ethylenisch ungesättigten $C_3$-$C_6$-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellithsäure, Phthalsäure, Terephthalsäure, Zitronensäure oder Bernsteinsäure.

Weitere geeignete Vernetzer g) sind von (Meth)acrylestern verschiedene Ester ungesättigter Carbonsäuren mit den oben beschriebenen mehrwertigen Alkoholen, beispielsweise der Ölsäure, Crotonsäure, Zimtsäure oder 10-Undecensäure.

Geeignet als Vernetzer g) sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z. B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Als Vernetzer g) sind ferner geeignet die Acrylsäureamide, Methacrylsäureamide und N-Allylamine von mindestens zweiwertigen Aminen. Solche Amine sind zum Beispiel 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren, wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z. B. Triallylmethylammoniumchlorid oder -methylsulfat, als Vernetzer g) geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z. B.

N,N'-Divinylethylenharnstoff oder N,N'-Divinylpropylenharnstoff.

Weitere geeignete Vernetzer g) sind Divinyldioxan, Tetraallylsilan oder Tetravinylsilan.

Selbstverständlich können auch Mischungen der vorgenannten Verbindungen g) eingesetzt werden.

Ganz besonders bevorzugt als Vernetzer g) sind Ethylenglykoldi(meth)acrylat, Polyethylenglykoldi(meth)acrylate, Pentaerythrittriallylether, Methylenbisacrylamid, N,N'-Divinylethylenharnstoff, Triallylamin und Triallylmonoalkylammoniumsalze.

Weitere Monomere h)

In einer weiteren bevorzugten Ausführungsform kann die zur Herstellung der Copolymerzusammensetzung A) eingesetzte Monomerzusammensetzung zusätzlich wenigstens ein weiteres von den Monomeren a) bis g) verschiedenes Monomer h) einpolymerisiert enthalten.

Bevorzugt ist das weitere Monomer h) ausgewählt unter

h1) Estern α,β-ethylenisch ungesättigter Mono- und Dicarbonsäuren mit $C_1$-$C_{30}$-Alkanolen,
h2) Estern α,β-ethylenisch ungesättigter Mono- und Dicarbonsäuren mit $C_2$-$C_{30}$-Diolen,
h3) Amiden α,β-ethylenisch ungesättigter Mono- und Dicarbonsäuren mit $C_2$-$C_{30}$-Aminoalkoholen, die eine primäre oder sekundäre Aminogruppe aufweisen,
h4) amidgruppenhaltigen Monomeren, die eine Gruppe der Formeln (IVa) oder (IVb)

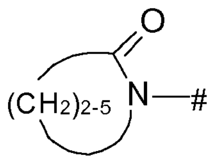
(IVa)

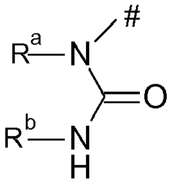
(IVb)

aufweisen, worin

| | |
|---|---|
| # | für die Bindungsstelle zu einer Gruppe mit einer radikalisch polymerisierbaren, α,β-ethylenisch ungesättigten Doppelbindung steht, wobei in den Verbindungen (IIIa) # nicht für die Bindungsstelle zu einer Gruppe der Formel $CH_2$=$CR^7$- mit $R^7$ = H oder $C_1$-$C_4$-Alkyl steht, |
| $R^a$ | für H oder $C_1$-$C_4$-Alkyl steht, |
| $R^b$ | für H oder $C_1$-$C_4$-Alkyl steht, oder |
| $R^a$ und $R^b$ | gemeinsam für $(CH_2)_{1-4}$ stehen, |

h5) Urethan(meth)acrylaten mit Alkylenoxidgruppen,
h6) $C_1$-$C_{30}$-Alkylvinylethern,
h7) Vinylaromaten,
h8) Vinylhalogeniden, Vinylidenhalogeniden.

und Mischungen davon.

Monomer h1)

Geeignete Ester α,β-ethylenisch ungesättigter Mono- und Dicarbonsäuren mit $C_1$-$C_{30}$-Alkanolen sind z. B. Methyl(meth)acrylat, Methylethacrylat, Ethyl(meth)acrylat, Ethylethacrylat, n-Propyl(meth)acrylat, Isopropyl(meth)acrylat, n-Butyl(meth)acrylat, tert.-Butyl(meth)acrylat, tert.-Butylethacrylat, n-Pentyl(meth)acrylat, n-Hexyl(meth)acrylat, n-Heptyl(meth)acrylat, n-Octyl(meth)acrylat,1,1,3,3-Tetramethylbutyl(meth)acrylat, Ethylhexyl(meth)acrylat, n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)acrylat, Heptadecyl(meth)acrylat, Nonadecyl(meth)acrylat, Arrachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat und Mischungen davon.

Die Monomere h1) eignen sich vorteilhaft zur Modifizierung der Eigenschaften der Copolymerzusammensetzung A). So zeichnen sich beispielsweise $C_1$-$C_4$-Alkyl(meth)-acrylate durch eine höhere Polarität und Wasserlöslichkeit aus als Alkyl(meth)acrylate mit mehr als 4 Kohlenstoffatomen im Alkylrest.

Bevorzugte Monomere h1) sind die Ester α,β-ethylenisch ungesättigter Mono- und Dicarbonsäuren mit $C_1$-$C_3$-Alkanolen, insbesondere Methylmethacrylat.

Bevorzugte Monomere h1) sind weiterhin die Ester α,β-ethylenisch ungesättigter Mono- und Dicarbonsäuren mit $C_8$-$C_{22}$-Alkanolen, insbesondere Stearyl(meth)acrylat, Lauryl(meth)acrylat und Mischungen davon.

In einer bevorzugten Ausführungsform ist das Monomer h1) ausgewählt unter $C_1$-$C_3$-Alkyl(meth)acrylaten, $C_8$-$C_{22}$-Alkyl(meth)acrylaten und Mischungen davon.

Sofern vorhanden, wird die Komponente h1) in einer Menge von 0,1 bis 40 Gew.-%, besonders bevorzugt 0,5 bis 20 Gew.-%, bezogen auf das Gesamtgewicht der zur Polymerisation eingesetzten Monomere, eingesetzt. (d. h. Monomere a), Monomere b), Monomere c) und, soweit vorhanden, Monomere d), e), f) und g) addieren sich zu 100 Gew.-%).

Monomer h2)

Geeignete zusätzliche Monomere h2) sind weiterhin 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat und 3-Hydroxy-2-ethylhexylmethacrylat.

Sofern vorhanden, wird die Komponente h2) in einer Menge von 0,1 bis 10 Gew.-%, bezogen auf das Gesamtgewicht der zur Polymerisation eingesetzten Monomere, eingesetzt.

Monomer h3)

Geeignete zusätzliche Monomere h3) sind 2-Hydroxyethylacrylamid, 2-Hydroxyethylmethacrylamid, 2-Hydroxyethylethacrylamid, 2-Hydroxypropylacrylamid, 2-Hydroxypropylmethacrylamid, 3-Hydroxypropylacrylamid, 3-Hydroxypropylmethacrylamid, 3-Hydroxybutylacrylamid, 3-Hydroxybutylmethacrylamid, 4-Hydroxybutylacrylamid, 4-Hydroxybutylmethacrylamid, 6-Hydroxyhexylacrylamid, 6-Hydroxyhexylmethacrylamid, 3-Hydroxy-2-ethylhexylacrylamid und 3-Hydroxy-2-ethylhexylmethacrylamid.

Sofern vorhanden, wird die Komponente h3) in einer Menge von 0,1 bis 10 Gew.-%, bezogen auf das Gesamtgewicht der zur Polymerisation eingesetzten Monomere, eingesetzt (d. h. Monomere a) und, soweit vorhanden, Monomere b), c), d), e), f), g) und h) addieren sich zu 100 Gew.-%).

Amidgruppenhaltige Monomere h4)

In einer weiteren bevorzugten Ausführungsform enthält die zur Herstellung der Copolymerzusammensetzung A) eingesetzte Monomerzusammensetzung wenigstens ein amidgruppenhaltiges Monomer c), das eine Gruppe der Formeln (IIIa) oder (IIIb)

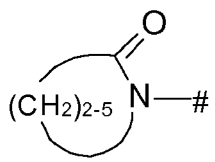
(IVa)

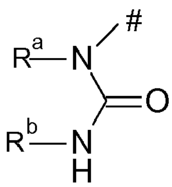
(IVb)

aufweist, worin

# für die Bindungsstelle zu einer Gruppe mit einer radikalisch polymerisierbaren, α,β-ethylenisch ungesättigten Doppelbindung steht, wobei in den Verbindungen (IIIa) # nicht für die Bindungsstelle zu einer Gruppe der Formel $CH_2$=$CR^7$- mit $R^7$ = H oder $C_1$-$C_4$-Alkyl steht,

$R^a$ für H oder $C_1$-$C_4$-Alkyl steht,

$R^b$ für H oder $C_1$-$C_4$-Alkyl steht, oder

$R^a$ und $R^b$ gemeinsam für $(CH_2)_{1-4}$ stehen.

Bevorzugt sind die Monomere c) der Formeln (IVa) oder (IVb) ausgewählt unter Monomeren mit einer Gruppe der Formeln (IVa.1) oder (IVb.1)

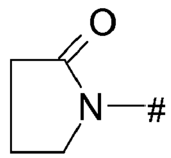

(IIIa.1)

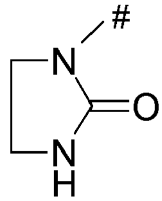

(IIIb.1)

Bevorzugt als Monomere h4) sind die Verbindungen der Formel:

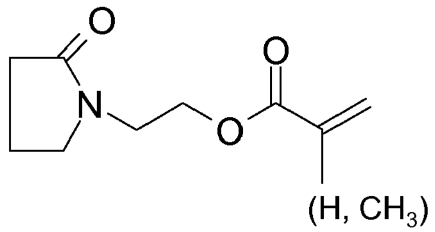

Geeignete Harnstoffgruppen aufweisende Monomere h4) sind z. B. N-Vinyl- oder N-Allylharnstoff oder Derivate des Imidazolidin-2-ons. Dazu zählen N-Vinyl- und N-Allylimidazolidin-2-on, N-Vinyloxyethylimidazolidin-2-on, N-(2-(Meth)acrylamidoethyl)imidazolidin-2-on, N-(2-(Meth)acryloxyethyl)imidazolidin-2-on (= 2-Ureido(meth)acrylat), N-[2-((Meth)acryloxyacetamido)ethyl]imidazolidin-2-on, etc.

Bevorzugte Harnstoffgruppen aufweisende Monomere h4) sind N-(2-Acryloxyethyl)imidazolidin-2-on und N-(2-Methacryloxyethyl)imidazolidin-2-on. Besonders bevorzugt ist N-(2-Methacryloxyethyl)imidazolidin-2-on (2-Ureidomethacrylat, UMA).

Vorzugsweise werden die amidgruppenhaltigen Monomere h4), die eine Gruppe der Formeln (IIIa) oder (IIIb) aufweisen, in einer Menge von 0,1 bis 20 Gew.-%, besonders bevorzugt 0,5 bis 10 Gew.-%, bezogen auf das Gesamtgewicht der zur Polymerisation eingesetzten Monomere, eingesetzt.

<u>Monomer h5)</u>

Geeignete Urethan(meth)acrylate mit Alkylenoxidgruppen h5) sind in der DE 19838851 (Komponente e2)) beschrieben, worauf hier in vollem Umfang Bezug genommen wird. Sofern vorhanden, wird die Komponente h5) in einer Menge von 0,1 bis 25 Gew.-%, besonders bevorzugt 0,5 bis 20 Gew.-%, bezogen auf das Gesamtgewicht der zur Polymerisation eingesetzten Monomere, eingesetzt (d. h. Monomere a) und, soweit vorhanden, Monomere b), c), d), e), f), g) und h) addieren sich zu 100 Gew.-%).

<u>Monomer h6)</u>

Geeignete Monomere h6) sind z. B. n-Octylvinylether, 1,1,3,3-Tetramethylbutylvinylether, Ethylhexylvinylether, n-Nonylvinylether, n-Decylvinylether, n-Undecylvinylether, Tridecylvinylether, Myristylvinylether, Pentadecylvinylether, Palmitylvinylether, Heptadecylvinylether, Octadecylvinylether, Nonadecylvinylether, Arrachinylvinylether, Behenylvinylether, Lignocerenylvinylether, Cerotinylvinylether, Melissinylvinylether, Palmitoleinylvinylether, Oleylvinylether, Linolylvinylether, Linolenylvinylether, Stearylvinylether, Laurylvinylether und Mischungen davon.

Sofern vorhanden, wird die Komponente h6) in einer Menge von 0,1 bis 10 Gew.-%, bezogen auf das Gesamtgewicht der zur Polymerisation eingesetzten Monomere, eingesetzt.

Monomer h7)

Bevorzugte Vinylaromaten h7) sind Styrol, 2-Methylstyrol, 4-Methylstyrol, 2-(n-Butyl)styrol, 4-(n-Butyl)styrol, 4-(n-Decyl)styrol und Mischungen davon. Besonders bevorzugt sind Styrol und 2-Methylstyrol, insbesondere Styrol.
Sofern vorhanden, wird die Komponente h7) in einer Menge von 0,1 bis 10 Gew.-%, bezogen auf das Gesamtgewicht der zur Polymerisation eingesetzten Monomere, eingesetzt.

Monomer h8)

Bevorzugte Monomere h8) sind Vinylchlorid, Vinylidenchlorid, Vinylfluorid, Vinylidenfluorid und Mischungen davon.
Sofern vorhanden, wird die Komponente h8) in einer Menge von 0,1 bis 10 Gew.-%, bezogen auf das Gesamtgewicht der zur Polymerisation eingesetzten Monomere, eingesetzt.
Die zuvor genannten Monomere h) können jeweils einzeln oder in Form von beliebigen Mischungen eingesetzt werden.
Sofern vorhanden, wird die Komponente h) (d. h., die Summe aus Monomer h1), h2), h3), h4), h5), h6), h7) und h8) in einer Menge von 0,1 bis 40 Gew.-%, bezogen auf das Gesamtgewicht der zur Polymerisation eingesetzten Monomere eingesetzt (d. h. Monomere a) und, soweit vorhanden, Monomere b), c), d), e), f), g) und h) addieren sich zu 100 Gew.-%). Eine geeignete Einsatzmenge für zusätzliche Monomere h) liegt in einem Bereich von 0,1 bis 35 Gew.-%, insbesondere 0,2 bis 30 Gew.-%, bezogen auf das Gesamtgewicht der der zur Polymerisation eingesetzten Monomere.

Herstellung der Copolymerzusammensetzung A)

Die Herstellung der Copolymerzusammensetzung A) kann nach üblichen, dem Fachmann bekannten Verfahren erfolgen. Bevorzugt erfolgt die Herstellung der Copolymerzusammensetzung A) nach der Methode der Lösungspolymerisation.
Bevorzugt wird zur Herstellung der Copolymerzusammensetzung A) ein im Wesentlichen wasserfreies Lösungsmittel eingesetzt. Als Lösungsmittel können dabei selbstverständlich auch Lösungsmittelgemische eingesetzt werden. Im Wesentlichen wasserfrei bedeutet im Sinne der Erfindung, dass die Lösungsmittel in einer technisch zur Verfügung stehenden Qualität eingesetzt werden können und in der Regel nicht einer Entfernung von Restwasser unterzogen werden müssen. Unter "im Wesentlichen wasserfrei" wird daher ein Wassergehalt von bis zu 3 Gew.-%, bevorzugt bis zu 2 Gew.-%, bezogen auf das Gesamtgewicht des Lösungsmittels verstanden. Bevorzugt liegt der Wassergehalt des zur Herstellung der Copolymerzusammensetzung A) eingesetzten Lösungsmittels in einem Bereich von 0,05 bis 2 Gew.-%.
Die Polymerisation zur Herstellung der Copolymerzusammensetzung A) erfolgt erfindungsgemäß in einem Lösungsmittel, das wenigstens einen Alkohol umfasst.
Geeignete Alkohole sind unter den Polymerisationsbedingungen flüssige ein- oder mehrwertige Alkohole mit vorzugsweise 1 bis 8 Kohlenstoffatomen. Bevorzugt ist der Alkohol ausgewählt unter Methanol, Ethanol, n-Propanol, Isopropanol, n-Butanol, Isobutanol, sec.-Butanol, tert.-Butanol, Ethylenglycol, Diethylenglycol, Propylenglycol, Dipropylenglycol, Glycerin und Mischungen davon.
Besonders bevorzugt ist der Alkohol ausgewählt unter Ethanol, n-Propanol, Isopropanol, n-Butanol, Ethylenglycol, Propylenglycol und Mischungen. Insbesondere ist der Alkohol ausgewählt unter Ethanol, Isopropanol und Mischungen davon. Ein besonders bevorzugter Alkohol ist Isopropanol.
In einer speziellen Ausführungsform erfolgt die Herstellung der Copolymerzusammensetzung A) als Lösungspolymerisation in einem Lösungsmittel erfolgt, das

- Ethanol und/oder Isopropanol, oder
- Isopropanol und Propylenglycol

enthält.
In einer ersten Ausführungsform wird für die Herstellung der Copolymerzusammensetzung A) durch Lösungspolymerisation ein Lösungsmittel eingesetzt, das ausschließlich aus einem oder aus mehreren Alkoholen besteht.
In einer zweiten Ausführungsform wird für die Herstellung der Copolymerzusammensetzung A) durch Lösungspolymerisation ein Lösungsmittel eingesetzt, das wenigstens einen Alkohol und wenigstens ein von Alkoholen verschiedenes organisches Lösungsmittel enthält. Bevorzugt beträgt der Alkoholgehalt dann wenigstens 25 Gew.-%, bevorzugt wenigstens 50 Gew.-%, insbesondere wenigstens 75 Gew.-%, bezogen auf das Gesamtgewicht des Lösungsmittels. Bevorzugt beträgt der Gehalt an dem von Alkoholen verschiedenen organischen Lösungsmittel wenigstens 1 Gew.-%,

bevorzugt wenigstens 5 Gew.-%, insbesondere wenigstens 10 Gew.-%, bezogen auf das Gesamtgewicht des Lösungsmittels.

In einer geeigneten Ausführungsform wird zur Herstellung der Copolymerzusammensetzung A) die Polymerisation in einem ersten Lösungsmittel gestartet und im Verlauf der Polymerisation ein zweites Lösungsmittel zugegeben oder das erste Lösungsmittel im Verlauf der Polymerisation zumindest teilweise durch ein zweites Lösungsmittel ersetzt. Dazu kann man beispielsweise so vorgehen, dass man als zweites Lösungsmittel ein höher als das erste Lösungsmittel siedendes Mittel einsetzt und das erste Lösungsmittel ersetzt, indem man es aus der Lösung des Polymers abdestilliert und das zweite Lösungsmittel zumindest teilweise vor und/oder während der Destillation zugibt. So kann beispielsweise die Polymerisation in Isopropanol gestartet werden und das Isopropanol im Verlauf der Polymerisation teilweise oder vollständig durch Propylenglycol ersetzt werden.

Die Polymerisation wird üblicherweise bei Temperaturen von 40 bis 100 °C, bevorzugt 50 bis 95 °C, insbesondere 60 bis 90 °C durchgeführt.

Die Polymerisation wird üblicherweise bei Drücken von 1 bis 15 bar, insbesondere 1 bis 6 bar durchgeführt. Sofern die Polymerisation nicht unter erhöhtem Druck durchgeführt wird, bestimmt das Lösungsmittel bzw. Lösungsmittelgemisch durch die entsprechenden Siedetemperaturen die maximale Reaktionstemperatur.

Zur Herstellung der Polymerisate können die Monomeren mit Hilfe von Radikale bildenden Initiatoren polymerisiert werden.

Als Initiatoren für die radikalische Polymerisation können die hierfür üblichen Peroxound/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Düsopropylperoxidicarbamat, Bis-(o-toluoyl)-peroxid, Didecanoylperoxid, Dioctanoylperoxid, tert.-Butylperoctoat, Dilauroylperoxid, tert.-Butylperisobutyrat, tert.-Butylperacetat, Di-tert.-amylperoxid, tert.-Butylhydroperoxid, 2,2'-Azobisisobutyronitril, Azobis(2-amidinopropan)dihydrochlorid, 2,2'-Azobis(2,4-dimethylvaleronitril) oder 2,2'-Azobis(2-methyl-butyronitril).

<u>Hilfsstoffe</u>

In einer speziellen Ausführungsform wird zur Herstellung der Copolymerzusammensetzung A) zusätzlich wenigstens ein weiterer Hilfsstoff H1) eingesetzt, der ausgewählt ist unter silicongruppenhaltigen Tensiden, bevorzugt Polydialkylsiloxan-Polyether-Copolymere, die zusätzlich wenigstens eine Aminogruppe aufweisen können.

Sofern vorhanden, wird die Komponente H1) in einer Menge von 0,1 bis 15 Gew.-Teilen, vorzugsweise 0,5 bis 10 Gew.-Teilen, bezogen auf das Gesamtgewicht der zur Polymerisation eingesetzten Monomere a) und soweit vorhanden, Monomere b), c), d), e), f), g) und h) eingesetzt.

Geeignete silicongruppenhaltige Tenside (Silicontenside) sind im Allgenmeinen Polydialkylsiloxane, speziell Polydimethylsiloxane, in denen eine oder mehrere Alkylgruppen durch lipophile, hydrophile ionische oder nichtionische Reste ersetzt worden sind.

Geeignete silicongruppenhaltige Tenside H1) sind die unter den INCI-Namen Dimethicone-Copolyole oder Silicontenside bekannten Verbindungen, wie z. B. die unter den Markennamen Abil® (der Fa. Th. Goldschmidt), Alkasil® (der Fa. Rhône-Poulenc), Silicone Polyol Copolymer® (der Fa Genesee), Belsil® (der Fa. Wacker), Silwet® (der Fa. OSI) oder Dow Corning (der Fa. Dow Corning) erhältlich Verbindungen. Diese beinhalten Verbindungen mit den CAS-Nummern 64365-23-7; 68937-54-2; 68938-54-5; 68937-55-3. Eine geeignete, kommerziell erhältliche Verbindung ist Belsil ® DMC 6031.

Bevorzugte silicongruppenhaltige Tenside H1) sind Polydialkylsiloxan-Polyether-Copolymere, die zusätzlich wenigstens eine Aminogruppe aufweisen können.

Bevorzugte Siliconverbindungen H1) sind Polysiloxane der allgemeinen Formel (E)

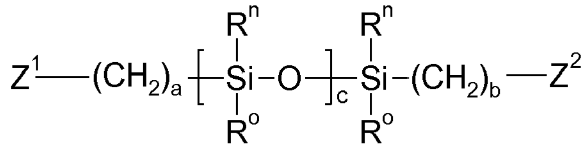

(E)

worin

| | |
|---|---|
| a und b | unabhängig voneinander für 1 bis 8 stehen, |
| c | für 2 bis 1000 steht, |

$R^n$ und $R^o$ unabhängig voneinander für Alkyl, Cycloalkyl, Aryl oder Arylalkyl stehen,

$Z^1$ und $Z^2$ unabhängig voneinander für Reste der Formel (F) $-(OCH_2CH_2)_u(OCH(CH_3)CH_2)_v-X^1-H$ (F)

stehen, wobei in der Formel (F) die Reihenfolge der Alkylenoxideinheiten beliebig ist,

u und v unabhängig voneinander für eine ganze Zahl von 0 bis 500 stehen, wobei die Summe aus u und v > 0 ist,

$X^1$ für O oder $NR^p$ steht, worin $R^p$ für Wasserstoff, Alkyl, Cycloalkyl oder Aryl steht.

Vorzugsweise ist in der Formel (E) die Summe aus u und v so gewählt, dass das Molekulargewicht der Polysiloxane H1) in einem Bereich von etwa 300 bis 30 000 liegt.

Bevorzugt liegt die Gesamtzahl der Alkylenoxideinheiten der Polysiloxane H1), d. h. die Summe aus u und v, in einem Bereich von etwa 3 bis 200, bevorzugt 5 bis 180.

Bevorzugt sind in den Verbindungen der Formel (E) die Reste $R^n$ und $R^o$ unabhängig voneinander ausgewählt unter Methyl, Ethyl, Cyclohexyl, Phenyl und Benzyl. Besonders bevorzugt stehen $R^n$ und $R^o$ beide für Methyl.

Bevorzugte Siliconverbindungen H1) sind weiterhin ausgewählt unter ethoxilierten und/oder propoxylierten Polydimethylsiloxane der allgemeinen Formel (G1)

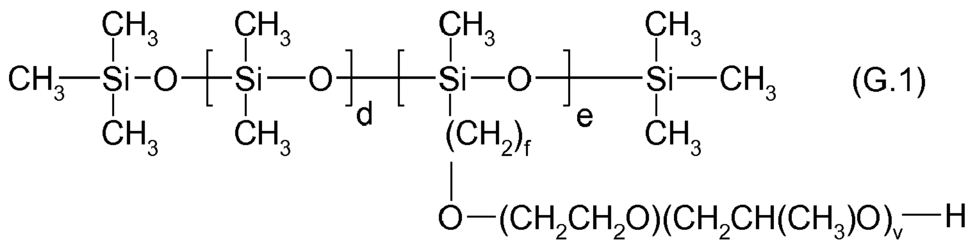

(G.1)

worin die Reihenfolge der Siloxaneinheiten beliebig ist,

d für eine ganze Zahl von 2 bis 1000, bevorzugt 3 bis 500, insbesondere 5 bis 100, steht,

e für eine ganze Zahl von 2 bis 100, bevorzugt 3 bis 50, insbesondere 4 bis 20, steht,

f für eine ganze Zahl von 2 bis 8 steht,

und

u und v unabhängig voneinander für eine ganze Zahl von 0 bis 500, bevorzugt 0 bis 250, steht, wobei die Summe aus u und v $\geq 1$, bevorzugt $\geq 5$, insbesondere $\geq 10$, ist.

Geeignete Verbindungen der Formel G.1 sind unter der Bezeichnung Wacker-Belsil® DMC 6031 und Pluriol® ST 4005 (Fa. BASF SE) erhältlich.

Bevorzugte Siliconverbindungen H1) sind weiterhin Polysiloxane mit sich wiederholenden Einheiten der allgemeinen Formel (J)

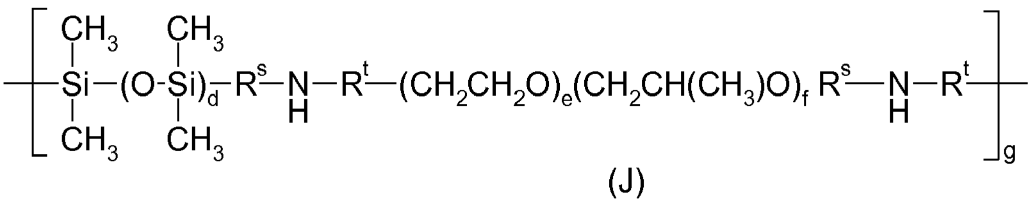

(J)

worin

d für eine ganze Zahl von 0 bis 100 steht,

g für eine ganze Zahl von 1 bis 8 steht,

$R^s$ und $R^t$ unabhängig voneinander für $C_1$-bis $C_8$-Alkylen stehen,

die Reihenfolge der Alkylenoxideinheiten beliebig ist und e und f unabhängig voneinander für eine ganze Zahl von 0 bis 200 stehen, wobei die Summe aus r und s > 0 ist.

Bevorzugt stehen in der Formel (J) $R^s$ und $R^t$ unabhängig voneinander für einen $C_2$-$C_4$-Alkylenrest.

Insbesondere stehen $R^s$ und $R^t$ unabhängig voneinander für einen $C_2$-$C_3$-Alkylenrest.

Vorzugsweise liegt das Molekulargewicht der Verbindung der Formel (J) in einem Bereich von etwa 300 bis 100 000.

Vorzugsweise steht in der Formel 1.3 d für eine ganze Zahl von 1 bis 20, wie z. B. 2 bis 10.

Bevorzugt liegt die Gesamtzahl der Alkylenoxideinheiten der Verbindung der Formel (J), d. h. die Summe aus e und f, in einem Bereich von etwa 3 bis 200, bevorzugt 5 bis 180.

Bevorzugt sind die Endgruppen der Polysiloxane mit Wiederholungseinheiten der allgemeinen Formel (J) ausgewählt unter $(CH_3)_3SiO$, H, $C_1$-$C_8$-Alkyl und Mischungen davon. Eine bevorzugte Alkylendgruppe ist Methyl.

Aminogruppenhaltige Verbindungen mit Wiederholungseinheiten der allgemeinen Formel (J) weisen bevorzugt eine Aminzahl in einem Bereich von etwa 2 bis 50, insbesondere 3 bis 20 auf.

Geeignete alkoxylierte Siloxanamine der Formel J sind z. B. in der WO-A-97/32917 beschrieben, auf die hier in vollem Umfang Bezug genommen wird. Kommerziell erhältliche Verbindungen sind z. B. die Silsoft-Marken der Momentive Performance Materials (vormals Fa. Witco), z. B. Silsoft A-843.

Bevorzugte Siliconverbindungen H1) sind ferner unter dem Markennamen Wetsoft® NE 810 und Wetsoft® NE 820 (Fa. Wacker) erhältlich.

Bevorzugte Siliconverbindungen H1) sind weiterhin Polysiloxane mit sich wiederholenden Einheiten der allgemeinen Formel (K)

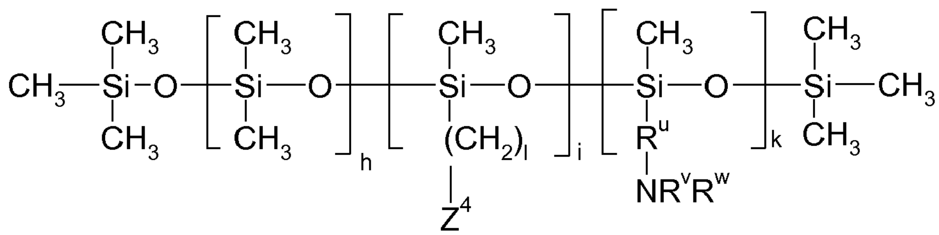

(K)

worin

| | |
|---|---|
| $R^u$ | für einen $C_1$-$C_8$-Alkylenrest steht, |
| $R^v$ und $R^w$ | unabhängig voneinander für Wasserstoff, $C_1$-$C_8$-Alkyl oder $C_5$-$C_8$-Cycloalkyl stehen, |

die Reihenfolge der Siloxaneinheiten beliebig ist,

| | |
|---|---|
| h, i und k | unabhängig voneinander für 0 bis 100 stehen, wobei die Summe aus h, i und k mindestens 3 ist, |
| l | für eine ganze Zahl von 2 bis 8 steht, |
| $Z^4$ | für einen Rest der Formel (F.1) |

$$-(OCH_2CH_2)_u(OCH(CH_3)CH_2)_v-OR^x \qquad (F.1)$$

steht, wobei
in der Formel (F.1) die Reihenfolge der Alkylenoxideinheiten beliebig ist,
u und v unabhängig voneinander für eine ganze Zahl von 0 bis 500 stehen, wobei die Summe aus u und v > 0 ist,
$R^x$ für Wasserstoff oder einen $C_1$-$C_8$-Alkylrest steht,

und Mischungen davon.

Bevorzugt steht in der Formel (K) der Rest $R^u$ für einen $C_2$-$C_4$-Alkylenrest.

Bevorzugt stehen in der Formel (K) $R^v$ und $R^w$ unabhängig voneinander für Wasserstoff oder $C_1$-$C_4$-Alkyl.

Vorzugsweise wird die Summe aus h, I und k so gewählt, dass das Molekulargewicht der Verbindung der Formel (K) in einem Bereich von etwa 300 bis 100 000, bevorzugt 500 bis 50 000, liegt.

Bevorzugt liegt die Gesamtzahl der Alkylenoxideinheiten des Restes der Formel (F.1), d. h. die Summe aus u

und v, in einem Bereich von etwa 3 bis 200, bevorzugt 5 bis 80.

Bevorzugt steht in der Formel (F.1) der Rest $R^x$ für Wasserstoff oder $C_1$-$C_4$-Alkyl.

Eine geeignete Verbindung der Formel (K) ist z. B. Silsoft A-858 der Fa. der Momentive Performance Materials (vormals Fa. Witco).

Eine weitere bevorzugte Siliconverbindungen H1) ist die Verbindung mit der INCI-Bezeichnung Methoxy PEG/PPG-7/3 Aminopropyl Dimethicone. Diese ist kommerziell unter der Bezeichnung ABIL® Soft AF 100 von Evonik Industries erhältlich.

Geeignete Polysiloxandiamine H1) sind Tegomer® A-Si 2122 (Mn = 900 g/mol) und Tegomer® A-Si 2322 (Mn = 2800 g/mol) der Evonik Industries (vormals Th. Gold-schmidt).

Hilfsstoff H2)

In einer speziellen Ausführungsform wird zur Herstellung der Copolymerzusammensetzung A) zusätzlich wenigstens ein weiterer Hilfsstoff H2) eingesetzt, der ausgewählt ist unter nichtionischen Tensiden. Bevorzugt ist der Hilfsstoff H2) ausgewählt unter nichtionischen Tensiden mit einem HLB-Wert von höchstens 7, insbesondere nichtionischen Tensiden mit einem HLB-Wert von höchstens 5.

Sofern vorhanden, wird die Komponente H2) in einer Menge von 0,1 bis 30 Gew.-Teilen, vorzugsweise 0,5 bis 15 Gew.-Teilen, bezogen auf das Gesamtgewicht der zur Polymerisation eingesetzten Monomere a) und soweit vorhanden, Monomere b), c), d), e), f), g) und h) eingesetzt.

Als Hilfsstoff H2) geeignete nichtionische Tenside sind vorzugsweise ausgewählt unter

- alkoxilierten primären $C_8$-$C_{30}$-Alkoholen,
- Estern von $C_8$-$C_{30}$-Carbonsäuren mit mehrwertigen Alkoholen und Alkoxilate davon,
- alkoxilierten Fettsäurealkylestern,
- Aminoxiden,
- Fettsäurealkanolamiden,
- Polyhydroxyfettsäureamiden,
- Mischungen der zuvor genannten Tenside.

Als H2) geeignete alkoxilierte primäre Alkohole weisen vorzugsweise 10 bis 22 C-Atome, besonders bevorzugt 12 bis 20 C-Atome auf. Sie sind vorzugsweise mit 1 bis 50, besonders bevorzugt 1 bis 30, wie z. B. 2 bis 20 Mol, Alkylenoxid pro Mol Alkohol alkoxiliert. Zur Alkoxilierung können z. B. Ethylenoxid, Propylenoxid, 1,2-Butylenoxid und Mischungen davon eingesetzt werden. Bevorzugt ist Ethylenoxid (EO). Bevorzugt handelt es sich um Alkohole mit linearen oder verzweigten Alkyl- oder Alkenylresten, wobei letztere auch mehrere nicht benachbarte Doppelbindungen aufweisen können. Bevorzugt sind Alkohole, in denen der Alkoholrest linear oder in 2-Stellung methylverzweigt ist bzw. sind Alkoholgemische mit linearen und methylverzweigten Resten, so wie sie üblicherweise in Oxoalkoholresten vorliegen. Besonders bevorzugt sind Alkoxilate, speziell Ethoxilate mit Alkoholen nativen Ursprungs, sowie Oxoalkoholalkoxilate und Guerbetalkoholalkoxilate. Dazu zählen insbesondere Alkoxilate von durch Reduktion natürlicher Fettsäuren erhältlichen Alkoholen und Alkoholgemischen mit 8 bis 30, vorzugsweise 10 bis 22 C-Atomen, z. B. von n-Decanol, Laurinalkohol, Myristinalkohol, Cetylalkohol, Stearinalkohol, Oleinalkohol, Lignocerylalkohol, Cerylalkohol, etc. Die angegebenen Alkoxylierungsgrade stellen jeweils statistische Mittelwerte dar, die für ein spezielles Produkt eine ganze oder eine gebrochene Zahl sein können. Geeignet sind auch alkoxilierte Alkohole, die eine eingeengte Homologenverteilung aufweisen (narrow range ethoxylates, NRE). Auch alkoxilierte Alkohole, die EO- und PO-Gruppen zusammen im Molekül enthalten, sind einsetzbar. Hierbei können Blockcopolymere mit EO-PO-Blockeinheiten bzw. PO-EO-Blockeinheiten eingesetzt werden, aber auch EO-PO-EO-Copolymere bzw. PO-EO-PO-Copolymere. Selbstverständlich sind als H2) auch gemischt alkoxilierte primäre Alkohole einsetzbar, in denen EO- und PO-Einheiten nicht blockweise, sondern statistisch verteilt sind. Solche Produkte sind z. B. durch gleichzeitige Einwirkung von Ethylen- und Propylenoxid auf primäre Alkohole erhältlich.

Als Hilfsstoff H2) geeignete Ester von $C_8$-$C_{30}$-Carbonsäuren mit mehrwertigen Alkoholen leiten sich vorzugsweise von linearen oder verzweigten, gesättigten oder einfach oder mehrfach ungesättigten Carbonsäuren mit 8 bis 30 C-Atomen, vorzugsweise 10 bis 22 C-Atomen, insbesondere 10 bis 18 C-Atomen im Alkyl- oder Alkenylrest, wie Caprylsäure, Pelargonsäure, Undecansäure, Laurinsäure, Tridecansäure, Myristinsäure, Pentadecansäure, Palmitinsäure, Margarinsäure, Stearinsäure, Isostearinsäure, Nonadecansäure, Arachinsäure, Behensäure, Palmitoleinsäure, Ölsäure, Linolsäure, Linolensäure, Arachidonsäure und deren Gemischen ab. Bevorzugt sind Laurinsäure, Stearinsäure, Isostearinsäure, Palmitinsäure und Ölsäure.

Geeignete mehrwertige Alkohole sind vorzugsweise ausgewählt unter geradkettigen, verzweigten oder carbocyclischen, gesättigten oder ungesättigten Kohlenwasserstoffverbindungen mit wenigstens drei Kohlenstoffatomen und wenigstens drei (veresterbaren) Hydroxylgruppen. Ungesättigte Kohlenwasserstoffverbindungen können dabei eine

oder mehrere, vorzugsweise 1, 2 oder 3 C-C-Doppelbindungen aufweisen. Gemische solcher Polyole sind ebenfalls einsetzbar. Das Polyol ist insbesondere ein geradkettiger oder verzweigter gesättigter Kohlenwasserstoff mit 3 bis 30 Kohlenstoffatomen und 3 bis 10 Hydroxylgruppen. Als bevorzugte Beispiele für brauchbare Polyole sind zu nennen: Glycerin, Di-, Tri- und Polyglycerine, niedermolekulares teil- oder vollständig hydrolisiertes Polyvinylacetat, 1,2,4-Butantriol, Trimethylolmethan, Trimethylolethan, Trimethylolpropan, Trimethylolbutan, 2,2,4-Trimethyl-1,3-pentandiol, Pentaerythrit, Ditrimethylolpropan, Dipentaerythrit, Tripentaerythrit, D-, L- und Meso-Erythrit, D- und L-Arabit, Adonit, Xylit, Sorbit, Mannit, Dulcit, Inositole sowie deren Mischungen.

Als Hilfsstoff H2) eignen sich auch Alkoxilate von Estern von $C_8$-$C_{30}$-Carbonsäuren mit mehrwertigen Alkoholen. Die Alkoxylierung kann mit Ethylenoxid, Propylenoxid und/oder Butylenoxid erfolgen. Es können Blockcopolymerisate oder statistische Copolymere vorliegen. Pro mol Alkohol enthalten sie üblicherweise 1 bis 50 mol, vorzugsweise 2 bis 30 mol, mindestens eines Alkylenoxids. Bevorzugtes Alkylenoxid ist Ethylenoxid.

Eine weitere Klasse bevorzugt eingesetzter nichtionischer Tenside, die als Hilfsstoff H2) eingesetzt werden können, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester, wie sie beispielsweise in der japanischen Patentanmeldung JP 58/217598 beschrieben sind oder die vorzugsweise nach dem in der internationalen Patentanmeldung WO-A-90/13533 beschriebenen Verfahren hergestellt werden.

Als Hilfsstoffe H2) eignen sich auch nichtionische Tenside vom Typ der Aminoxide, beispielsweise N-Kokosalkyl-N,N-dimethylaminoxid und N-Talgalkyl-N,N-dihydroxyethylaminoxid sowie Fettsäurealkanolamide. Die Menge dieser nichtionischen Tenside beträgt vorzugsweise nicht mehr als die der ethoxylierten Fettalkohole, insbesondere nicht mehr als die Hälfte davon.

Weitere geeignete Hilfsstoffe H2) sind Polyhydroxyfettsäureamide der Formel (2),

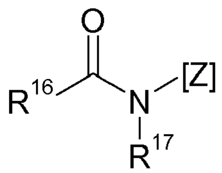

(2)

worin

- $R^{16}$ C(=O) für einen aliphatischen Acylrest mit 6 bis 22 Kohlenstoffatomen,
- $R^{17}$ für Wasserstoff, einen Alkyl- oder Hydroxyalkylrest mit 1 bis 4 Kohlenstoffatomen und
- [Z] für einen linearen oder verzweigten Polyhydroxyalkylrest mit 3 bis 10 Kohlenstoffatomen und 3 bis 10 Hydroxylgruppen steht.

Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel (3)

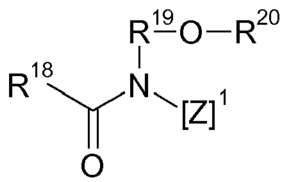

(3)

worin

- $R^{18}$ für einen linearen oder verzweigten Alkyl- oder Alkenylrest mit 7 bis 12 Kohlenstoffatomen,
- $R^{19}$ für einen linearen, verzweigten oder cyclischen Alkylenrest mit 2 bis 8 Kohlenstoffatomen oder einen Arylenrest mit 6 bis 8 Kohlenstoffatomen und
- $R^{20}$ für einen linearen, verzweigten oder cyclischen Alkylrest oder einen Arylrest oder einen Oxy-Alkylrest mit 1 bis 8 Kohlenstoffatomen steht, wobei $C_1$-$C_4$-Alkyl- oder Phenylreste bevorzugt sind, und
- $[Z]^1$ für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes.
- $[Z]^1$ wird vorzugsweise durch reduktive Aminierung eines Zuckers erhalten, beispielsweise Glucose, Fructose, Maltose, Lactose, Galactose, Mannose oder Xylose. Die N-Alkoxy- oder N-Aryloxy-substituierten Verbindungen können dann beispielweise gemäß WO-A-95/07331 durch Umsetzung mit Fettsäuremethylestern in Gegenwart eines Alkoxids als Katalysator in die gewünschten Polyhydroxyfettsäureamide überführt werden.

Als Hilfsstoff H2) geeignete nichtionische Tenside sind speziell (HLB-Werte und gegebenenfalls Markenbezeichnung in Klammern):

Sorbitantrioleat (1,8; Span 85)
Polyoxyethylensorbit-Bienenwachsderivat (4,0; Atlas G-1727),
Propylenglykol-Fettsäureester (4,1; Emcol PM-50),
Sorbitanmonooleat (4,3; Span 80),
Sorbitanmonooleat (4,3; Arlacel 80),
Propylenglykolmonolaurat (4,5; Atlas G-917),
Propylenglykolmonolaurat (4,5; Atlas G-3851),
Propylenglykol-Fettsäureester (4,5; Emcol PL-50),
Sorbitanmonostearat (4,7; Span 60 oder Arlacel 60),
Diethylenglykolmonooleat (4,7; Atlas G-2139),
Diethylenglykol-Fettsäureester (4,7; Emcol DO-50),
Diethylenglykolmonostearat (4,7; Atlas G-2146),
Diethylenglykol-Fettsäureester (4,7; Emcol DS-50),
Polyoxyethylen-sorbitol-Bienenwachs-Derivat (5,0; Atlas G-1702),
Diethylenglykol-Fettsäureester (5,1; Emcol DP-50),
Diethylenglykol-Fettsäureester (5,6; Emcol DM-50),
Polyoxyethylen-sorbitol-Bienenwachs-Derivat (6,0; Atlas G-1725),
Diethylenglykolmonolaurat (6,1; Atlas G-2124),
Diethylenglykol-Fettsäureester (6,1; Emcol DL-50),
Diethylenglykolmonolaurat (6,5; Glaurin),
Sorbitanmonopalmitat (6,7; Span 40 oder Arlacel 40),

und Mischungen davon.

Als Hilfsstoff H2) besonders geeignete nichtionische Tensiden sind die Dehymuls®-Marken der Fa. COGNIS Care Chemicals. Dazu zählen:

Dehymuls HRE 7 (mit 7 Mol Ethylenoxid alkoxiliertes hydriertes Castoröl),
Dehymuls LE (PEG-30 Dipolyhydroxystearat),
Dehymuls E (Gemisch aus höher molekularen Fettsäureestern, Fettsäuresalzen und ölbindenden Additiven; Dicocoyl-Pentaerythrityl-Distearyl-citrat und Sorbitansesquioleat und Bienenwachs (cera alba) und Aluminiumstearat),
Dehymuls K (Petrolatum und Decyloleat und Dicocoyl-Pentaerythrityl-Distearyl-citrat und Sorbitansesquioleat und mikrokristallines Wachs (cera microcristallina) und Mineralöl und Bienenwachs (cera alba) und Aluminiumstearat),
Dehymuls PGPH (Polyglycerin-poly-12-hydroxystearinsäureester),
Dehymuls SML (Sorbitanmonolaurat),
Dehymuls SMO (Sorbitanmonooleat),
Dehymuls SMS (sorbitanmonostearat),
Dehymuls SSO (Sorbitansesquioleat),

und Mischungen davon.

Als Hilfsstoff H2) besonders geeignete nichtionische Tensiden sind weiterhin die Hypermer®-Marken der Fa. ICI. Dazu zählen:

Hypermer LP6 (polymerer Fettsäureester, Molekulargewicht (MW) etwa 4300),
Hypermer LP7,
Hypermer B239 (Blockcopolymer einer Polyhydroxyfettsäure und Polyethylenoxid, MW ca. 3500),
Hypermer B246 (Blockcopolymer einer Polyhydroxyfettsäure und Polyethylenoxid, MW ca. 7500),
Hypermer B261 (Blockcopolymer einer Polyhydroxyfettsäure und Polyethylenoxid, MW ca. 9600),
Hypermer 2234,
Hypermer E-464 (Copolymer mit einer langen hydrophoben Alkylenkette und verschiedenen anionischen/nichtionischen Hydrophilen, MW ca. 2300),
Hypermer IL2296,

Hypermer A-109 (Blockcopolymer einer Fettsäure oder langkettigen Alkylen und EO),
Hypermer A-409 (Blockcopolymer einer Fettsäure oder langkettigen Alkylen und EO).
In einer speziellen Ausführungsform wird als Hilfsstoff H2) Sorbitantrioleat eingesetzt. Sorbitantrioleat ist unter dem Markennamen Span® 85 kommerziell erhältlich.

In einer weiteren speziellen Ausführungsform wird als Hilfsstoff H2) ein Gemisch aus Cetearylglucosid und Cetearylalkohol eingesetzt. Eine solche Mischung ist unter dem Markennamen Emulgade® PL 68/50 kommerziell erhältlich.

Soweit zur Herstellung der der Copolymerzusammensetzung A) wenigstens ein silcon-haltiges Tensid als Hilfsstoff H1) eingesetzt wird, wird die Komponente H1) vorzugsweise zumindest teilweise vor Beginn der Polymerisation vorgelegt. Besonders bevorzugt werden H1) vollständig vor Beginn der Polymerisation vorgelegt.

Soweit zur Herstellung der Copolymerzusammensetzung A) wenigstens ein nichtionisches Tensid als Hilfsstoff H2) eingesetzt wird, wird die Komponente H2) vorzugsweise zumindest teilweise vor Beginn der Polymerisation vorgelegt. Besonders bevorzugt werden H2) vollständig vor Beginn der Polymerisation vorgelegt.

Die durch Lösungspolymerisation erhaltene Copolymerzusammensetzung A) kann nach üblichen, dem Fachmann bekannten Verfahren isoliert werden. Dazu kann das Lösungsmittel durch übliche Verfahren, vorzugsweise destillativ, entfernt werden.

<u>Variante A)</u>

In einer bevorzugten Ausführungsform enthält die zur Copolymerisation eingesetzte Monomerzusammensetzung

- 3 bis 25 Gew.-%, bezogen auf das Gesamtgewicht der zur Polymerisation eingesetzten Monomere, 2-Acrylamido-2-methylpropansulfonsäure a),
- 0 bis 90 Gew.-%, bezogen auf das Gesamtgewicht der zur Polymerisation eingesetzten Monomere, wenigstens einer Verbindung c), ausgewählt unter $C_1$-$C_6$-Alkyl(meth)acrylamiden,
- 0 bis 90 Gew.-%, bezogen auf das Gesamtgewicht der zur Polymerisation eingesetzten Monomere, wenigstens einer Verbindung h1), ausgewählt unter $C_1$-$C_6$-Alkyl(meth)acrylaten,
- 0 bis 20 Gew.-%, bezogen auf das Gesamtgewicht der zur Polymerisation eingesetzten Monomere, wenigstens einer N-Vinylimidazol-Verbindung b),
- 0 bis 50 Gew.-%, bezogen auf das Gesamtgewicht der zur Polymerisation eingesetzten Monomere, wenigstens einer Verbindung d), ausgewählt unter Acrylsäure, Methacrylsäure, Salzen der Acrylsäure, Salzen der Methacrylsäure und Mischungen davon,

enthält, mit der Maßgabe, dass das Gesamtgewicht der Monomere c) und h1) 10 bis 90 Gew.-%, bezogen auf das Gesamtgewicht der zur Polymerisation eingesetzten Monomere, beträgt, und dass sich die zur Polymerisation eingesetzten Monomere auf 100 Gew.-% addieren.

Bevorzugt ist die Komponente c) ausgewählt unter N-Methyl(meth)acrylamid, N-Ethyl(meth)acrylamid, N-Propyl(meth)acrylamid, N-(n-Butyl)(meth)acrylamid, N-tert.-Butyl(meth)acrylamid und Mischungen davon.

Bevorzugt ist die Komponente h1) ausgewählt unter Methyl(meth)acrylat, Ethyl(meth)acrylat, n-Butyl(meth)acrylat, tert.-Butyl(meth)acrylat, und Mischungen davon.

Bevorzugt erfolgt die Polymerisation nach der Variante A) als Lösungspolymerisation in einem Lösungsmittel, das wenigstens einen bei Normalbedingungen flüssigen Alkohol enthält. Besonders bevorzugt enthält das Lösungsmittel Isopropanol und/oder Ethanol. In einer speziellen Ausführung besteht das Lösungsmittel aus Isopropanol oder aus Ethanol oder aus einem Gemisch von Isopropanol und Ethanol.

Die nach der Variante A) hergestellten Copolymerzusammensetzungen eignen sich insbesondere als Haarfestigerpolymere.

<u>Variante B)</u>

In einer bevorzugten Ausführungsform enthält die zur Copolymerisation eingesetzte Monomerzusammensetzung

- 0,5 bis 30 Gew.-%, bezogen auf das Gesamtgewicht der zur Polymerisation eingesetzten Monomere, 2-Acrylamido-2-methylpropansulfonsäure a),

- 5 bis 70 Gew.-%, bezogen auf das Gesamtgewicht der zur Polymerisation eingesetzten Monomere, wenigstens einer Verbindung d), die ausgewählt ist unter Acrylsäure, Methacrylsäure, Salzen der Acrylsäure, Salzen der Methacrylsäure und Mischungen davon,

- 5 bis 50 Gew.-%, bezogen auf das Gesamtgewicht der zur Polymerisation eingesetzten Monomere, wenigstens einer Verbindung e), die ausgewählt ist mit $C_8$-$C_{30}$-Alkylgruppen terminierten Polyether(meth)acrylaten und Mischungen davon,

- 0 bis 25 Gew.-%, bezogen auf das Gesamtgewicht der zur Polymerisation eingesetzten Monomere, wenigstens einer Verbindung c), ausgewählt unter $C_1$-$C_6$-Alkyl(meth)acrylamiden,

- 0 bis 25 Gew.-%, bezogen auf das Gesamtgewicht der zur Polymerisation eingesetzten Monomere, wenigstens einer Verbindung h1), ausgewählt unter $C_1$-$C_{30}$-Alkyl(meth)acrylaten,

mit der Maßgabe, dass sich die zur Polymerisation eingesetzten Monomere auf 100 Gew.-% addieren.

Bevorzugt ist die Komponente c) ausgewählt unter N-Methyl(meth)acrylamid, N-Ethyl(meth)acrylamid, N-Propyl(meth)acrylamid, N-(n-Butyl)(meth)acrylamid, N-tert.-Butyl(meth)acrylamid und Mischungen davon.

Bevorzugt ist die Komponente h1) ausgewählt unter Methyl(meth)acrylat, Ethyl(meth)acrylat, n-Butyl(meth)acrylat, tert.-Butyl(meth)acrylat, Lauryl(meth)acrylat, Stearyl(meth)acrylat und Mischungen davon.

Bevorzugt ist die Komponente e) ausgewählt ist unter insbesondere mit $C_{12}$-$C_{24}$-Alkylgruppen terminierten Polyethylenglycol(meth)acrylaten und Mischungen davon.

Bevorzugt erfolgt die Polymerisation nach der Variante B) als Lösungspolymerisation in einem Lösungsmittel, das wenigstens einen bei Normalbedingungen flüssigen Alkohol enthält. Besonders bevorzugt enthält das Lösungsmittel Isopropanol und/oder Propylenglycol. In einer speziellen Ausführung besteht das Lösungsmittel aus Isopropanol oder aus Propylenglycol oder aus einem Gemisch von Isopropanol und Propylenglycol.

Die nach der Variante B) hergestellten Copolymerzusammensetzungen eignen sich insbesondere als polymere oberflächenaktive Verbindung (Polymertensid).

Variante C)

In einer bevorzugten Ausführungsform enthält die zur Copolymerisation eingesetzte Monomerzusammensetzung

- 0,5 bis 50 Gew.-%, bezogen auf das Gesamtgewicht der zur Polymerisation eingesetzten Monomere, 2-Acrylamido-2-methylpropansulfonsäure a),

- 5 bis 90 Gew.-%, bezogen auf das Gesamtgewicht der zur Polymerisation eingesetzten Monomere, wenigstens einer Verbindung d), die ausgewählt ist unter Acrylsäure, Methacrylsäure, Salzen der Acrylsäure, Salzen der Methacrylsäure und Mischungen davon,

- 5 bis 50 Gew.-%, bezogen auf das Gesamtgewicht der zur Polymerisation eingesetzten Monomere, wenigstens einer Verbindung e), die ausgewählt ist unter mit $C_8$-$C_{30}$-Alkylgruppen terminierten Polyether(meth)acrylaten,

- 0,1 bis 3 Gew.-%, bezogen auf das Gesamtgewicht der zur Polymerisation eingesetzten Monomere, wenigstens eines Vernetzers g),

- 0 bis 40 Gew.-%, bezogen auf das Gesamtgewicht der zur Polymerisation eingesetzten Monomere, wenigstens einer Verbindung c), ausgewählt unter $C_1$-$C_6$-Alkyl(meth)acrylamiden,

- 0 bis 40 Gew.-%, bezogen auf das Gesamtgewicht der zur Polymerisation eingesetzten Monomere, wenigstens einer Verbindung h1), ausgewählt unter $C_1$-$C_{30}$-Alkyl(meth)acrylaten,

mit der Maßgabe, dass sich die zur Polymerisation eingesetzten Monomere auf 100 Gew.-% addieren.

Bevorzugt ist die Komponente c) ausgewählt ist unter N-Methyl(meth)acrylamid, N-Ethyl(meth)acrylamid, N-Propyl(meth)acrylamid, N-(n-Butyl)(meth)acrylamid, N-tert.-Butyl(meth)acrylamid und Mischungen davon.

Bevorzugt ist die Komponente h1) ausgewählt ist unter Methyl(meth)acrylat, Ethyl(meth)acrylat, n-Butyl(meth)acrylat, tert.-Butyl(meth)acrylat, Lauryl(meth)acrylat, Stearyl(meth)acrylat, und Mischungen davon.

Bevorzugt ist die Komponente e) ausgewählt ist unter insbesondere mit $C_{12}$-$C_{24}$-Alkylgruppen terminierten Polyethylenglycol(meth)acrylaten und Mischungen davon.

Bevorzugt ist die Komponente g) ausgewählt ist unter Ethylenglykoldi(meth)acrylat, Pentaerythrittriallylether und Mischungen davon.

Bevorzugt erfolgt die Polymerisation nach der Variante C) als Lösungspolymerisation in einem Lösungsmittel, das wenigstens einen bei Normalbedingungen flüssigen Alkohol enthält. Besonders bevorzugt enthält das Lösungsmittel Isopropanol und/oder Propylenglycol. In einer speziellen Ausführung besteht das Lösungsmittel aus Isopropanol oder aus Propylenglycol oder aus einem Gemisch von Isopropanol und Propylenglycol.

In einer speziellen Ausführungsform der Variante C) wird die Polymerisation in einem Lösungsmittel gestartet, das Isopropanol enthält, wobei im Verlauf der Polymerisation Propylenglycol zugegeben wird oder Isopropanol zumindest teilweise durch Propylenglycol ersetzt wird.

Die nach der Variante C) hergestellten Copolymerzusammensetzungen eignen sich insbesondere für einen Einsatz zur Einstellung der rheologischen Eigenschaften diverser Produkte, z. B. von Shampoos.

Ein weiterer Gegenstand der Erfindung ist die nach dem zuvor beschriebenen Verfahren erhältliche Copolymerzusammensetzung A).

Im Rahmen der vorliegenden Erfindung umfasst der Begriff "Copolymerzusammensetzung A)" auch die daraus durch wenigstens einen Aufarbeitungs- und/oder Reinigungsschritt erhaltenen Produkte. Geeignete Aufarbeitungs- und Reinigungsschritte sind beispielsweise Waschen, Trocknen und Formgebung.

Die Copolymerzusammensetzung A) liegt vorzugsweise in Form oder einer Lösung in einem wässrig/alkoholischen Medium vor.

Die erfindungsgemäße Copolymerzusammensetzung A) kann zusätzlich zu den bei der Fällungspolymerisation erhaltenen Polymerteilchen wenigstens eine weitere Komponente enthalten. Dazu zählt wenigstens einer der Hilfsstoffe H1) und/oder H2).

Die Hilfsstoffe H1) und/oder H2) können sich auch vorteilhaft auf eine oder mehrere andere anwendungstechnische Eigenschaften der Copolymerzusammensetzung A) auswirken, z. B. durch die Förderung rieselarmer Produkte oder eine Steuerung von Teilchengröße, Molekulargewicht, Morphologie, etc.

Die erfindungsgemäßen Copolymerzusammensetzungen A) und die darin enthaltenen Copolymere zeichnen sich durch ihre pH-abhängige Löslichkeit aus.

Die von AMPS abgeleiteten Säuregruppen der in den Copolymerzusammensetzungen A) enthaltenen Copolymere liegen erfindungsgemäß (teil)neutralisiert vor. Sofern die in den Copolymerzusammensetzungen A) enthaltenen Copolymere von anderen Säuren abgeleiteten Säuregruppen aufweisen (z. B. von (Meth)acrylsäure), können diese ebenfalls mit einer Base teilweise oder vollständig neutralisiert werden. Als Base für die Neutralisation der Polymere können Alkalimetallbasen wie Natronlauge, Kalilauge, Soda, Natriumhydrogencarbonat, Kaliumcarbonat oder Kaliumhydrogencarbonat und Erdalkalimetallbasen, wie Calciumhydroxid, Calciumoxid, Magnesiumhydroxid oder Magnesiumcarbonat sowie Amine verwendet werden. Geeignete Amine sind z. B. $C_1$-$C_6$-Alkylamine, bevorzugt n-Propylamin und n-Butylamin, Dialkylamine, bevorzugt Diethylpropylamin und Dipropylmethylamin, Trialkylamine, bevorzugt Triethylamin und Triisopropylamin. Bevorzugt sind Aminoalkohole, z. B. Trialkanolamine, wie Triethanolamin, Alkyldialkanolamine, wie Methyl- oder Ethyldiethanolamin und Dialkylalkanolamine, wie Dimethylethanolamin sowie 2-Amino-2-methyl-1-propanol. Die Neutralisation der Säuregruppen kann auch mit Hilfe von Mischungen mehrerer Basen vorgenommen werden.

Die in der Copolymerzusammensetzung A) enthaltenen Copolymere eignen sich in vorteilhafter Weise zur Modifizierung der rheologischen Eigenschaften wässriger Zusammensetzungen. Dabei kann es sich z. B. um eine wässriger Wirk- oder Effektstoffzusammensetzung handeln. Es kann sich ganz allgemein beispielsweise um kosmetische Zusammensetzungen, pharmazeutische Zusammensetzungen, Hygieneprodukte, Anstrichmittel, Zusammensetzungen für die Papierindustrie sowie die Textilindustrie handeln.

In einer bevorzugten Ausführungsform enthalten die Zusammensetzungen wenigstens einen wasserlöslichen oder zumindest wasserdispergierbaren Wirk- oder Effektstoff. Selbstverständlich eignen sich die in der Copolymerzusammensetzung A) enthaltenen Copolymere auch zur Modifizierung der rheologischen Eigenschaften von Zusammensetzungen, die wenigstens einen wasserunlöslichen (hydrophoben) Wirk- oder Effektstoff enthalten.

"Modifizierung rheologischer Eigenschaften" wird im Rahmen der vorliegenden Erfindung weit verstanden. Die in der Copolymerzusammensetzung A) enthaltenen Copolymere eignen sich im Allgemeinen zur Verdickung der Konsistenz von wässrigen Zusammensetzungen in einem weiten Bereich. Je nach Grundkonsistenz der flüssigen Zusammensetzungen können in Abhängigkeit von der Einsatzmenge des Copolymers in der Regel Fließeigenschaften von dünnflüssig bis hin zu fest (im Sinne von "nicht mehr fließend") erzielt werden. Unter "Modifizierung rheologischer Eigenschaften" wird daher u. a. die Erhöhung der Viskosität von Flüssigkeiten, die Verbesserung der Tixotropie-Eigenschaften von Gelen, die Verfestigung von Gelen und Wachsen, etc. verstanden. Die erfindungsgemäßen Mittel eignen

sich vorzugsweise zur Formulierung von wässrigen kosmetischen und pharmazeutischen Produkten. Vorzugsweise sind dabei die Copolymerzusammensetzung A) im Allgemeinen klar. Vorteilhafterweise können somit Formulierungen, insbesondere kosmetische Formulierungen, ohne Beeinträchtigung durch die Eigenfarbe der Zusammensetzungen angefärbt werden. Des Weiteren können die Zusammensetzungen in Form von klaren Gelen formuliert werden.

Die in Gegenwart des erfindungsgemäßen Hilfsstoffsystems hergestellten Copolymerzusammensetzungen A) zeichnen sich insgesamt durch vorteilhafte rheologische Eigenschaften aus. Eine weitere Steuerung der rheologiemodifizierenden Eigenschaften kann über die Art und Einsatzmenge der zur Herstellung der Copolymerzusammensetzungen A) eingesetzten Monomere erfolgen. Dies gilt speziell für die Art und Menge des eingesetzten Vernetzers c). Dies gilt weiterhin speziell für den Einsatz oberflächenaktiver Monomere bei der Herstellung von A), wie z. B. die Polyetheracrylate IV c) oder Allylalkoholalkoxilate IV d).

Die Copolymerzusammensetzungen A) eignen sich sowohl zur Herstellung homogenphasiger wässriger Zusammensetzungen als auch zur Formulierung von heterogenphasigen Zusammensetzungen, die zusätzlich wenigstens eine wasserunlösliche (hydrophobe) flüssige oder feste Verbindung umfassen. "Homogenphasige Zusammensetzungen" weisen unabhängig von der Anzahl ihrer Bestandteile nur eine einzige Phase auf. "Heterogenphasige Zusammensetzungen" sind disperse Systeme von zwei oder mehreren miteinander nicht mischbaren Komponenten. Dazu zählen fest/flüssig-, flüssig/flüssig- und fest/flüssig/flüssig-Zusammensetzungen, wie Dispersionen und Emulsionen, z. B. O/W- und W/O-Formulierungen, die wenigstens eine der im Folgenden näher beschriebenen Öl- bzw. Fettkomponenten und Wasser als nicht mischbare Phasen aufweisen. Prinzipiell können die Copolymerzusammensetzungen A) sowohl in der Wasserphase als auch in der Ölphase eingesetzt werden. Im Allgemeinen enthalten heterogenphasige flüssig/flüssig-Zusammensetzungen die Copolymerzusammensetzungen A) im Wesentlichen in der Wasserphase.

Die erfindungsgemäßen Copolymerzusammensetzungen A) eignen sich ganz allgemein zur Herstellung von Wirk- oder Effektstoffzusammensetzungen, enthaltend

A) wenigstens eine Copolymerzusammensetzung, wie zuvor definiert,
B) wenigstens einen Wirk- oder Effektstoff und
C) gegebenenfalls wenigstens einen weiteren, von A) und B) verschiedenen Hilfsstoff.

Wirkstoffe für Kosmetika (z. B. Haar- oder Hautkosmetika), Arzneimittel, Hygienemittel, Textilbehandlungsmittel, etc., d. h. Substanzen, die im Allgemeinen bereits in geringer Konzentration eine Wirkung entfalten, z. B. eine kosmetische Wirkung auf Haut und/oder Haaren, eine pharmakologische Wirkung in einem Organismus, eine reinigende und/oder desinfizierende Wirkung, eine Modifizierung eines textilen Stoffes, z. B. eine knitterfreie Ausrüstung, sowie Effektstoffe, die Lebewesen oder unbelebten Substraten eine bestimmte Eigenschaft verleihen, beispielsweise Farbpigmente für Schminken oder Dispersionsfarben, werden häufig in Form wässriger Wirk- oder Effektstoffzusammensetzungen formuliert und angewendet.

Die erfindungsgemäßen Wirk- und Effektstoffzusammensetzungen enthalten die Copolymerzusammensetzungen A) vorzugsweise in einer Menge von 0,01 bis 50 Gew.-%, besonders bevorzugt 0,05 bis 30 Gew.-%, insbesondere 0,1 bis 20 Gew.-%, bezogen auf das Gesamtgewicht des Mittels. Vorteilhafterweise zeigen die erfindungsgemäßen Copolymerzusammensetzungen bereits in geringen Einsatzmengen gute anwendungstechnische Eigenschaften, z. B. eine gute verdickende Wirkung. In einer speziellen Ausführung enthalten die erfindungsgemäßen Wirk- und Effektstoffzusammensetzungen Polymerkomponente A) in einer Menge von 0,1 bis 5 Gew.-%, bezogen auf das Gesamtgewicht des Mittels.

Die Komponenten B) und C) werden nach dem gewünschten Einsatzbereich der Zusammensetzung ausgewählt. Neben einsatzbereichstypischen Komponenten (z. B. bestimmten pharmazeutischen Wirkstoffen) sind sie z. B. ausgewählt unter Trägern, Exzipienten, Emulgatoren, Tensiden, Konservierungsmitteln, Duftstoffen, von Komponente A) verschiedenen Verdickern, Polymeren, Gelbildnern, Farbstoffen, Pigmenten, Lichtschutzmitteln, Konsistenzgebern, Antioxidantien, Entschäumern, Antistatika, Harzen, Lösemitteln, Lösungsvermittlern, Neutralisierungsmitteln, Stabilisatoren, Sterilantien, Treibmitteln, Trocknungsmitteln, Trübungsmitteln, etc.

Vorzugsweise weisen die Zusammensetzungen eine Trägerkomponente C) auf, die ausgewählt ist unter Wasser, von Wasser verschiedenen hydrophilen Trägern und Mischungen davon.

Geeignete hydrophile Träger C) sind z. B. ein-, zwei- oder mehrwertige Alkohole mit vorzugsweise 1 bis 8 Kohlenstoffatomen, wie Ethanol, n-Propanol, Isopropanol, Propylenglycol, Glycerin, Sorbit, etc.

Die erfindungsgemäßen Zusammensetzungen können als Wirkstoff, z. B. als kosmetischen und/oder pharmazeutischen Wirkstoff B) (wie auch gegebenenfalls als Hilfsstoff C)), wenigstens ein Polymer enthalten, das sich von den erfindungsgemäßen Copolymerzusammensetzungen A) unterscheidet. Dazu zählen ganz allgemein anionische, kationische, amphotere und neutrale Polymere.

Beispiele für anionische Polymere sind Copolymere von Acrylsäure und Acrylamid und deren Salze; Natriumsalze von Polyhydroxycarbonsäuren, wasserlösliche oder wasserdispergierbare Polyester, Polyurethane, z. B. Luviset PUR® der Fa. BASF, und Polyharnstoffe. Besonders geeignete Polymere sind Copolymere aus t-Butylacrylat, Ethylacrylat, Methacrylsäure (z. B. Luvimer® 100P), Copolymere aus Ethylacrylat und Methacrylsäure (z. B. Luvimer® MAE), Copolymere aus N-tert.-Butylacrylamid, Ethylacrylat, Acrylsäure (Ultrahold® 8, strong), Copolymere aus Vinylacetat, Crotonsäure und gegebenenfalls weitere Vinylester (z. B. Luviset®-Marken), Maleinsäureanhydridcopolymere, gegebenenfalls mit Alkohol umgesetzt, anionische Polysiloxane, z. B. carboxyfunktionelle, t-Butylacrylat, Methacrylsäure (z. B. Luviskol® VBM), Copolymere von Acrylsäure und Methacrylsäure mit hydrophoben Monomeren, wie z. B. $C_4$-$C_{30}$-Alkylester der (Meth)acrylsäure, $C_4$-$C_{30}$-Alkylvinylester, $C_4$-$C_{30}$-Alkylvinylether und Hyaluronsäure. Ein Beispiel für ein anionisches Polymer ist weiterhin das unter der Bezeichnung Luviset® Shape (INCI Name: Polyacrylate-22) erhältliche Methylmethacrylat/Methacrylsäure/Acrylsäure/Urethanacrylat-Copolymer. Beispiele für anionische Polymere sind weiterhin Vinylacetat/Crotonsäure-Copolymere, wie sie beispielsweise unter den Bezeichnungen Resyn® (National Starch) und Gafset® (GAF) im Handel sind und Vinylpyrrolidon/Vinylacrylat-Copolymere, erhältlich beispielsweise unter dem Warenzeichen Luviflex® (BASF). Weitere geeignete Polymere sind das unter der Bezeichnung Luviflex® VBM-35 (BASF) erhältliche Vinylpyrrolidon/Acrylat-Terpolymer und Natriumsulfonat-haltige Polyamide oder Natriumsulfonat-haltige Polyester. Weiterhin geeignet sind Vinylpyrrolidon/Ethylmethacrylat/Methacrylsäure-Copolymere wie sie von der Fa. Stepan unter den Bezeichnungen Stepanhold-Extra und -R1 vertrieben werden und die Carboset®-Marken der Fa. BF Goodrich.

Geeignete kationische Polymere sind z. B. kationische Polymere mit der Bezeichnung Polyquaternium nach INCI, z. B. Copolymere aus Vinylpyrrolidon/N-Vinylimidazoliumsalzen (Luviquat® FC, Luviquat® HM, Luviquat® MS, Luviset Clear ®, Luviquat Supreme ®, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidocopolymere (Polyquaternium-7) und Chitosan. Geeignete kationische (quaternisierte) Polymere sind auch Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z. B. Guarpolymere, wie die Jaguar®-Marken der Fa. Rhodia.

Ganz besonders geeignete Polymere sind neutrale Polymere, wie Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und Derivate. Dazu zählt beispielsweise Luviflex® Swing (teilverseiftes Copolymerisat von Polyvinylacetat und Polyethylenglykol, Fa. BASF).

Geeignete Polymere sind auch nichtionische, wasserlösliche bzw. wasserdispergierbare Polymere oder Oligomere, wie Polyvinylcaprolactam, z. B. Luviskol® Plus (BASF SE), oder Polyvinylpyrrolidon und deren Copolymere, insbesondere mit Vinylestern, wie Vinylacetat, z. B. Luviskol® VA 37, VA 55, VA 64, VA 73 (BASF SE); Polyamide, z. B. auf Basis von Itaconsäure und aliphatischen Diaminen, wie sie z. B. in der DE-A-43 33 238 beschrieben sind.

Geeignete Polymere sind auch amphotere oder zwitterionische Polymere, wie die unter den Bezeichnungen Amphomer® (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®).

Geeignete Polymere sind auch nichtionische, siloxanhaltige, wasserlösliche oder -dispergierbare Polymere, z. B. Polyethersiloxane, wie Tegopren® (Fa. Goldschmidt) oder Belsil® (Fa. Wacker).

In einer speziellen Ausführung enthalten die erfindungsgemäßen Zusammensetzungen wenigstens ein Polymer, das sich von den in den Copolymerzusammensetzungen A) enthaltenen Polymeren unterscheidet und das als Verdicker wirkt.

Geeignete polymere Verdicker sind beispielsweise gegebenenfalls modifizierte polymere Naturstoffe (Carboxymethylcellulose und andere Celluloseether, Hydroxyethyl- und -propylcellulose und dergleichen) sowie synthetische polymere Verdicker (Polyacryl- und Polymethacryl-Verbindungen, Vinylpolymere, Polycarbonsäuren, Polyether, Polyimine, Polyamide). Dazu zählen die zum Teil bereits zuvor genannten Polyacryl- und Polymethacryl-Verbindungen, beispielsweise die hochmolekularen mit einem Polyalkenylpolyether, insbesondere einem Allylether von Saccharose, Pentaerythrit oder Propylen, vernetzten Homopolymere der Acrylsäure (INCI-Bezeichnung: Carbomer). Solche Polyacrylsäuren sind u. a. von der Fa. BF Goodrich unter dem Handelsnamen Carbopol ® erhältlich, z. B. Carbopol 940 (Molekulargewicht ca. 4000000), Carbopol 941 (Molekulargewicht ca. 1250000) oder Carbopol 934 (Molekulargewicht ca. 3000000). Weiterhin fallen darunter Acrylsäure-Copolymere, die beispielsweise von der Fa. Rohm & Haas unter den Handelsnamen Aculyn ® und Acusol ® erhältlich sind, z. B. die anionischen, nicht-assoziativen Polymere Aculyn 22, Aculyne 28, Aculyn 33 (vernetzt), Acusol 810, Acusol 823 und Acusol 830 (CAS 25852-37-3). Speziell geeignet sind auch assoziative Verdicker, z. B. auf Basis modifizierter Polyurethane (HEUR) oder hydrophob modifizierter Acryl- oder Methacrylsäure-Copolymere (HASE-Verdicker, High Alkali Swellable Emulsion).

Die Einsatzmenge der zusätzlichen Verdicker liegt vorzugsweise in einem Bereich von 0,001 bis 10 Gew.-%, bevorzugt 0,1 bis 5 %, bezogen auf das Gesamtgewicht der Zusammensetzung.

Beispiele für Effektstoffe, die als erfindungsgemäße wässrige Wirkstoffzusammensetzung formuliert werden können, sind Farbstoffe: z. B. die in DE-A 102 45 209 beschriebenen Farbstoffe sowie die gemäß Colour-Index als Disperse-Farbstoffe und als Solvent-Farbstoffe bezeichneten Verbindungen, die auch als Dispersionsfarbstoffe bezeichnet werden. Eine Zusammenstellung geeigneter Dispersionsfarbstoffe findet sich beispielsweise in Ullmanns Enzyklopädie der technischen Chemie, 4. Auflage, Bd. 10, S. 155-165 (siehe auch Bd. 7, S. 585ff - Anthrachinonfarbstoffe; Bd. 8, S. 244ff-Azofarbstoffe; Bd. 9, S. 313ff - Chinophthalonfarbstoffe). Auf diese Literaturstelle und die darin genannten Verbindungen wird hiermit ausdrücklich Bezug genommen. Erfindungsgemäß geeignete Dispersionsfarbstoffe und Solvent-Farbstoffe umfassen verschiedenste Farbstoffklassen mit unterschiedlichen Chromophoren, beispielsweise Anthrachinonfarbstoffe, Monoazo- und Disazofarbstoffe, Chinophthalone, Methin- und Azamethinfarbstoffe, Naphthalimidfarbstoffe, Naphthochinonfarbstoffe und Nitrofarbstoffe. Beispiele für erfindungsgemäß geeignete Dispersionsfarbstoffe sind die Dispersionsfarbstoffe der folgenden Colour-Index Liste: C. I. Disperse Yellow 1 - 228, C. I. Disperse Orange 1 - 148, C. I. Disperse Red 1 - 349, C. I. Disperse Violet 1 - 97, C. I. Disperse Blue 1 - 349, C. I. Disperse Green 1 - 9, C. I. Disperse Brown 1 - 21, C. I. Disperse Black 1 - 36. Beispiele für erfindungsgemäß geeignete Solvent-Farbstoffe sind die Verbindungen der folgenden Colour-Index Liste: C. I. Solvent Yellow 2 - 191, C. I. Solvent Orange 1 - 113, C. I. Solvent Red 1 - 248, C. I. Solvent Violet 2 - 61, C. I. Solvent Blue 2 - 143, C. I. Solvent Green 1 - 35, C. I. Solvent Brown 1 - 63, C. I. Solvent Black 3 - 50. Erfindungsgemäß geeignete Farbstoffe sind weiterhin Derivate des Naphthalins, des Anthracens, des Perylens, des Terylens, des Quarterylens, sowie Diketopyrrolopyrrolfarbstoffe, Perinonfarbstoffe, Cumarinfarbstoffe, Isoindolin- und Isoindolinonfarbstoffe, Porphyrinfarbstoffe, Phthalocyanin- und Naphthalocyaninfarbstoffe.

Neben den vorgenannten Bestandteilen können die erfindungsgemäßen Wirk- und Effektstoffzusammensetzungen auch konventionelle oberflächenaktive Substanzen und sonstige Additive enthalten. Zu den oberflächenaktiven Substanzen zählen Tenside, Dispergierhilfsmittel und Netzmittel. Zu den sonstigen Additiven zählen insbesondere Verdickungsmittel, Entschäumer, Konservierungsmittel, Frostschutzmittel, Stabilisierungsmittel, etc.

Prinzipiell brauchbar sind anionische, kationische, nichtionische und amphotere Tenside, wobei Polymertenside sowie Tenside mit Heteroatomen in der hydrophoben Gruppe eingeschlossen sind.

Zu den anionischen Tensiden gehören beispielsweise Carboxylate, insbesondere Alkali-, Erdalkali- und Ammoniumsalze von Fettsäuren, z. B. Kaliumstearat, die üblicherweise auch als Seifen bezeichnet werden; Acylglutamate; Sarkosinate, z. B. Natriumlauroylsarkosinat; Taurate; Methylcellulosen; Alkylphosphate, insbesondere Mono- und Diphosphorsäurealkylester; Sulfate, insbesondere Alkylsulfate und Alkylethersulfate; Sulfonate, weitere Alkyl- und Alkylarylsulfonate, insbesondere Alkali-, Erdalkali- und Ammoniumsalze von Arylsulfonsäuren sowie alkylsubstituierten Arylsulfonsäuren, Alkylbenzolsulfonsäuren, wie beispielsweise Lignin- und Phenolsulfonsäure, Naphthalinund Dibutylnaphthalinsulfonsäuren, oder Dodecylbenzolsulfonate, Alkylnaphthalinsulfonate, Alkylmethylestersulfonate, Kondensationsprodukte von sulfoniertem Naphthalin und Derivaten davon mit Formaldehyd, Kondensationsprodukte von Naphthalinsulfonsäuren, Phenol- und/oder Phenolsulfonsäuren mit Formaldehyd oder mit Formaldehyd und Harnstoff, Mono- oder Dialkylbernsteinsäureestersulfonate; sowie Eiweißhydrolysate und Lignin-Sulfitablaugen. Die zuvor genannten Sulfonsäuren werden vorteilhafterweise in Form ihrer neutralen oder gegebenenfalls basischen Salze verwendet.

Zu den kationischen Tensiden gehören beispielsweise quaternierte Ammoniumverbindungen, insbesondere Alkyltrimethylammonium- und Dialkyldimethylammonium-Halogenide und -alkylsulfate sowie Pyridin- und Imidazolin-Derivate, insbesondere Alkylpyridinium-Halogenide.

Zu den nichtionischen Tensiden gehören beispielsweise:

- Fettalkoholpolyoxyethylenester, beispielsweise Laurylalkoholpolyoxyethylenetheracetat,
- Alkyl-Polyoxyethylen- und -polyoxypropylenether, z. B. von iso-Tridecylalkohol und Fettalkohol-Polyoxyethylenether,
- Alkylarylalkohol-Polyoxyethylenether, z. B. Octylphenol-Polyoxyethylenether,
- alkoxylierte tierische und/oder pflanzliche Fette und/oder Öle, beispielsweise Maisölethoxylate, Rizinusölethoxylate, Talgfettethoxylate,
- Glycerinester, wie beispielsweise Glycerinmonostearat,
- Fettalkoholalkoxylate und Oxoalkoholalkoxylate, insbesondere vom Typ $RO-(R_{18}O)_r(R_{19}O)_sR_{20}$ mit $R_{18}$ und $R_{19}$ unabhängig voneinander = $C_2H_4$, $C_3H_6$, $C_4H_8$ und $R_{20}$ = H, oder $C_1$-$C_{12}$-Alkyl, R = $C_3$-$C_{30}$-Alkyl oder $C_6$-$C_{30}$-Alkenyl, r und s unabhängig voneinander 0 bis 50, wobei nicht beide für 0 stehen können, wie iso-Tridecylalkohol und Oleylalkoholpolyoxyethylenether,
- Alkylphenolalkoxylate, wie beispielsweise ethoxyliertes iso-Octyl-, Octyl- oder Nonylphenol, Tributylphenolpolyoxyethylenether,
- Fettaminalkoxylate, Fettsäureamid- und Fettsäurediethanolamidalkoxylate, insbesondere deren Ethoxylate,
- Zuckertenside, Sorbitester, wie beispielsweise Sorbitanfettsäureester (Sorbitan-monooleat, Sorbitantristearat),

Polyoxyethylensorbitanfettsäureester, Alkylpolyglycoside, N-Alkylgluconamide,
- Alkylmethylsulfoxide,
- Alkyldimethylphosphinoxide, wie beispielsweise Tetradecyldimethylphosphinoxid.

Zu den amphoteren Tensiden gehören beispielsweise Sulfobetaine, Carboxybetaine und Alkyldimethylaminoxide, z. B. Tetradecyldimethylaminoxid.

Weitere Tenside, die hier beispielhaft genannt werden sollen, sind Perfluortenside, Silicontenside, Phospholipide, wie beispielsweise Lecithin oder chemisch modifizierte Lecithine, Aminosäuretenside, z. B. N-Lauroylglutamat.

Sofern nicht spezifiziert, handelt es sich bei den Alkylketten der oben aufgeführten Tenside um lineare oder verzweigte Reste mit üblicherweise 8 bis 20 Kohlenstoffatomen.

Die erfindungsgemäßen Wirk- oder Effektstoffzusammensetzungen können wasserlösliche Salze als Komponente B) und/oder C) enthalten, z. B. NaCl.

Die erfindungsgemäßen Wirk- oder Effektstoffzusammensetzungen können für einige Anwendungen organische Lösungsmittel, Öle und/oder Fette enthalten. Bevorzugt sind solche Lösungsmittel, Öle und/oder Fette, die umweltverträglich oder biokompatibel sind. Dazu zählen z. B.:

- Paraffinöle, aromatische Kohlenwasserstoffe und aromatische Kohlenwasserstoffgemische, z. B. Xylole, Solvesso 100, 150 oder 200, und dergleichen,
- Phenole und Alkylphenole, z. B. Phenol, Hydrochinon, Nonylphenol, etc.,
- Ketone mit mehr als 4 C-Atomen wie Cyclohexanon, Isophoron, Isopheron, Acetophenon, Acetonaphthon,
- Alkohole mit mehr als 4 C-Atomen wie acetylierter Lanolinalkohol, Cetylalkohol, 1-Decanol, 1-Heptanol, 1-Hexanol, Isooctadecanol, Isopropylalkohol, Oleylalkohol, Benzylalkohol,
- Carbonsäureester, z. B. Adipinsäuredialkylester, wie Adipinsäurebis(2-ethylhexyl)ester, Phthalsäuredialkylester, wie Phthalsäurebis(2-ethylhexyl)ester, Essigsäurealkylester (auch verzweigte Alkylgruppen), wie Ethylacetat und Acetoessigsäureethylester, Stearate, wie Butylstearat, Glycerinmonostearat, Citrate, wie Acetyltributylcitrat, weiterhin Cetyloctanoat, Methyloleat, Methyl-p-hydroxybenzoat, Methyltetradecanoat, Propyl-p-hydroxybenzoat, Methylbenzoat, Milchsäureester, wie Isopropyllactat, Butyllactat und 2-Ethylhexyllactat,
- Pflanzenöle, wie Palmöl, Rapsöl, Rizinusöl und Derivate davon, wie z. B. oxydiert, Kokosnussöl, Lebertran, Maiskeimöl, Sojabohnenöl, Leinsamenöl, Olivenöl, Erdnussöl, Färberdistelöl, Sesamsamenöl, Grapefruitöl, Basilikumöl, Aprikosenöl, Ingweröl, Geranienöl, Orangenöl, Rosmarinöl, Macadamiaöl, Zwiebelöl, Mandarinenöl, Kiefernöl, Sonnenblumenöl,
- hydrogenierte Pflanzenöle wie hydrogeniertes Palmöl, hydrogeniertes Rapsöl, hydrogeniertes Sojabohnenöl,
- tierische Öle wie Schweinefettöl, Fischöle,
- Dialkylamide mittel- bis langkettiger Fettsäuren, z. B. Hallcomide, sowie
- Pflanzenölester wie Rapsölmethylester.

Die Copolymerzusammensetzungen A) können gemeinsam mit konventionellen Verdickern eingesetzt werden. Dazu zählen die zuvor genannten als Verdicker wirksamen Polymere. Dazu zählen weiterhin Polysaccharide und organische Schichtmineralien wie Xanthan Gum® (Kelzan® der Fa. Kelco), Rhodopol® 23 (Rhone Poulenc) oder Veegum® (Firma R. T. Vanderbilt) oder Attaclay® (Firma Engelhardt). Geeignete Verdickungsmittel sind auch organische natürliche Verdickungsmittel (Agar-Agar, Carrageen, Tragant, Gummi arabicum, Alginate, Pektine, Polyosen, Guar-Mehl, Johannisbrotbaumkernmehl, Stärke, Dextrine, Gelatine, Casein) und anorganische Verdickungsmittel (Polykieselsäuren, Tonmineralien wie Montmorillonite, Zeolithe, Kieselsäuren). Weitere Verdickungsmittel sind die Polysaccharide und Heteropolysaccharide, insbesondere die Polysaccharidgummen, beispielsweise Gummi arabicum, Agar, Alginate, Carrageene und ihre Salze, Guar, Guaran, Traganth, Gellan, Ramsan, Dextran oder Xanthan und ihre Derivate, z. B. propoxyliertes Guar, sowie ihre Mischungen. Andere Polysaccharidverdicker sind beispielsweise Stärken verschiedensten Ursprungs und Stärkederivate, z. B. Hydroxyethylstärke, Stärkephosphatester oder Stärkeacetate, oder Carboxymethylcellulose bzw. ihr Natriumsalz, Methyl-, Ethyl-, Hydroxyethyl-, Hydroxypropyl-, Hydroxypropylmethyl- oder Hydroxyethylmethylcellulose oder Celluloseacetat. Als Verdickungsmittel können weiterhin Schichtsilikate eingesetzt werden. Hierzu zählen beispielsweise die unter dem Handelsnamen Laponite ® erhältlichen Magnesium-oder Natrium-Magnesium-Schichtsilikate der Firma Solvay Alkali sowie die Magnesiumsilikate der Firma Süd-Chemie.

Die Einsatzmenge der zusätzlichen Verdicker liegt vorzugsweise in einem Bereich von 0,001 bis 10 Gew.-%, bevorzugt 0,1 bis 5 %, bezogen auf das Gesamtgewicht der Zusammensetzung.

Als für erfindungsgemäße Dispersionen geeignete Antischaummittel kommen beispielsweise Siliconemulsionen (wie z. B. Silikon® SRE, Firma Wacker oder Rhodorsil® der Firma Rhodia), langkettige Alkohole, Fettsäuren, fluororganische Verbindungen und deren Gemische in Betracht.

Bakterizide können zur Stabilisierung den erfindungsgemäßen Zusammensetzungen gegen Befall mit Mikroorganismen zugesetzt werden. Geeignete Bakterizide sind beispielsweise Proxel® der Fa. ICI oder Acticide® RS der

Fa. Thor Chemie und Kathon® MK der Firma Rohm & Haas.

Geeignete Frostschutzmittel sind organische Polyole, z. B. Ethylenglycol, Propylenglycol oder Glycerin. Diese werden üblicherweise in Mengen von nicht mehr als 10 Gew.-%, bezogen auf das Gesamtgewicht der Wirkstoffzusammensetzung eingesetzt, um den gewünschten Gehalt an flüchtigen Verbindungen nicht zu überschreiten. In einer Ausführungsform der Erfindung beträgt der Anteil hiervon verschiedener flüchtiger organischer Verbindungen vorzugsweise nicht mehr als 1 Gew.-%, insbesondere nicht mehr als 1000 ppm.

Gegebenenfalls können die erfindungsgemäßen Wirkstoffzusammensetzungen 1 bis 5 Gew.-% Puffer, bezogen auf die Gesamtmenge der hergestellten Formulierung, zur pH-Wert Regulation enthalten, wobei sich die Menge und Art des eingesetzten Puffers nach den chemischen Eigenschaften des Wirkstoffes bzw. der Wirkstoffe richtet. Beispiele für Puffer sind Alkalisalze schwacher anorganischer oder organischer Säuren wie z. B. Phosphorsäure, Borsäure, Essigsäure, Propionsäure, Citronensäure, Fumarsäure, Weinsäure, Oxalsäure und Bernsteinsäure.

In einer besonders bevorzugten Ausführungsform werden die erfindungsgemäßen Copolymerzusammensetzungen als Komponente in einem kosmetischen Mittel eingesetzt. Sie können dabei, wie zuvor beschrieben, zur Modifizierung der rheologischen Eigenschaften eines kosmetischen Mittels auf Basis eines wässrigen Mediums dienen.

Ein weiterer Gegenstand der Erfindung ist ein kosmetisches Mittel, enthaltend

A) wenigstens eine Copolymerzusammensetzung, erhältlich durch ein Verfahren, wie zuvor definiert,

B) wenigstens einen kosmetisch akzeptablen Wirkstoff und

C) gegebenenfalls wenigstens einen weiteren, von A) und B) verschiedenen kosmetisch akzeptablen Hilfsstoff.

Bevorzugt umfasst die Komponente C) wenigstens einen kosmetisch oder pharmazeutisch akzeptablen Träger.

Vorzugsweise ist die Träger-Komponente C) ausgewählt unter

i) Wasser,
ii) wassermischbaren organischen Lösungsmitteln, vorzugsweise $C_2$-$C_4$-Alkanolen, insbesondere Ethanol,
iii) Ölen, Fetten, Wachsen,
iv) von iii) verschiedenen Estern von $C_6$-$C_{30}$-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen,
viii) Treibgasen,

und Mischungen davon.

Geeignete Komponenten C) sind die zuvor genannten organischen Lösungsmittel, Öle und Fette.

Speziell geeignete kosmetisch verträgliche Öl- bzw. Fettkomponenten C) sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.

Bei den erfindungsgemäßen kosmetischen Mitteln kann es sich um hautkosmetische, haarkosmetische, dermatologische, hygienische oder pharmazeutische Mittel handeln. Aufgrund ihrer verdickenden Eigenschaften eignen sich die zuvor beschriebenen Copolymerzusammensetzungen A) insbesondere als Zusatzstoffe für Haar- und Hautkosmetika. Sie eignen sich speziell zur Formulierung von Gelen.

Vorzugsweise liegen die erfindungsgemäßen Mittel in Form eines Gels, Schaums, Sprays, einer Salbe, Creme, Emulsion, Suspension, Lotion, Milch oder Paste vor. Gewünschtenfalls können auch Liposomen oder Mikrosphären eingesetzt werden.

Die Copolymerzusammensetzungen eignen sich ebenfalls insbesondere als Emulgator oder Stabilisator für Emulsionen.

Die erfindungsgemäßen kosmetisch aktiven Mittel können zusätzlich kosmetisch und/oder dermatologisch aktive Wirk- und Effektstoffe sowie Hilfsstoffe enthalten. Geeignet sind prinzipiell die zuvor genannten Wirk- und Effektstoffe B) sowie Hilfsstoffe C).

Vorzugsweise enthalten die erfindungsgemäßen kosmetischen Mittel wenigstens eine wie vorstehend definierte Copolymerzusammensetzung A), wenigstens einen wie vorstehend definierten Träger C) und wenigstens einen davon verschiedenen Bestandteil, der vorzugsweise ausgewählt ist unter kosmetisch aktiven Wirkstoffen, Emulgatoren, Tensiden, Konservierungsmitteln, Parfümölen, zusätzlichen Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Tönungsmitteln, Bräunungsmitteln, Farbstoffen, Pigmenten, Konsistenzgebern, Feuchthaltemitteln, Rückfettern, Collagen, Eiweißhydrolysaten, Lipiden, Antioxidantien, Entschäumern, Antistatika, Emollienzien

und Weichmachern.

Zusätzlich zu den Copolymerzusammensetzungen A) für den Einsatz in kosmetischen Mitteln geeignete konventionelle Verdickungsmittel sind die zuvor genannten.

Geeignete kosmetisch und/oder dermatologisch aktive Wirkstoffe sind z. B. Haut- und Haarpigmentierungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, desodorierende Wirkstoffe, sebostatische Wirkstoffe, Pflanzenextrakte, antierythimatös oder antiallergisch aktive Wirkstoffe und Mischungen davon.

Künstlich hautbräunende Wirkstoffe, die geeignet sind, die Haut ohne natürliche oder künstliche Bestrahlung mit UV-Strahlen zu bräunen, sind z. B. Dihydroxyaceton, Alloxan und Walnussschalenextrakt. Geeignete Keratin-härtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat, etc. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure, etc. Derartige desodorierend wirkende Stoffe sind z. B. Zinkricinoleat, Triclosan, Undecylensäurealkylolamide, Citronensäuretriethylester, Chlorhexidin etc. Geeignete Lichtfilterwirkstoffe sind Stoffe, die UV-Strahlen im UV-B- und/oder UV-A-Bereich absorbieren. Geeignete UV-Filter sind die zuvor genannten. Geeignet sind weiterhin p-Aminobenzoesäureester, Zimtsäureester, Benzophenone, Campherderivate sowie UV-Strahlen abhaltende Pigmente, wie Titandioxid, Talkum und Zinkoxid. Geeignete Repellentwirkstoffe sind Verbindungen, die in der Lage sind, bestimmte Tiere, insbesondere Insekten, vom Menschen abzuhalten oder zu vertreiben. Dazu gehört z. B. 2-Ethyl-1,3-hexandiol, N,N-Diethyl-m-toluamid, etc. Geeignete hyperemisierend wirkende Stoffe, welche die Durchblutung der Haut anregen, sind z. B. ätherische Öle, wie Latschenkiefer, Lavendel, Rosmarin, Wacholderbeer, Rosskastanienextrakt, Birkenblätterextrakt, Heublumenextrakt, Ethylacetat, Campher, Menthol, Pfefferminzöl, Rosmarinextrakt, Eukalyptusöl, etc. Geeignete keratolytisch und keratoplastisch wirkende Stoffe sind z. B. Salicylsäure, Kalziumthioglykolat, Thioglykolsäure und ihre Salze, Schwefel, etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z. B. Allantoin, Bisabolol, Dragosantol, Kamillenextrakt, Panthenol, etc.

Die erfindungsgemäßen kosmetischen Mittel können als kosmetischen Wirkstoff (wie auch gegebenenfalls als Hilfsstoff) wenigstens ein kosmetisch oder pharmazeutisch akzeptables Polymer enthalten, das sich vor den erfindungsgemäßen Copolymerzusammensetzungen A) unterscheidet. Dazu zählen ganz allgemein anionische, kationische, amphotere und neutrale Polymere. Auf die zuvor genannten Polymere wird hier voll Bezug genommen.

Nach einer bevorzugten Ausführungsform handelt es sich bei den erfindungsgemäßen Mitteln um ein Hautreinigungsmittel.

Bevorzugte Hautreinigungsmittel sind Seifen von flüssiger bis gelförmiger Konsistenz, wie Transparentseifen, Luxusseifen, Deoseifen, Cremeseifen, Babyseifen, Hautschutzseifen, Abrasivseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und -cremes.

Nach einer weiteren bevorzugten Ausführungsform handelt es sich bei den erfindungsgemäßen Mitteln um kosmetische Mittel zur Pflege und zum Schutz der Haut, Nagelpflegemittel oder Zubereitungen für die dekorative Kosmetik.

Geeignete hautkosmetische Mittel sind z. B. Gesichtswässer, Gesichtsmasken, Deodorantien und andere kosmetische Lotionen. Mittel für die Verwendung in der dekorativen Kosmetik umfassen beispielsweise Abdeckstifte, Theaterfarben, Mascara und Lidschatten, Lippenstifte, Kajalstifte, Eyeliner, Rouges, Puder und Augenbrauenstifte.

Außerdem können die Copolymerzusammensetzungen A) verwendet werden in Nose-Strips zur Porenreinigung, in Antiaknemitteln, Repellents, Rasiermitteln, Haarentfernungsmitteln, Intimpflegemitteln, Fußpflegemitteln sowie in der Babypflege.

Bei den erfindungsgemäßen Hautpflegemitteln handelt es sich insbesondere um W/O-oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen.

Hautkosmetische und dermatologische Mittel auf Basis der zuvor beschriebenen Copolymerzusammensetzung A) zeigen vorteilhafte Wirkungen. Die Copolymerzusammensetzung kann unter anderem zur Feuchthaltung und Konditionierung der Haut und zur Verbesserung des Hautgefühls beitragen. Durch Zusatz der erfindungsgemäßen Copolymerzusammensetzung kann in bestimmten Formulierungen eine erhebliche Verbesserung der Hautverträglichkeit erreicht werden.

Hautkosmetische und dermatologische Mittel enthalten vorzugsweise wenigstens eine Copolymerzusammensetzung A) in einem Anteil von etwa 0,001 bis 30 Gew.-%, vorzugsweise 0,01 bis 20 Gew.-%, ganz besonders bevorzugt 0,1 bis 12 Gew.-%, bezogen auf das Gesamtgewicht des Mittels.

Je nach Anwendungsgebiet können die erfindungsgemäßen Mittel in einer zur Hautpflege geeigneten Form, wie z. B. als Creme, Schaum, Gel, Stift, Mousse, Milch, Spray (Pumpspray oder treibmittelhaltiger Spray) oder Lotion, appliziert werden.

Die hautkosmetischen Zubereitungen können neben den Copolymerzusammensetzungen A) und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Bräunungsmittel, Collagen, Eiweißhydrolysate, Stabilisatoren, pH-Wert-Regulatoren, Farbstoffe, Salze, Verdicker, Gelbildner, Konsistenzgeber, Silicone, Feuchthaltemittel, Rückfetter und weitere übliche Additive.

Bevorzugte Öl- und Fettkomponenten der hautkosmetischen und dermatologischen Mittel sind die zuvor genannten mineralischen und synthetischen Öle, wie z. B. Paraffine, Siliconöle und aliphatische Kohlenwasserstoffe mit mehr als 8 Kohlenstoffatomen, tierische und pflanzliche Öle, wie z. B. Sonnenblumenöl, Kokosöl, Avocadoöl, Olivenöl, Lanolin, oder Wachse, Fettsäuren, Fettsäureester, wie z. B. Triglyceride von $C_6$-$C_{30}$-Fettsäuren, Wachsester, wie z. B. Jojobaöl, Fettalkohole, Vaseline, hydriertes Lanolin und acetyliertes Lanolin sowie Mischungen davon.

Man kann die erfindungsgemäßen Copolymerzusammensetzungen A) auch mit herkömmlichen Polymeren, wie zuvor beschrieben, abmischen, falls spezielle Eigenschaften eingestellt werden sollen.

Zur Einstellung bestimmter Eigenschaften wie z. B. Verbesserung des Anfassgefühls, des Spreitverhaltens, der Wasserresistenz und/oder der Bindung von Wirk- und Hilfsstoffen, wie Pigmenten, können die hautkosmetischen und dermatologischen Zubereitungen zusätzlich auch konditionierende Substanzen auf Basis von Siliconverbindungen enthalten. Geeignete Siliconverbindungen sind beispielsweise Polyalkylsiloxane, Polyarylsiloxane, Polyarylalkylsiloxane, Polyethersiloxane oder Siliconharze.

Die Herstellung der kosmetischen oder dermatologischen Zubereitungen erfolgt nach üblichen, dem Fachmann bekannten Verfahren.

Bevorzugt liegen die kosmetischen und dermatologischen Mittel in Form von Emulsionen, insbesondere als Wasser-in-Öl-(W/O)- oder Öl-in-Wasser-(O/W)-Emulsionen vor. Es ist aber auch möglich, andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen, usw.

Die Herstellung von Emulsionen erfolgt nach bekannten Methoden. Die Emulsionen enthalten neben wenigstens einer Copolymerzusammensetzung A) in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der Emulsionstypspezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion, z. B. für eine Hautcreme, etc., enthält im Allgemeinen eine wässrige Phase, die mittels eines geeigneten Emulgatorsystems in einer Öl- oder Fettphase emulgiert ist. Zur Bereitstellung der wässrigen Phase kann eine Copolymerzusammensetzung A) eingesetzt werden.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karité-Öl, Hoplostethus-Öl; mineralische Öle, deren Destillationsbeginn unter Atmosphärendruck bei ca. 250 °C und deren Destillationsendpunkt bei 410 °C liegt, wie z. B. Vaselinöl; Ester gesättigter oder ungesättigter Fettsäuren, wie Alkylmyristate, z. B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglykol-Copolymer, Fettsäuren und Fettalkohole enthalten.

Neben den Copolymerzusammensetzungen A) können auch Wachse verwendet werden, wie z. B. Carnaubawachs, Candilillawachs, Bienenwachs, mikrokristallines Wachs, Ozokeritwachs und Ca-, Mg- und Al-Oleate, -Myristate, -Linoleate und -Stearate.

Weiterhin kann eine erfindungsgemäße Emulsion als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt. Als Emulgatoren kommen vorzugsweise O/W-Emulgatoren, wie Polyglycerinester, Sorbitanester oder teilveresterte Glyceride, in Betracht.

Nach einer weiteren bevorzugten Ausführungsform handelt es sich bei den erfindungsgemäßen Mitteln um ein Duschgel, eine Shampoo-Formulierung oder ein Badepräparat.

Solche Formulierungen enthalten wenigstens eine Copolymerzusammensetzung A) sowie üblicherweise anionische Tenside als Basistenside und amphotere und/oder nichtionische Tenside als Cotenside. Weitere geeignete

Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/Gelbildnern, Hautkonditioniermitteln und Feuchthaltemitteln.

Diese Formulierungen enthalten vorzugsweise 2 bis 50 Gew.-%, bevorzugt 5 bis 40 Gew.-%, besonders bevorzugt 8 bis 30 Gew.-% Tenside, bezogen auf das Gesamtgewicht der Formulierung.

In den Wasch-, Dusch- und Badepräparaten können alle in Körperreinigungsmitteln üblicherweise eingesetzten anionischen, neutralen, amphoteren oder kationischen Tenside verwendet werden.

Geeignete Tenside sind die zuvor genannten.

Weiterhin können die Duschgel-/Shampoo-Formulierungen zusätzliche Verdicker, wie z. B. Kochsalz, PEG-55, Propylenglykol-Oleat, PEG-120-Methylglucosedioleat und andere, sowie Konservierungsmittel, weitere Wirk- und Hilfsstoffe und Wasser enthalten.

Nach einer weiteren bevorzugten Ausführungsform handelt es sich bei den erfindungsgemäßen Mitteln um ein Haarbehandlungsmittel.

Erfindungsgemäße Haarbehandlungsmittel enthalten vorzugsweise wenigstens eine Copolymerzusammensetzung A) in einer Menge im Bereich von etwa 0,01 bis 10 Gew.-%, bevorzugt 0,1 bis 8 Gew.-%, bezogen auf das Gesamtgewicht des Mittels.

Vorzugsweise liegen die erfindungsgemäßen Haarbehandlungsmittel in Form eines Haarmousses, Haargels, Shampoos, Duschgels vor. Je nach Anwendungsgebiet können die haarkosmetischen Zubereitungen als (Aerosol-)Spray, (Aerosol-)Schaum, Mousse, Gel, Gelspray, Creme, Lotion oder Wachs appliziert werden. Haarsprays umfassen dabei sowohl Aerosolsprays als auch Pumpsprays ohne Treibgas. Haarschäume umfassen sowohl Aerosolschäume wie auch Pumpschäume ohne Treibgas. Haarsprays und Haarschäume umfassen vorzugsweise überwiegend oder ausschließlich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%.

In einer bevorzugten Ausführungsform liegt das Haarbehandlungsmittel in Form eines Gels vor. In einer weiteren bevorzugten Ausführungsform liegt das Haarbehandlungsmittel in Form einer Emulsion vor. Bevorzugt ist hierbei die Verwendung in tensidhaltigen Formulierungen.

Die erfindungsgemäßen haarkosmetischen Formulierungen enthalten in einer bevorzugten Ausführungsform

a) 0,05 bis 5 Gew.-%, vorzugsweise 0,1 bis 3 Gew.-%, wenigstens einer Copolymerzusammensetzung A),

b) 20 bis 99,95 Gew.-% Wasser oder Wasser/Alkohol-Gemisch,

c) 0 bis 70 Gew.-% wenigstens eines Tensids,

d) 0 bis 5 Gew.-% wenigstens eines von a) verschiedenen Emulgators oder Stabilisators,

e) 0 bis 3 Gew.-% wenigstens eines von a) verschiedenen Verdickers, sowie

f) 0 bis 20 Gew.-%, vorzugsweise 0 bis 10 Gew.-%, wenigstens eines von a) bis e) und g) verschiedenen wasserlöslichen oder wasserdispergierbaren Haarpolymers,

g) 0 bis 45 Gew.-%, vorzugsweise 0,05 bis 25 Gew.-%, weitere Bestandteile,

wobei sich die Komponenten a) bis g) zu 100 Gew.-% addieren.

Unter Alkohol sind alle in der Kosmetik üblichen Alkohole zu verstehen, z. B. Ethanol, Isopropanol, n-Propanol. Der Anteil der Alkohole b) an den erfindungsgemäßen haarkosmetischen Formulierungen beträgt vorzugsweise 0 bis 20 Gew.-%.

Unter weiteren Bestandteilen sind die in der Kosmetik üblichen Zusätze zu verstehen, beispielsweise Entschäumer, grenzflächenaktive Verbindungen, d. h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z. B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Stabilisatoren, pH-Wert-Regulatoren, Farbstoffe, Viskositätsregulierer, Gelbildner, Salze, Feuchthaltemittel, Rückfetter, Komplexbildner und weitere übliche Additive.

Haarpolymere sind alle in der Kosmetik bekannten Styling- und Conditionerpolymere, die in Kombination mit

der erfindungsgemäß eingesetzten Copolymerzusammensetzung A) eingesetzt werden können, falls ganz spezielle Eigenschaften eingestellt werden sollen.

Als herkömmliche Haarkosmetik-Polymere eignen sich beispielsweise die zuvor genannten kationischen, anionischen, neutralen, nichtionischen und amphoteren Polymere, auf die hier Bezug genommen wird.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen Copolymerzusammensetzungen eignen sich insbesondere als Verdicker in Haarstyling-Zubereitungen, insbesondere Haarschäumen und Haargelen.

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Eine erfindungsgemäß für Styling-Gele geeignete Zubereitung enthält vorzugsweise:

a) 0,1 bis 5 Gew.-% wenigstens einer Copolymerzusammensetzung A),
b) 0 bis 5 Gew.-% wenigstens eines von A) verschiedenen kosmetisch akzeptablen wasserlöslichen oder wasserdispergierbaren Haarpolymers,
c) 80 bis 99,90 Gew.-% Lösungsmittel, ausgewählt unter Wasser, Alkoholen oder Gemischen davon,
d) 0 bis 1 Gew.-% eines von A) verschiedenen Gelbildners,
e) 0 bis 20 Gew.-% weitere Bestandteile.

Der Anteil der Alkohole c) an den erfindungsgemäßen Styling-Gel-Formulierungen beträgt vorzugsweise 0 bis 20 Gew.-%.

Als zusätzliche Gelbildner können alle in der Kosmetik üblichen Gelbildner eingesetzt werden. Hierzu wird auf die zuvor genannten konventionellen Verdicker Bezug genommen.

Die erfindungsgemäßen Copolymerzusammensetzungen A) eignen sich auch für Shampooformulierungen.

Bevorzugte Shampooformulierungen enthalten:

a) 0,05 - 10 Gew.-% der nach dem erfindungsgemäßen Verfahren erhältlichen Copolymerzusammensetzung A),
b) 25 - 94,95 Gew.-% Wasser,
c) 5 - 50 Gew.-% Tenside,
d) 0 - 5 Gew.-% wenigstens eines Konditioniermittels,
e) 0 - 10 Gew.-% weitere kosmetische Bestandteile.

In den Shampooformulierungen können alle in Shampoos üblicherweise eingesetzten anionischen, neutralen, amphoteren oder kationischen Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkaliund Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxid-Einheiten, bevorzugt 1 bis 3 Ethylenoxid-Einheiten im Molekül aufweisen.

Geeignet sind z. B. Natriumlaurylsulfat, Ammoniumlaurylsulfat, Natriumlaurylethersulfat, Ammoniumlaurylethersulfat, Natriumlauroylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Monooder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z. B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den Copolymerzusammensetzungen A) eingesetzt werden. Hierzu zählen beispielsweise die zuvor genannten kationischen Polymere mit der Bezeichnung Polyquaternium nach INCI, insbesondere Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidcopolymere (Polyquaternium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

Die erfindungsgemäßen Copolymerzusammensetzungen A) eignen sich insbesondere für ölhaltige Formulierungen.

Die erfindungsgemäß zu verwendenden Copolymerzusammensetzungen A) eignen sich ebenso für die Verwendung zur Modifizierung der rheologischen Eigenschaften in pharmazeutischen Zubereitungen jeder Art.

Bevorzugte Mittel, die die nach dem erfindungsgemäßen Verfahren erhältliche Copolymerzusammensetzung A) enthalten, sind:

- Shampoos,
- Hairstyling Gele,
- Hydroalkoholische Gele,
- Moisturizing Gele,
- Badegele,
- Hand-, Körper- und Gesichtslotionen,
- Cremes,
- Sonnenschutzlotionen.

Ein weiterer Gegenstand der Erfindung ist die Verwendung einer Copolymerzusammensetzung A), wie zuvor definiert, als Hilfsstoff in der Pharmazie.

Typische pharmazeutische Zusammensetzungen enthalten

A) eine Copolymerzusammensetzung, wie zuvor definiert,
B) wenigstens einen pharmazeutisch akzeptablen Wirkstoff und
C) gegebenenfalls wenigstens einen weiteren, von A) und B) verschiedenen pharmazeutisch akzeptablen Hilfsstoff.

Pharmazeutisch akzeptable Hilfsstoffe C) sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. DAB, Ph. Eur., BP, NF) gelisteten sowie andere Hilfsstoffe, deren Eigenschaften einer physiologischen Anwendung nicht entgegenstehen.

Geeignete Hilfsstoffe C) können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Siliconderivate, Stabilisatoren, Sterilantien, Treibmittel, Trocknungsmittel, Trübungsmittel, zusätzliche Verdicker, Wachse, Weichmacher, Weißöle. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Zur Herstellung von erfindungsgemäßen pharmazeutischen Mitteln können die Wirkstoffe mit einem geeigneten Exzipienten vermischt oder verdünnt werden. Exzipienten können feste, halb feste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise. Insbesondere handelt es sich dabei um wässrige Lösungen bzw. Solubilisate zur oralen oder zur parenteralen Applikation. Des Weiteren eignen sich die erfindungsgemäß zu verwendende Copolymerzusammensetzung auch zum Einsatz in oralen Darreichungsformen wie Tabletten, Kapseln, Pulvern, Lösungen. Hier können sie den schwerlöslichen Arzneistoff mit einer erhöhten Bioverfügbarkeit zur Verfügung stellen. Bei der parenteralen Applikation können neben Solubilisaten auch Emulsionen, beispielsweise Fettemulsionen eingesetzt werden.

Pharmazeutische Formulierungen der oben genannten Art können durch Verarbeiten der erfindungsgemäß zu verwendenden Copolymerzusammensetzungen A) mit pharmazeutischen Wirkstoffen nach herkömmlichen Methoden und unter Einsatz bekannter und neuer Wirkstoffe erhalten werden.

Der Gehalt an Copolymer A) in den pharmazeutischen Mitteln liegt, abhängig vom Wirkstoff, im Bereich von 0,01 bis 20 Gew.-%, bevorzugt 0,1 bis 10 Gew.-%, besonders bevorzugt 0,5 bis 5 Gew.-%, bezogen auf das Gesamtgewicht des Mittels.

Zur Herstellung der erfindungsgemäßen pharmazeutischen Mittel eignen sich prinzipiell alle pharmazeutischen Wirkstoffe und Pro-Drugs. Hierzu zählen Benzodiazepine, Antihypertensiva, Vitamine, Cytostatika - insbesondere Taxol, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel. Beispiele für geeignete pharmazeutische Wirkstoffe sind die insbesondere die in den Absätzen 0105 bis 0131 der US 2003/0157170 genannten Wirkstoffe.

Neben der Anwendung in der Kosmetik und in der Pharmazie eignen sich die erfindungsgemäß zu verwendenden Copolymerzusammensetzungen A) auch im Lebensmittelbereich zur Modifizierung der rheologischen Eigenschaften. Gegenstand der Erfindung sind daher auch lebensmitteltechnische Zubereitungen, die mindestens eine der erfindungsgemäß zu verwendenden Copolymerzusammensetzungen A) enthalten. Zu den Lebensmittelzubereitungen sind im Rahmen der vorliegenden Erfindung auch Nahrungsergänzungsmittel wie z. B. Lebensmittelfarbstoffe enthaltende Zubereitungen und diätetische Lebensmittel zu verstehen. Darüber hinaus eigenen sich die genannten Copolymerzusammensetzungen A) auch zur Modifizierung der rheologischen Eigenschaften für Futtermittelzusätze für die Tierernährung.

Außerdem eignen sich die Copolymerzusammensetzungen A) zur Herstellung wässriger Zubereitungen von Nahrungsergänzungsmitteln wie wasserunlöslichen Vitaminen und Provitaminen wie Vitamin A, Vitamin A-Acetat, Vitamin D, Vitamin E, Tocopherol-Derivate wie Tocopherolacetat und Vitamin K.

Ein weiterer Gegenstand der Erfindung ist die Verwendung einer Copolymerzusammensetzung A), wie zuvor definiert, als Hilfsmittel in der Pharmazie, bevorzugt als oder in Beschichtungsmittel(n) für feste Arzneiformen, zur Modifizierung rheologischer Eigenschaften, als oberflächenaktive Verbindung, als oder in Klebemittel(n) sowie als oder in Beschichtungsmittel(n) für die Textil-, Papier-, Druck- und Lederindustrie.

Die Erfindung wird anhand der folgenden, nicht einschränkenden Beispiele näher erläutert.

Beispiele:

Abkürzungen:

| | |
|---|---|
| AMPS | 2-Acrylamido-2-methylpropansulfonsäure |
| AS | Acrylsäure |
| MAS | Methacrylsäure |
| VI | Vinylimidazol |
| LUMA | $C_{16}$-$C_{18}$-Alkyl-$(EO)_{25}$-methacrylat |
| Lutencryl® 250 | LUMA : MAS (Gewichtsverhältnis 1:1) |
| Plex 6877-O | Stearylpolyethylenglykol-1100-methacrylat: MMA (1:3) |
| t-BA | tert.-Butylacrylat |
| EA | Ethylacrylat |
| EMA | Ethylmethacrylat |
| LA | Laurylacrylat |
| N-tBAM | N-tert.-Butylacrylamid |
| DMAPMAM | N-[3-(Dimethylamino)propyl]acrylamid |
| PETAE | Pentaerythrittriallylether |
| PEGDMA | Polyethylenglykoldimethacrylat |
| DMEA | N,N-Dimethylethanolamin |
| NMDEA | N-Methyldiethanolamin |
| Melm | N-Methylimidazol |

Allgemeines Verfahren A

Lösungspolymerisation, kein Vernetzer, kein Lactammonomer

(Beispiel I.1 in Tabelle I, Mengenangabe siehe Tabelle)

| | | |
|---|---|---|
| Vorlage | Ethanol | |
| Zulauf 1 | Ethanol | |
| | AMPS (neutralisiert mit Dimethyletanolamin) | |
| | t-BA | |
| | EMA | |
| | MAS | |
| Zulauf 2 | Ethanol | |
| | Wako® V65 | 0,06 g |
| | tert.-Butylperoctoat | 0,55 g |

Durchführung der Reaktion:

In einer Druckapparatur mit Rückflusskühler, Innenthermometer und zwei separaten Zulaufvorrichtungen wurde die Vorlage unter $N_2$-Durchleitung und Rühren bei 200 Upm auf ca. 70 °C erwärmt. Danach startete man bei dieser Temperatur die Zugabe von Zulauf 1 und Zulauf 2. Die Zuläufe 1 und 2 wurden in 5 h zudosiert. Nach beendeter Zugabe der Zuläufe wurde die Reaktionsmischung auf 75 °C erwärmt und unter Rühren bei 75 °C über einen Zeitraum von 12 h nachpolymerisiert.

Das erhaltene Produkt wurde nach dem Abkühlen auf Raumtemperatur abfiltriert und zweimal mit Ethanol gewaschen. Danach wurde das feuchte Produkt in einem Trockenschrank 20 h bei ca. 70 °C getrocknet.

In analoger Weise wurden die Lösungspolymerisate der Beispiele 1.2 bis 1.4 in Tabelle I hergestellt.

Tabelle I: Erfindungsgemäße Lösungspolymerisate

| Bsp. | AMPS [Gew.-%] | VI [Gew.-%] | DMAPMAM [Gew.-%] | t-BA [Gew.-%] | EMA [Gew.-%] | N-tBAM [Gew.-%] | MAS [Gew.-%] | Film | Biegedehnung [cN] | Auswaschbarkeit |
|---|---|---|---|---|---|---|---|---|---|---|
| I.1 | 5,00[a)] | -- | | 60,00 | 15,00 | -- | 20,00 | hart, klar, glatt | 130 | 2-3 |
| I.2 | 2,50[b)] | | 2,50 | 60,00 | 15,00 | | 20,00 | | | |
| I.3 | 5,00[a)] | -- | | 55,00 | -- | 20,00 | 20,00 | hart, klar, glatt | 137 | 2 |
| I.4 | 5,00[c)] | 4,00 | -- | 55,00 | -- | 20,00 | 15,00 | hart, klar, glatt, | 140 | 2-3 |

alle Monomerenangaben in Gew.-%, bezogen auf 100 Gew.-% der Monomerenzusammensetzung

a) vor Polymerisation neutralisiert mit DMEA

b) vor Polymerisation neutralisiert mit DMAPMAM

c) vor Polymerisation neutralisiert mit VI

Filmeigenschaften:

Die erfindungsgemäßen Copolymerzusammensetzungen der Beispiele I.1 bis I.4 wurden zu Aerosol-Haarspray-Formulierungen mit einem VOC-Gehalt von 90 Gew.-% formuliert:

| | |
|---|---|
| Copolymer | 10,00 Gew.-% |
| Ethanol | 55,00 Gew.-% |
| Dimethylether | 34,96 Gew.-% |
| Parfüm, Zusatzstoffe | q.s. |

Die zuvor genannten Haarspray-Formulierungen lieferten klare, feste, glatte Filme. Sie wurden auf eine Glasplatte aufgetragen und die resultierenden Filme wurden im Hinblick auf 3 Kriterien, die in Tabelle 2 angegeben sind, geprüft und mit Noten von 1 bis 4 bewertet. Die Bewertungen der Filme sind in Tabelle 3 wiedergegeben.

Messung der Biegesteifigkeit:

Zur Messung der Biegesteifigkeit werden 3,0 gew.-% ige Lösungen der erfindungsgemäßen Copolymerzusammensetzungen in Ethanol eingesetzt. Die Messung der Biegesteifigkeit wird an 5 bis 10 Haarsträhnen (ca. 3 g und 24 cm Länge) bei 20°C und 65 % rel. Feuchte durchgeführt. Die Werte der Biegesteifigkeit sind ebenfalls in Tabelle 3 wiedergegeben.

Tabelle 2: Bewertungskriterien

| | | |
|---|---|---|
| A) Glätte | spröde (leicht klebrig) | 4 |
| | weich (leicht klebrig) | 3 |
| | hart (nicht klebrig) | 2 |
| | elastisch (nicht klebrig) | 1 |
| B) Auswaschbarkeit | schlecht | 4 |
| | mäßig | 3 |
| | gut | 2 |
| | sehr gut | 1 |
| C) Aussehen Polymerlösung | ungleichmäßig trüb | 4 |
| | ungleichmäßig klar | 3 |
| | gleichmäßig klar | 2 |
| | gleichmäßig glänzend | 1 |

Tabelle 3: Bewertung

| Bsp. -Nr. | Glätte | Auswaschbarkeit | Aussehen Lösung | Biegesteifigkeit [cN] | Treibgasverträglichkeit[d)] | Langzeit-Treibgasverträglichkeite |
|---|---|---|---|---|---|---|
| I.1 | 2 | 1 | 1 | 128 | 1 | 1 |
| I.2 | 2 | 1 | 1 | 114 | 1 | 1 |

[d)] sofort nach Formulierung der Haarsprayformulierung 40%ig in Dimethylether

[e)] 4 Wochen nach Formulierung der Haarsprayformulierung 40%ig in Dimethylether

Allgemeines Verfahren B

Lösungspolymerisation, assoziatives Monomer, kein Vernetzer

Variante B.1:

Durch Lösungspolymerisation in einem Isopropanol/Propylenglycol-Gemisch (Gewichtverhältnis 40 : 30) wird eine Polymerlösung mit einem Feststoffgehalt von 30 % erhalten. Durch Abtrennung des Isopropanols lässt sich einen Polymerlösung mit einem Feststoffgehalt von 50 % in Propylenglycol erzielen.

Variante B.2:

Durch Lösungspolymerisation in Isopropanol wird eine Polymerlösung mit einem Feststoffgehalt von 30 % erhalten. Durch Abtrennung des Isopropanols lässt sich eine pulverförmige Polymerzusammensetzung erzielen, die sich in Propylenglycol leicht auflösen lässt, wobei sich eine Polymerlösung mit einem Feststoffgehalt von 50 % in Propylenglycol erzielen lässt.

Variante B.3:

Durch Lösungspolymerisation in Isopropanol wird eine Polymerlösung mit einem Feststoffgehalt von 30 % erhalten. Das alkoholische Lösungsmittel kann vollständig gegen Wasser ausgetauscht werden, wobei eine klare Lösung erhalten wird. Durch Gefriertrocknen lassen sich gut lösliche weiße Pulver erzielen.

Tabelle II: Erfindungsgemäße Lösungspolymerisate (Variante B)

| Bsp. | Plex 6877-O | Lutencryl 250 | (M)AS, Acrylatmonomer | AMPS +Me-Im [1:1,1] | Verfahren | Eigenschaften |
|---|---|---|---|---|---|---|
| II.1 | -- | 40,00 | 50 AS | 10 | (B.3) | als 5%ige Lösung in $H_2O$ mit pH 3-4 klar löslich |
| II.2 | -- | 40,00 | 50 MAS | 10 | (B.3) | als 5%ige Lösung in $H_2O$ mit pH 3 - 4 klar löslich |
| II.3 | 60,00 | 38,00 | | 2 | (B.2) | gute Eignung als Polymer-Emulgatoren / Stabilisator / Löslichkeitsvermittler /Dispergiermittel |
| II.4 | 60,00 | 34,00 | | 6 | (B.2) | gute Eignung als Polymer-Emulgatoren / Stabilisator / Löslichkeitsvermittler /Dispergiermittel |
| II.5 | 60,00 | 38,00 | | 2 | (B.2) | gute Eignung als Polymer-Emulgatoren / Stabilisator / Löslichkeitsvermittler /Dispergiermittel |
| II.6 | 50,00 | 40,00 | 5 POEA | 5 | (B.1) | gute Eignung als Polymer-Emulgatoren / Stabilisator / Löslichkeitsvermittler /Dispergiermittel |
| II.7 | 50,00 | 40,00 | 5 LA | 5 | (B.1) | gute Eignung als Polymer-Emulgatoren / Stabilisator / Löslichkeitsvermittler /Dispergiermittel |
| II.10 | -- | 90,00 | 5 POEA | 5 | (B1) | gute Eignung für Shampoos |

Allgemeines Verfahren C

Lösungspolymerisation, Monomerzusammensetzung mit assoziativem Monomer und Vernetzer

Tabelle III: Erfindungsgemäße Lösungspolymerisate

| Bsp. | LUMA | MAS | AS | EA | 4MPS[f)] | PEGDMA | Verfahren | Verwendung |
|---|---|---|---|---|---|---|---|---|
| III.1 | 40 | 40 | | -- | 20 | 0,15 % | (C.1) | Als Verdicker für Shampoo |
| III.2 | 40 | 40 | | -- | 20 | 0,15 % | (C.1) | Als Verdicker für Shampoo |
| III.3 | 40 | 40 | | -- | 20 | 0,20 % | (C.1) aber mit 0,5 % Initiator | Als Verdicker für Shampoo |
| III.4 | 40 | 40 | | -- | 20 | 0,25 % | (C1) | Als Verdicker für Shampoo |
| III.5 | 40 | 40 | | -- | 20 | 0,30 % | (C1) aber Polymerisation bei 70-75 °C mit 0,5 % Initiator | Als Verdicker für Shampoo |
| III.6 | 20 | 60 | | -- | 20 | 0,30 % | (C1) aber Polymerisation bei 70-75 °C mit 0,5 % Initiator | Als Verdicker für Shampoo |
| III.7 | 30 | 30 | 20 | -- | 20 | 0,30 % | (C1) aber Polymerisation bei 70-75 °C mit 0,5 % Initiator | |
| III.8 | 20 | 20 | 40 | -- | 20 | 0,30 % | (C1) aber Polymerisation bei 70-75 °C mit 0,5 % Initiator | Als Verdicker für Shampoo Funktioniert mit ~ 1 % Polymer |
| III.9 | 20 | 20 | 40 | -- | 20 | 0,40 % | (C1) aber Polymerisation bei 70-75 °C mit 0,5 % Initiator | |
| III.10 | 30 | 30 | -- | 20 | 20 | 0,40 % | (C1) aber Polymerisation bei 70-75 °C mit 0,5 % Initiator | Als Verdicker für Shampoo |
| III.11 | 20 | 20 | -- | 30 | 30 | 0,40 % | (C1) aber Polymerisation bei 70-75 °C mit 0,5 % Initiator | |
| III.12 | 10 | 10 | 20 | 30 | 30 | 0,40 % | (C1) aber Polymerisation bei 70-75 °C mit 0,5 % Initiator | Zur Herstellung von Gelformulierungen |
| III.13 | 10 | 30 | 30 | -- | 30 | 0,40 % | (C.2) | |

[f)] vor Polymerisation neutralisiert mit Methylimidazol

Allgemeines Verfahren C

Lösungspolymerisation, assoziatives Monomer, Vernetzer

Variante C.1:

Durch Lösungspolymerisation in Isopropanol bei 75 bis 80 °C mit einer Initiatormenge von 1 Gew.-% bezogen auf das Gesamtgewicht der zu polymerisierenden Monomere wird eine Polymerlösung mit einem Feststoffgehalt von 30 % erhalten. Durch Abtrennung des Isopropanols lässt sich eine pulverförmige Polymerzusammensetzung erzielen, die sich in Propylenglycol leicht auflösen lässt, wobei sich eine Polymerlösung mit einem Feststoffgehalt von 40 % in Propylenglycol erzielen lässt.

Variante C.2:

Durch Lösungspolymerisation in Isopropanol bei 70 bis 75°C mit einer Initiatormenge von 0,5 Gew.-% bezogen auf das Gesamtgewicht der zu polymerisierenden Monomere wird eine Polymerlösung mit einem Feststoffgehalt von 30 % erhalten. Durch Abtrennung des Isopropanols lässt sich eine pulverförmige Polymerzusammensetzung erzielen, die sich in Wasser leicht auflösen lässt, wobei sich eine Polymerlösung mit einem Feststoffgehalt von 40 % in Wasser erzielen lässt.

## Patentansprüche

**1.** Verfahren zur Herstellung einer Copolymerzusammensetzung A) durch radikalische Copolymerisation einer Monomerzusammensetzung, die 2-Acrylamido-2-methylpropansulfonsäure (Monomer a) und wenigstens ein damit copolymerisierbares Comonomer enthält, mit der Maßgabe,

- dass die 2-Acrylamido-2-methylpropansulfonsäure a) vor ihrem Einsatz in der Polymerisation mit einer Aminkomponente AM) neutralisiert wird, die aus Aminen ausgewählt ist, die ausschließlich sekundäre und/oder tertiäre Aminogruppen aufweisen,
- wobei die Aminogruppen der Aminkomponente AM) in wenigstens äquimolarer Menge bezogen auf die Sufonsäuregruppen der 2-Acrylamido-2-methylpropansulfonsäure eingesetzt werden und
- die Polymerisation in einem Lösungsmittel erfolgt, das wenigstens einen Alkohol umfasst oder aus wenigstens einem Alkohol besteht.

**2.** Verfahren nach Anspruch 1, wobei der Wassergehalt der zur Polymerisation eingesetzten Reaktionsmischung höchstens 5 Gew.-%, bevorzugt höchstens 3 Gew.-%, insbesondere höchstens 1 Gew.-%, bezogen auf das Gesamtgewicht der Reaktionsmischung, beträgt.

**3.** Verfahren nach einem der Ansprüche 1 oder 2, wobei die Monomerzusammensetzung wenigstens eine N-Vinylimidazol-Verbindung b) der allgemeinen Formel (I)

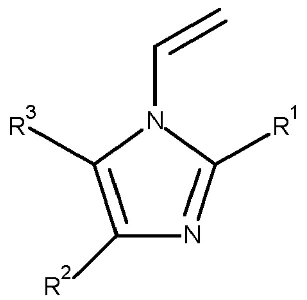

(I)

enthält, worin $R^1$, $R^2$ und $R^3$ unabhängig voneinander für Wasserstoff, $C_1$-$C_4$-Alkyl oder Phenyl stehen.

**4.** Verfahren nach einem der vorhergehenden Ansprüche, wobei die zur Herstellung der Copolymerzusammensetzung A) eingesetzte Monomerzusammensetzung wenigstens ein amidgruppenhaltiges Monomer c) enthält, das ausgewählt ist unter einer α,β-ethylenisch ungesättigten amidgruppenhaltigen Verbindung der allgemeinen Formel (II)

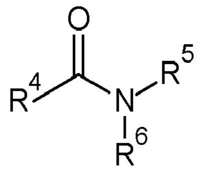

(II)

wobei

einer der Reste $R^4$ bis $R^6$ für eine Gruppe der Formel $CH_2=CR^7$- mit $R^7$ = H oder $C_1$-$C_4$-Alkyl steht und die übrigen Reste $R^4$ bis $R^6$ unabhängig voneinander für H, Alkyl, Cycloalkyl, Heterocycloalkyl, Aryl oder Hetaryl stehen,
wobei $R^5$ und $R^6$ gemeinsam mit dem Stickstoffatom, an das sie gebunden sind, auch für einen fünf- bis siebengliedrigen Heterocyclus stehen können.

**5.** Verfahren nach einem der vorhergehenden Ansprüche, wobei die Monomerzusammensetzung wenigstens eine von den Verbindungen a) verschiedene Verbindung d) mit einer radikalisch polymerisierbaren, $\alpha,\beta$-ethylenisch ungesättigten Doppelbindung und wenigstens einer anionogenen und/oder anionischen Gruppen pro Molekül enthält, vorzugsweise ausgewählt unter Acrylsäure, Methacrylsäure und Mischungen davon.

**6.** Verfahren nach einem der vorhergehenden Ansprüche, wobei die zur Herstellung der Copolymerzusammensetzung A) eingesetzte Monomerzusammensetzung zusätzlich wenigstens ein ethergruppenhaltiges Monomer e) enthält, das ausgewählt ist unter Verbindungen der allgemeinen Formeln III

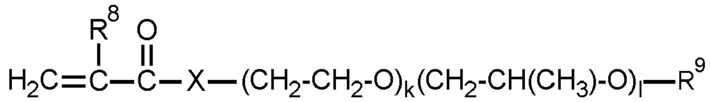

(III)

worin
die Reihenfolge der Alkylenoxideinheiten in den Verbindungen (III) beliebig ist,

k und l unabhängig voneinander für eine ganze Zahl von 0 bis 1000 stehen, wobei die Summe aus k und l mindestens 5 beträgt,
$R^8$ für Wasserstoff oder $C_1$-$C_4$-Alkyl, vorzugsweise Methyl, steht,
$R^9$ für Wasserstoff, $C_1$-$C_{30}$-Alkyl, $C_2$-$C_{30}$-Alkenyl oder $C_5$-$C_8$-Cycloalkyl steht,
X für O oder eine Gruppe der Formel $NR^{10}$ steht, worin $R^{10}$ für H, Alkyl, Alkenyl, Cycloalkyl, Heterocycloalkyl, Aryl oder Hetaryl steht.

**7.** Verfahren nach einem der vorhergehenden Ansprüche, wobei die Monomerzusammensetzung wenigstens eine radikalisch polymerisierbare vernetzende Verbindung g) enthält, die wenigstens zwei $\alpha,\beta$-ethylenisch ungesättigte Doppelbindungen pro Molekül enthält.

**8.** Verfahren nach einer der vorhergehenden Ausführungsformen, wobei die zur Herstellung der Copolymerzusammensetzung A) eingesetzte Monomerzusammensetzung wenigstens ein weiteres Monomer h) enthält, das ausgewählt ist unter
h1) Estern $\alpha,\beta$-ethylenisch ungesättigter Mono- und Dicarbonsäuren mit $C_1$-$C_{30}$-Alkanolen,
h2) Estern $\alpha,\beta$-ethylenisch ungesättigter Mono- und Dicarbonsäuren mit $C_2$-$C_{30}$-Diolen,
h3) Amiden $\alpha,\beta$-ethylenisch ungesättigter Mono- und Dicarbonsäuren mit $C_2$-$C_{30}$-Aminoalkoholen, die eine primäre oder sekundäre Aminogruppe aufweisen,
h4) amidgruppenhaltigen Monomeren, die eine Gruppe der Formeln (IIIa) oder (IIIb)

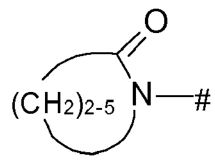
(IVa)

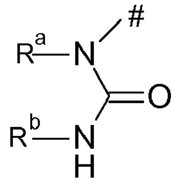
(IVb)

aufweisen, worin

# für die Bindungsstelle zu einer Gruppe mit einer radikalisch polymerisierbaren, α,β-ethylenisch ungesättigten Doppelbindung steht, wobei in den Verbindungen (IIIa) # nicht für die Bindungsstelle zu einer Gruppe der Formel $CH_2=CR^7$- mit $R^7$ = H oder $C_1$-$C_4$-Alkyl steht,
$R^a$ für H oder $C_1$-$C_4$-Alkyl steht,
$R^b$ für H oder $C_1$-$C_4$-Alkyl steht, oder
$R^a$ und $R^b$ gemeinsam für $(CH_2)_{1-4}$ stehen,

h5) Urethan(meth)acrylaten mit Alkylenoxidgruppen,
h6) $C_1$-$C_{30}$-Alkylvinylethern,
h7) Vinylaromaten,
h8) Vinylhalogeniden, Vinylidenhalogeniden,
und Mischungen davon.

**9.** Verfahren nach einem der vorhergehenden Ansprüche, wobei die Aminkomponente AM) ausgewählt ist unter N-Vinylimidazolverbindungen (= Monomer b)), N-($C_1$-$C_8$)-Alkylimidazolen, N-(Di-($C_1$-$C_4$)-Alkylamino-($C_1$-$C_8$)-alkyl)imidazolen, N-($C_1$-$C_8$)-Alkylpiperazinen, N-Hydroxyalkylpiperazinen, Alkyldialkanolaminen, Trialkanolaminen und Mischungen davon.

**10.** Verfahren nach einem der vorhergehenden Ansprüche, wobei die Aminkomponente AM) ausgewählt ist unter N-Vinylimidazol, N-Methylimidazol, Methyldiethanolamin, Triethanolamin und Mischungen davon.

**11.** Verfahren nach einem der vorhergehenden Ansprüche, wobei die Polymerisation als Lösungspolymerisation in einem Lösungsmittel erfolgt, das

- Ethanol und/oder Isopropanol, oder
- Isopropanol und Propylenglycol

enthält.

**12.** Verfahren nach einem der Ansprüche 1 bis 11, wobei die zur Copolymerisation eingesetzte Monomerzusammensetzung

- 3 bis 25 Gew.-%, bezogen auf das Gesamtgewicht der zur Polymerisation eingesetzten Monomere, 2-Acrylamido-2-methylpropansulfonsäure a),
- 0 bis 90 Gew.-%, bezogen auf das Gesamtgewicht der zur Polymerisation eingesetzten Monomere, wenigstens einer Verbindung c), ausgewählt unter $C_1$-$C_6$-Alkyl(meth)acrylamiden,
- 0 bis 90 Gew.-%, bezogen auf das Gesamtgewicht der zur Polymerisation eingesetzten Monomere, wenigstens einer Verbindung h1), ausgewählt unter $C_1$-$C_6$-Alkyl(meth)acrylaten,
- 0 bis 20 Gew.-%, bezogen auf das Gesamtgewicht der zur Polymerisation eingesetzten Monomere, wenigstens einer N-Vinylimidazol-Verbindung b),
- 0 bis 50 Gew.-%, bezogen auf das Gesamtgewicht der zur Polymerisation eingesetzten Monomere, wenigstens einer Verbindung d), ausgewählt unter Acrylsäure, Methacrylsäure, Salzen der Acrylsäure, Salzen der Methacrylsäure und Mischungen davon,

enthält, mit der Maßgabe, dass das Gesamtgewicht der Monomere c) und h1) 10 bis 90 Gew.-%, bezogen auf das Gesamtgewicht der zur Polymerisation eingesetzten Monomere, beträgt, und dass sich die zur Polymerisation eingesetzten Monomere auf 100 Gew.-% addieren.

**13.** Verfahren nach einem der Ansprüche 1 bis 11, wobei die zur Copolymerisation eingesetzte Monomerzusammensetzung

- 0,5 bis 30 Gew.-%, bezogen auf das Gesamtgewicht der zur Polymerisation eingesetzten Monomere, 2-Acrylamido-2-methylpropansulfonsäure a),
- 5 bis 70 Gew.-%, bezogen auf das Gesamtgewicht der zur Polymerisation eingesetzten Monomere, wenigstens einer Verbindung d), die ausgewählt ist unter Acrylsäure, Methacrylsäure, Salzen der Acrylsäure, Salzen der Methacrylsäure und Mischungen davon,
- 5 bis 50 Gew.-%, bezogen auf das Gesamtgewicht der zur Polymerisation eingesetzten Monomere, wenigstens einer Verbindung e), die ausgewählt ist mit $C_8$-$C_{30}$-Alkylgruppen terminierten Polyether(meth)acrylaten und Mischungen davon,
- 0 bis 25 Gew.-%, bezogen auf das Gesamtgewicht der zur Polymerisation eingesetzten Monomere, wenigstens einer Verbindung c), ausgewählt unter $C_1$-$C_6$-Alkyl(meth)acrylamiden,
- 0 bis 25 Gew.-%, bezogen auf das Gesamtgewicht der zur Polymerisation eingesetzten Monomere, wenigstens einer Verbindung h1), ausgewählt unter $C_1$-$C_{30}$-Alkyl(meth)acrylaten,

enthält, mit der Maßgabe, dass sich die zur Polymerisation eingesetzten Monomere auf 100 Gew.-% addieren.

**14.** Verfahren nach einem der Ansprüche 1 bis 11, wobei die zur Copolymerisation eingesetzte Monomerzusammensetzung

- 0,5 bis 50 Gew.-%, bezogen auf das Gesamtgewicht der zur Polymerisation eingesetzten Monomere, 2-Acrylamido-2-methylpropansulfonsäure a),
- 5 bis 90 Gew.-%, bezogen auf das Gesamtgewicht der zur Polymerisation eingesetzten Monomere, wenigstens einer Verbindung d), die ausgewählt ist unter Acrylsäure, Methacrylsäure, Salzen der Acrylsäure, Salzen der Methacrylsäure und Mischungen davon,
- 5 bis 50 Gew.-%, bezogen auf das Gesamtgewicht der zur Polymerisation eingesetzten Monomere, wenigstens einer Verbindung e), die ausgewählt ist unter mit $C_8$-$C_{30}$-Alkylgruppen terminierten Polyether(meth)acrylaten,
- 0,1 bis 3 Gew.-%, bezogen auf das Gesamtgewicht der zur Polymerisation eingesetzten Monomere, wenigstens eines Vernetzers g),
- 0 bis 40 Gew.-%, bezogen auf das Gesamtgewicht der zur Polymerisation eingesetzten Monomere, wenigstens einer Verbindung c), ausgewählt unter $C_1$-$C_6$-Alkyl(meth)acrylamiden,
- 0 bis 40 Gew.-%, bezogen auf das Gesamtgewicht der zur Polymerisation eingesetzten Monomere, wenigstens einer Verbindung h1), ausgewählt unter $C_1$-$C_{30}$-Alkyl(meth)acrylaten,

enthält, mit der Maßgabe, dass sich die zur Polymerisation eingesetzten Monomere auf 100 Gew.-% addieren.

**15.** Copolymerzusammensetzung A), erhältlich durch ein Verfahren, wie in einem der Ansprüche 1 bis 14 definiert.

**16.** Kosmetisches Mittel, enthaltend

A) wenigstens eine Copolymerzusammensetzung, erhältlich durch ein Verfahren, wie in einem der Ansprüche 1 bis 14 definiert,
B) wenigstens einen kosmetisch akzeptablen Wirkstoff und
C) gegebenenfalls wenigstens einen weiteren, von A) und B) verschiedenen kosmetisch akzeptablen Hilfsstoff.

**17.** Verwendung einer Copolymerzusammensetzung A), erhältlich durch ein Verfahren, wie in einem der Ansprüche 1 bis 14 definiert, als Hilfsstoff in der Kosmetik, als Hilfsstoff in der Pharmazie, als Hilfsstoff in der Lebensmittelindustrie, als oberflächenaktive Verbindung, als oder in Klebemittel(n) sowie als oder in Beschichtungsmittel(n) für die Textil-, Papier-, Druck- und Lederindustrie.

**18.** Verwendung einer Copolymerzusammensetzung A), erhältlich durch ein Verfahren, wie in Anspruch 12 definiert, als filmbildendes Polymer in der Kosmetik oder Pharmazie, insbesondere als Festigerpolymer in der Haarkosmetik.

**19.** Verwendung einer Copolymerzusammensetzung A), erhältlich durch ein Verfahren, wie in Anspruch 13 definiert, als grenzflächenaktive Verbindung, insbesondere als Polymeremulgator oder Polymerstabilisator.

**20.** Verwendung einer Copolymerzusammensetzung A), erhältlich durch ein Verfahren, wie in Anspruch 14 definiert,

als Verdickungsmittel.

Europäisches Patentamt
European Patent Office
Office européen des brevets

# EUROPÄISCHER RECHERCHENBERICHT

Nummer der Anmeldung
EP 14 15 7228

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br><br><br><br>Y | WO 2012/119746 A1 (CLARIANT INT LTD [CH]; KLUG PETER [DE]; FISCHER DIRK [DE]; LINDNER THO)<br>13. September 2012 (2012-09-13)<br>* Seite 58, Zeile 5 - Zeile 21 *<br>----- | 1-15<br><br><br><br>1-15 | INV.<br>C08F220/18<br>C08F220/20<br>C08F220/58<br>C08F220/60<br>A61Q5/00<br>A61K31/78 |
| Y,D | WO 2004/058837 A2 (BASF AG [DE]; NGUYEN-KIM SON [DE]; SCHUCH HORST [DE]; KAISER THOMAS [D)<br>15. Juli 2004 (2004-07-15)<br>* Ansprüche *<br>----- | 1-15 | |
| X,D | EP 0 003 235 A1 (BASF AG [DE])<br>8. August 1979 (1979-08-08)<br>* Seite 4, Zeile 1 - Zeile 20 *<br>* Ansprüche *<br>----- | 1-15 | |
| X,D | WO 2010/009953 A2 (HENKEL AG & CO KGAA [DE]; KNAPPE THORSTEN [DE]; FLODROP VAN HELGA [DE]) 28. Januar 2010 (2010-01-28)<br>* Ansprüche *<br>----- | 1-15 | RECHERCHIERTE SACHGEBIETE (IPC) |
| X,D | US 5 260 391 A (STEPHENS MICHAEL [US])<br>9. November 1993 (1993-11-09)<br>* Ansprüche *<br>----- | 1-15 | C08F |

Der vorliegende Recherchenbericht wurde für alle Patentansprüche erstellt

1

| Recherchenort | Abschlußdatum der Recherche | Prüfer |
|---|---|---|
| Den Haag | 7. Januar 2015 | Kaumann, Edgar |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 14 15 7228

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-01-2015

| Im Recherchenbericht angeführtes Patentdokument | Datum der Veröffentlichung | Mitglied(er) der Patentfamilie | Datum der Veröffentlichung |
|---|---|---|---|
| WO 2012119746 A1 | 13-09-2012 | CN 103492437 A | 01-01-2014 |
| | | DE 102011013341 A1 | 08-12-2011 |
| | | EP 2683751 A1 | 15-01-2014 |
| | | JP 2014511423 A | 15-05-2014 |
| | | US 2014086854 A1 | 27-03-2014 |
| | | WO 2012119746 A1 | 13-09-2012 |
| WO 2004058837 A2 | 15-07-2004 | AT 350409 T | 15-01-2007 |
| | | AU 2003303458 A1 | 22-07-2004 |
| | | CN 1756780 A | 05-04-2006 |
| | | DE 10261750 A1 | 15-07-2004 |
| | | EP 1581569 A2 | 05-10-2005 |
| | | ES 2279230 T3 | 16-08-2007 |
| | | JP 4634806 B2 | 23-02-2011 |
| | | JP 2006512433 A | 13-04-2006 |
| | | US 2006183822 A1 | 17-08-2006 |
| | | WO 2004058837 A2 | 15-07-2004 |
| EP 0003235 A1 | 08-08-1979 | DE 2758122 A1 | 05-07-1979 |
| | | DK 577378 A | 25-06-1979 |
| | | EP 0003235 A1 | 08-08-1979 |
| | | NO 784356 A | 26-06-1979 |
| WO 2010009953 A2 | 28-01-2010 | DE 102008034102 A1 | 28-01-2010 |
| | | EP 2337553 A2 | 29-06-2011 |
| | | US 2011110878 A1 | 12-05-2011 |
| | | WO 2010009953 A2 | 28-01-2010 |
| US 5260391 A | 09-11-1993 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5260391 A
- EP 0003235 A1
- US 20110319561 A1
- WO 2004058837 A
- WO 2010009953 A
- EP 1028129 A1
- DE 19838851
- WO 9732917 A
- JP 58217598 A
- WO 9013533 A
- WO 9507331 A
- DE 4333238 A
- DE 3929973
- DE 2150557
- DE 2817369
- DE 3708451
- DE 10245209 A
- US 20030157170 A


### In der Beschreibung aufgeführte Nicht-Patentliteratur


- **W. C. GRIFFIN.** *J. Soc. Cosmetic Chem.,* 1954, vol. 5, 249
- **K. H. SCHRADER.** Grundlagen und Rezepturen der Kosmetika. Hüthig Verlag, 1989
- Ullmanns Enzyklopädie der technischen Chemie. vol. 10, 155-165
- Anthrachinonfarbstoffe. ULLMANNS ENZYKLOPÄDIE DER TECHNISCHEN CHEMIE. vol. 7, 585ff
- Azofarbstoffe. ULLMANNS ENZYKLOPÄDIE DER TECHNISCHEN CHEMIE. vol. 8, 244ff
- Chinophthalonfarbstoffe. ULLMANNS ENZYKLOPÄDIE DER TECHNISCHEN CHEMIE. vol. 9, 313ff
- **KARL-HEINZ SCHRADER.** Grundlagen und Rezepturen der Kosmetika. Verlag Hüthig, 319-355
- **SCHRADER.** Grundlagen und Rezepturen der Kosmetika. Hüthig Buch Verlag, 1989
- **FIEDLER, H. P.** Lexikon der Hilfsstoffe für Pharmazie, Kosmetik und angrenzende Gebiete. ECV-Editio-Kantor-Verlag, 1996